# EUROPEAN PATENT APPLICATION

(11) **EP 1 134 230 A1**
(43) Date of publication of application: **19.09.2001**
(21) Application number: 99972643.3
(22) Date of filing: 19.11.1999
(51) Int. Cl.: C07K 14/47, C12N 15/12, C12N 1/21, C12P 21/02, C12Q 1/68, C07K 16/18, C12P 21/08, G01N 33/53, A61P 25/28, A61P 25/18, A61P 25/08, A61P 37/00, C12N 15/63

(54) **NOVEL POLYPEPTIDE**

(30) Priority: 24.11.1998 JP 33248498; 02.09.1999 JP 24844299
(71) Applicant: KYOWA HAKKO KOGYO CO., LTD., Chiyoda-ku, Tokyo 100-8185 (JP)
(72) Inventor: ICHIMURA, M., Pharm.Res., Kyowa Hakko Kogyo Co.Ltd, Sunto-gun, Shizuoka 411-8731 (JP); HIROSE, Ryo, Pharm.Res., Kyowa Hakko Kogyo Co. Ltd, Sunto-gun, Shizuoka 411-8731 (JP); YOSHIOKA, Katsuji, Kanazawa-shi, Ishikawa 920-0944 (JP)
(74) Representative: VOSSIUS & PARTNER
(86) International application number: JP9906487
(87) International publication number: WO0031132

(57) **Abstract**

It is to provide agents for diagnosing, preventing and treating a disease related to JNK3 cascade.

The present invention provides a novel polypeptide JSAP which binds to JNK3, a production method of the polypeptide, DNA encoding the polypeptide, a recombinant DNA obtained by inserting the DNA into a vector, a transformant comprising the recombinant vector, an antibody recognizing the polypeptide, a determination method and immunostaining method of the polypeptide of the present invention using the antibody, a screening method using the polypeptide, and agents for diagnosing, preventing and treating a disease related to JNK3 cascade using the polypeptide, the DNA or the antibody.

SEQUENCE LISTING

<110> KYOWA HAKKO KOGYO CO., LTD.,

<120> NOVEL POLYPEPTIDE

<130> 11169

<140>

<141>

<150> H10-332484
<151> 1998-11-24

<150> H11-248442
<151> 1999-09-02

<160> 18

<170> PatentIn Ver. 2.0

<210> 1

<211> 4173

<212> DNA

<213> Mouse

<220>

<221> CDS

<222> (107)..(4021)

<400> 1

ggcctgggcg gcggcacatc ctaaggtagc ggctgcctga ggtgacagct gcccgtggat 60

tcgggccccg gaacgagccg cgctggcggc ggcggcggta gccgcg atg atg gag 115
Met Met Glu
1

atc cag atg gac gag gga gga ggt gtg gtg gtg tac caa gac gac tac 163
Ile Gln Met Asp Glu Gly Gly Gly Val Val Val Tyr Gln Asp Asp Tyr
    5                   10                  15

tgc tcg ggc tcg gtc atg tcg gag cgt gtg tcg ggc ctg gcg ggc tcc 211
Cys Ser Gly Ser Val Met Ser Glu Arg Val Ser Gly Leu Ala Gly Ser
20                  25                  30                  35

atc tac cgc gag ttc gag cgc ctc att cac tgc tat gac gag gag gtg 259
Ile Tyr Arg Glu Phe Glu Arg Leu Ile His Cys Tyr Asp Glu Glu Val
                40                  45                  50

gtc aag gag ctc atg ccg ctg gtg gtg aac gtg ctg gag aac ctt gac 307
Val Lys Glu Leu Met Pro Leu Val Val Asn Val Leu Glu Asn Leu Asp
            55                  60                  65

tcg gtg ctg agc gag aac cag gag cac gag gtg gag ctg gag ctc cta 355
Ser Val Leu Ser Glu Asn Gln Glu His Glu Val Glu Leu Glu Leu Leu

        70                  75                  80

cgc gag gac aac gag cag ctg ctc acg caa tac gag cgc gag aag gcg 403
Arg Glu Asp Asn Glu Gln Leu Leu Thr Gln Tyr Glu Arg Glu Lys Ala
    85                  90                  95

ctg cgc aaa cag gcc gag gag aaa ttc atc gaa ttt gaa gat gcc ttg 451
Leu Arg Lys Gln Ala Glu Glu Lys Phe Ile Glu Phe Glu Asp Ala Leu
100                 105                 110                 115

gaa caa gag aag aaa gaa ctc cag atc cag gta gaa cat tat gag ttt 499
Glu Gln Glu Lys Lys Glu Leu Gln Ile Gln Val Glu His Tyr Glu Phe
                120                 125                 130

cag aca cgc cag ctg gag cta aag gcc aaa aac tat gca gat cag att 547
Gln Thr Arg Gln Leu Glu Leu Lys Ala Lys Asn Tyr Ala Asp Gln Ile
            135                 140                 145

tcc cga ctg gag gaa cga gaa tcg gag atg aag aag gaa tac aat gcc 595
Ser Arg Leu Glu Glu Arg Glu Ser Glu Met Lys Lys Glu Tyr Asn Ala
        150                 155                 160

ctg cac cag cgg cac aca gag atg atc cag acc tat gtg gaa cac att 643
Leu His Gln Arg His Thr Glu Met Ile Gln Thr Tyr Val Glu His Ile
    165                 170                 175

gaa aga tcc aag atg cag caa gtt ggg ggt agc ggc caa aca gaa agc    691
Glu Arg Ser Lys Met Gln Gln Val Gly Gly Ser Gly Gln Thr Glu Ser
180                 185                 190                 195

agc ctg ccc ggg cgg agg aag gag cgt ccc acc tct ctg aat gtc ttc    739
Ser Leu Pro Gly Arg Arg Lys Glu Arg Pro Thr Ser Leu Asn Val Phe
                200                 205                 210

ccc ctg gct gat ggc atg tgt cca aac gat gag atg tct gag tca ggc    787
Pro Leu Ala Asp Gly Met Cys Pro Asn Asp Glu Met Ser Glu Ser Gly
            215                 220                 225

cag tcc tca gca gct gca aca ccc agt acc aca ggt acc aag tcc aac    835
Gln Ser Ser Ala Ala Ala Thr Pro Ser Thr Thr Gly Thr Lys Ser Asn
        230                 235                 240

aca ccc acg tcc tcc gtg ccc tca gca gca gtc acg cca ctc aac gag    883
Thr Pro Thr Ser Ser Val Pro Ser Ala Ala Val Thr Pro Leu Asn Glu
    245                 250                 255

agc cta cag ccc ctg ggg gac tat gtc agt gtc aca aag aac aac aag    931
Ser Leu Gln Pro Leu Gly Asp Tyr Val Ser Val Thr Lys Asn Asn Lys
260                 265                 270                 275

cag gcc cga gag aag cgc aat agc cgt aac atg gag gtc cag gtc acc    979
Gln Ala Arg Glu Lys Arg Asn Ser Arg Asn Met Glu Val Gln Val Thr

                280                 285                 290

caa gag atg cgg aac gtc agt atc ggc atg ggc agc agt gac gag tgg 1027
Gln Glu Met Arg Asn Val Ser Ile Gly Met Gly Ser Ser Asp Glu Trp
            295                 300                 305

tcc gat gtt cag gac att atc gac tcc acc cca gag ctg gat gtg tgt 1075
Ser Asp Val Gln Asp Ile Ile Asp Ser Thr Pro Glu Leu Asp Val Cys
        310                 315                 320

cct gaa acc cgt ctg gag cgc aca gga agc agc cca acc cag gga att 1123
Pro Glu Thr Arg Leu Glu Arg Thr Gly Ser Ser Pro Thr Gln Gly Ile
    325                 330                 335

gta aac aaa gct ttt gga atc aac act gac tcc ttg tat cac gaa ctc 1171
Val Asn Lys Ala Phe Gly Ile Asn Thr Asp Ser Leu Tyr His Glu Leu
340                 345                 350                 355

tcc acg gcg gga tct gag gtc atc ggg gat gtg gac gag gga gct gat 1219
Ser Thr Ala Gly Ser Glu Val Ile Gly Asp Val Asp Glu Gly Ala Asp
                360                 365                 370

ctc cta ggg gag ttt tca gtg cgc gat gat ttt ttt gga atg ggc aaa 1267
Leu Leu Gly Glu Phe Ser Val Arg Asp Asp Phe Phe Gly Met Gly Lys
            375                 380                 385

gaa gtg ggg aac ctg ctg ctg gag aac tca cag ctt cta gag aca aaa 1315
Glu Val Gly Asn Leu Leu Leu Glu Asn Ser Gln Leu Leu Glu Thr Lys
        390                 395                 400

aat gct tta aat gta gtg aag aat gac ctc att gct aag gtt gac caa 1363
Asn Ala Leu Asn Val Val Lys Asn Asp Leu Ile Ala Lys Val Asp Gln
    405                 410                 415

ctg tca gga gaa cag gag gtc ctg aag ggt gag ctg gaa gca gcc aag 1411
Leu Ser Gly Glu Gln Glu Val Leu Lys Gly Glu Leu Glu Ala Ala Lys
420                 425                 430                 435

caa gcg aaa gtc aag ctg gag aac cga atc aaa gag ctt gaa gaa gaa 1459
Gln Ala Lys Val Lys Leu Glu Asn Arg Ile Lys Glu Leu Glu Glu Glu
                440                 445                 450

ctg aag aga gtc aag tca gag gca gta act gcc cgc cgt gag ccc aga 1507
Leu Lys Arg Val Lys Ser Glu Ala Val Thr Ala Arg Arg Glu Pro Arg
            455                 460                 465

gaa gag gtg gag gat gta agc agc tat ctc tgt aca gaa ttg gac aaa 1555
Glu Glu Val Glu Asp Val Ser Ser Tyr Leu Cys Thr Glu Leu Asp Lys
        470                 475                 480

atc ccc atg gcc cag cgc cga cgc ttc aca cgg gtg gag atg gcc cga 1603
Ile Pro Met Ala Gln Arg Arg Arg Phe Thr Arg Val Glu Met Ala Arg

    485                 490                 495

gtg ctc atg gaa cgc aac cag tac aag gaa cgc ctc atg gag ctg cag 1651
Val Leu Met Glu Arg Asn Gln Tyr Lys Glu Arg Leu Met Glu Leu Gln
500                 505                 510                 515

gag gct gtg agg tgg act gaa atg atc aga gca tca agg gaa cac cca 1699
Glu Ala Val Arg Trp Thr Glu Met Ile Arg Ala Ser Arg Glu His Pro
                520                 525                 530

tct gtc cag gag aag aag aag tcc acc atc tgg cag ttc ttt agt cgc 1747
Ser Val Gln Glu Lys Lys Lys Ser Thr Ile Trp Gln Phe Phe Ser Arg
            535                 540                 545

ctc ttc agc tcc tca tct agc ccc cct ccg gcc aaa cga tcc tac cca 1795
Leu Phe Ser Ser Ser Ser Ser Pro Pro Pro Ala Lys Arg Ser Tyr Pro
        550                 555                 560

tct gtg aac att cac tac aag tca ccc act gca gct ggc ttt agc cag 1843
Ser Val Asn Ile His Tyr Lys Ser Pro Thr Ala Ala Gly Phe Ser Gln
    565                 570                 575

cgt cgc agc cat gct ttg tgc cag atc tca gcc ggc agc agg ccc ctg 1891
Arg Arg Ser His Ala Leu Cys Gln Ile Ser Ala Gly Ser Arg Pro Leu
580                 585                 590                 595

gag ttc ttc cct gat gat gac tgc acc tct tct gcc cgg cgg gag cag 1939
Glu Phe Phe Pro Asp Asp Asp Cys Thr Ser Ser Ala Arg Arg Glu Gln
                600                 605                 610

aag cgg gag cag tac cgc cag gtt cgt gaa cac gtg cgc aat gat gac 1987
Lys Arg Glu Gln Tyr Arg Gln Val Arg Glu His Val Arg Asn Asp Asp
            615                 620                 625

ggg agg ctg cag gcc tgt ggg tgg agc ctg cct gcc aag tac aag cag 2035
Gly Arg Leu Gln Ala Cys Gly Trp Ser Leu Pro Ala Lys Tyr Lys Gln
        630                 635                 640

ctg agc ccc aat gga ggc cag gaa gac acc cgg atg aaa aat gtg cct 2083
Leu Ser Pro Asn Gly Gly Gln Glu Asp Thr Arg Met Lys Asn Val Pro
    645                 650                 655

gtc cct gtg tac tgt cgc cct ctg gtg gag aag gac cct tcg aca aag 2131
Val Pro Val Tyr Cys Arg Pro Leu Val Glu Lys Asp Pro Ser Thr Lys
660                 665                 670                 675

ctg tgg tgt gct gct ggt gtc aac ctg agt ggg tgg aag cca cat gaa 2179
Leu Trp Cys Ala Ala Gly Val Asn Leu Ser Gly Trp Lys Pro His Glu
                680                 685                 690

gag gac tct agc aat gga ccc aag cct gta cca ggt cga gac cct ctg 2227
Glu Asp Ser Ser Asn Gly Pro Lys Pro Val Pro Gly Arg Asp Pro Leu

            695                 700                 705

acc tgt gac cgg gaa gga gaa ggc gaa ccc aag agc aca cac cca tca 2275
Thr Cys Asp Arg Glu Gly Glu Gly Glu Pro Lys Ser Thr His Pro Ser
        710                 715                 720

cct gag aag aag aag gca aag gaa acc cct gag gca gat gct acc tcc 2323
Pro Glu Lys Lys Lys Ala Lys Glu Thr Pro Glu Ala Asp Ala Thr Ser
    725                 730                 735

agt cgg gta tgg atc ctc acc agc acc ctg aca acc agc aag gtg gtg 2371
Ser Arg Val Trp Ile Leu Thr Ser Thr Leu Thr Thr Ser Lys Val Val
740                 745                 750                 755

atc att gat gcc aac cag cca ggc aca att gtg gat cag ttc aca gtc 2419
Ile Ile Asp Ala Asn Gln Pro Gly Thr Ile Val Asp Gln Phe Thr Val
                760                 765                 770

tgc aat gcc cac gtc ctg tgt atc tcc agc att cct gcg gcc agt gac 2467
Cys Asn Ala His Val Leu Cys Ile Ser Ser Ile Pro Ala Ala Ser Asp
            775                 780                 785

agt gac tat ccc cct ggg gag atg ttc cta gac agt gat gtg aac cct 2515
Ser Asp Tyr Pro Pro Gly Glu Met Phe Leu Asp Ser Asp Val Asn Pro
        790                 795                 800

gaa gat tca ggt gct gat ggt gtg ctg gct ggc atc acc ctg gtg ggg 2563
Glu Asp Ser Gly Ala Asp Gly Val Leu Ala Gly Ile Thr Leu Val Gly
    805                 810                 815

tgt gct acc cgc tgc aat gtt cca cgt agc aac tgt tcc tca cga gga 2611
Cys Ala Thr Arg Cys Asn Val Pro Arg Ser Asn Cys Ser Ser Arg Gly
820                 825                 830                 835

gac acc cca gta ctg gac aag ggg cag ggg gat gtg gcg acc act gcc 2659
Asp Thr Pro Val Leu Asp Lys Gly Gln Gly Asp Val Ala Thr Thr Ala
                840                 845                 850

aat ggg aag gtc aac ccg tcc caa tcc aca gaa gaa gcc aca gaa gcc 2707
Asn Gly Lys Val Asn Pro Ser Gln Ser Thr Glu Glu Ala Thr Glu Ala
            855                 860                 865

aca gag gtg cca gac cct ggt ccc agc gag tca gaa gca acg aca gtc 2755
Thr Glu Val Pro Asp Pro Gly Pro Ser Glu Ser Glu Ala Thr Thr Val
        870                 875                 880

cgg ccc ggg cct ctc aca gag cat gtc ttt act gac cca gca ccc acc 2803
Arg Pro Gly Pro Leu Thr Glu His Val Phe Thr Asp Pro Ala Pro Thr
    885                 890                 895

cca tcc tcc agc acc cag cct gcc agt gag aat ggg tca gaa tcc aat 2851
Pro Ser Ser Ser Thr Gln Pro Ala Ser Glu Asn Gly Ser Glu Ser Asn

900                 905                 910                 915

ggc acc att gta cag cct cag gtg gag ccc agt ggg gaa ctc tca aca    2899
Gly Thr Ile Val Gln Pro Gln Val Glu Pro Ser Gly Glu Leu Ser Thr
                920                 925                 930

aca acc agt agc gct gca ccc act atg tgg cta gga gcc cag aat ggc    2947
Thr Thr Ser Ser Ala Ala Pro Thr Met Trp Leu Gly Ala Gln Asn Gly
            935                 940                 945

tgg ctc tat gtg cat tca gcg gta gcc aac tgg aag aag tgt ctg cac    2995
Trp Leu Tyr Val His Ser Ala Val Ala Asn Trp Lys Lys Cys Leu His
        950                 955                 960

tcc atc aag cta aaa gac tct gtg ctg agc ctg gtg cat gtc aaa ggc    3043
Ser Ile Lys Leu Lys Asp Ser Val Leu Ser Leu Val His Val Lys Gly
    965                 970                 975

cga gtg ctg gta gct ctt gca gat ggg acc ctg gct atc ttc cat cgt    3091
Arg Val Leu Val Ala Leu Ala Asp Gly Thr Leu Ala Ile Phe His Arg
980                 985                 990                 995

gga gag gat ggc cag tgg gac ctg agc aac tac cac cta atg gac ctg    3139
Gly Glu Asp Gly Gln Trp Asp Leu Ser Asn Tyr His Leu Met Asp Leu
                1000                1005                1010

ggc cac cca cac cac tcc atc cgc tgc atg gct gtt gtg aat gac cga 3187
Gly His Pro His His Ser Ile Arg Cys Met Ala Val Val Asn Asp Arg
            1015                1020                1025

gtt tgg tgt ggc tac aag aac aag gtg cat gtt atc cag ccc aag aca 3235
Val Trp Cys Gly Tyr Lys Asn Lys Val His Val Ile Gln Pro Lys Thr
        1030                1035                1040

atg cag att gag aaa tca ttt gat gcc cac cca agg cgg gaa agc cag 3283
Met Gln Ile Glu Lys Ser Phe Asp Ala His Pro Arg Arg Glu Ser Gln
    1045                1050                1055

gta cgt cag ctg gcc tgg atc ggt gat gga gtg tgg gtc tct att cgc 3331
Val Arg Gln Leu Ala Trp Ile Gly Asp Gly Val Trp Val Ser Ile Arg
1060                1065                1070                1075

ttg gat tct acc ctt cgg ctc tac cat gct cac acc cac cag cac ctg 3379
Leu Asp Ser Thr Leu Arg Leu Tyr His Ala His Thr His Gln His Leu
                1080                1085                1090

cag gat gtg gac att gag ccc tat gtt agc aag atg cta gga acc ggc 3427
Gln Asp Val Asp Ile Glu Pro Tyr Val Ser Lys Met Leu Gly Thr Gly
            1095                1100                1105

aag ctg ggc ttc tcc ttc gtg cgc atc aca gcc tta ctc att gca ggc 3475
Lys Leu Gly Phe Ser Phe Val Arg Ile Thr Ala Leu Leu Ile Ala Gly

1110                    1115                    1120

aac cgt ctg tgg gtg ggc act ggc aat ggg gtt gtc atc tcc atc ccc    3523
Asn Arg Leu Trp Val Gly Thr Gly Asn Gly Val Val Ile Ser Ile Pro
    1125                    1130                    1135

ttg act gag act gtg gtc ctg cat cga ggc cag ctc cta ggg ctc cga    3571
Leu Thr Glu Thr Val Val Leu His Arg Gly Gln Leu Leu Gly Leu Arg
1140                    1145                    1150                    1155

gcc aac aag aca tcc cca aca tct ggg gag ggg acc cgc cca ggg ggc    3619
Ala Asn Lys Thr Ser Pro Thr Ser Gly Glu Gly Thr Arg Pro Gly Gly
                    1160                    1165                    1170

atc atc cat gtg tat ggg gac gac agc agt gac aag gcc gcc agt agt    3667
Ile Ile His Val Tyr Gly Asp Asp Ser Ser Asp Lys Ala Ala Ser Ser
                1175                    1180                    1185

ttc atc ccc tac tgc tcc atg gca cag gct cag ctt tgc ttc cat ggg    3715
Phe Ile Pro Tyr Cys Ser Met Ala Gln Ala Gln Leu Cys Phe His Gly
            1190                    1195                    1200

cac cgt gat gct gtc aaa ttc ttt gtc tct gtg cca gga aat gtg ctg    3763
His Arg Asp Ala Val Lys Phe Phe Val Ser Val Pro Gly Asn Val Leu
    1205                    1210                    1215

gcc act ctc aat ggc agt gtg cta gac agc cca tca gag ggc cct ggg 3811
Ala Thr Leu Asn Gly Ser Val Leu Asp Ser Pro Ser Glu Gly Pro Gly
1220                1225                1230                1235

cct gct gca ccc gct gca gat gct gag ggc cag aag ttg aag aat gca 3859
Pro Ala Ala Pro Ala Ala Asp Ala Glu Gly Gln Lys Leu Lys Asn Ala
                1240                1245                1250

ctg gtg ctg agt ggt ggt gaa ggt tac att gac ttc cgt atc gga gac 3907
Leu Val Leu Ser Gly Gly Glu Gly Tyr Ile Asp Phe Arg Ile Gly Asp
            1255                1260                1265

gga gag gat gat gaa act gag gaa tgt gcc ggg gac gtg aac cag aca 3955
Gly Glu Asp Asp Glu Thr Glu Glu Cys Ala Gly Asp Val Asn Gln Thr
        1270                1275                1280

aag ccc tcg ttg tcc aag gct gag cgc agc cac atc atc gtg tgg cag 4003
Lys Pro Ser Leu Ser Lys Ala Glu Arg Ser His Ile Ile Val Trp Gln
    1285                1290                1295

gtg tcc tac acc cct gag tgagaccctg tcctacctga tgccaactgt 4051
Val Ser Tyr Thr Pro Glu
1300                1305

acataggacc ctacctgcct gcctccccgc ctgttccctg gggcagccag gttcgtccat 4111

ccccttttaa cctctcaact tgcagctttt gcctgaggtc cagcccctag ctgttagaga 4171

gg 4173

<210> 2
<211> 4200
<212> DNA
<213> Mouse

<220>
<221> CDS
<222> (107)..(4045)

<400> 2
ggcctgggcg gcggcacatc ctaaggtagc ggctgcctga ggtgacagct gcccgtggat 60

tcgggccccg gaacgagccg cgctggcggc ggcggcggta gccgcg atg atg gag 115
Met Met Glu
1

atc cag atg gac gag gga gga ggt gtg gtg gtg tac caa gac gac tac 163
Ile Gln Met Asp Glu Gly Gly Gly Val Val Val Tyr Gln Asp Asp Tyr
    5                   10                  15

tgc tcg ggc tcg gtc atg tcg gag cgt gtg tcg ggc ctg gcg ggc tcc 211

Cys Ser Gly Ser Val Met Ser Glu Arg Val Ser Gly Leu Ala Gly Ser
20                  25                  30                  35

atc tac cgc gag ttc gag cgc ctc att cac tgc tat gac gag gag gtg    259
Ile Tyr Arg Glu Phe Glu Arg Leu Ile His Cys Tyr Asp Glu Glu Val
                40                  45                  50

gtc aag gag ctc atg ccg ctg gtg gtg aac gtg ctg gag aac ctt gac    307
Val Lys Glu Leu Met Pro Leu Val Val Asn Val Leu Glu Asn Leu Asp
            55                  60                  65

tcg gtg ctg agc gag aac cag gag cac gag gtg gag ctg gag ctc cta    355
Ser Val Leu Ser Glu Asn Gln Glu His Glu Val Glu Leu Glu Leu Leu
        70                  75                  80

cgc gag gac aac gag cag ctg ctc acg caa tac gag cgc gag aag gcg    403
Arg Glu Asp Asn Glu Gln Leu Leu Thr Gln Tyr Glu Arg Glu Lys Ala
    85                  90                  95

ctg cgc aaa cag gcc gag gag aaa ttc atc gaa ttt gaa gat gcc ttg    451
Leu Arg Lys Gln Ala Glu Glu Lys Phe Ile Glu Phe Glu Asp Ala Leu
100                 105                 110                 115

gaa caa gag aag aaa gaa ctc cag atc cag gta gaa cat tat gag ttt    499
Glu Gln Glu Lys Lys Glu Leu Gln Ile Gln Val Glu His Tyr Glu Phe
                120                 125                 130

cag aca cgc cag ctg gag cta aag gcc aaa aac tat gca gat cag att 547
Gln Thr Arg Gln Leu Glu Leu Lys Ala Lys Asn Tyr Ala Asp Gln Ile
            135                 140                 145

tcc cga ctg gag gaa cga gaa tcg gag atg aag aag gaa tac aat gcc 595
Ser Arg Leu Glu Glu Arg Glu Ser Glu Met Lys Lys Glu Tyr Asn Ala
        150                 155                 160

ctg cac cag cgg cac aca gag atg atc cag acc tat gtg gaa cac att 643
Leu His Gln Arg His Thr Glu Met Ile Gln Thr Tyr Val Glu His Ile
    165                 170                 175

gaa aga tcc aag atg cag caa gtt ggg ggt agc ggc caa aca gaa agc 691
Glu Arg Ser Lys Met Gln Gln Val Gly Gly Ser Gly Gln Thr Glu Ser
180                 185                 190                 195

agc ctg ccc ggg cgg agt cct cgc cag tcg tgg agg aaa agc agg aag 739
Ser Leu Pro Gly Arg Ser Pro Arg Gln Ser Trp Arg Lys Ser Arg Lys
                200                 205                 210

gag cgt ccc acc tct ctg aat gtc ttc ccc ctg gct gat ggc atg tgt 787
Glu Arg Pro Thr Ser Leu Asn Val Phe Pro Leu Ala Asp Gly Met Cys
            215                 220                 225

cca aac gat gag atg tct gag tca ggc cag tcc tca gca gct gca aca 835

Pro Asn Asp Glu Met Ser Glu Ser Gly Gln Ser Ser Ala Ala Ala Thr
        230                 235                 240

ccc agt acc aca ggt acc aag tcc aac aca ccc acg tcc tcc gtg ccc 883
Pro Ser Thr Thr Gly Thr Lys Ser Asn Thr Pro Thr Ser Ser Val Pro
    245                 250                 255

tca gca gca gtc acg cca ctc aac gag agc cta cag ccc ctg ggg gac 931
Ser Ala Ala Val Thr Pro Leu Asn Glu Ser Leu Gln Pro Leu Gly Asp
260                 265                 270                 275

tat gtc agt gtc aca aag aac aac aag cag gcc cga gag aag cgc aat 979
Tyr Val Ser Val Thr Lys Asn Asn Lys Gln Ala Arg Glu Lys Arg Asn
                280                 285                 290

agc cgt aac atg gag gtc cag gtc acc caa gag atg cgg aac gtc agt 1027
Ser Arg Asn Met Glu Val Gln Val Thr Gln Glu Met Arg Asn Val Ser
            295                 300                 305

atc ggc atg ggc agc agt gac gag tgg tcc gat gtt cag gac att atc 1075
Ile Gly Met Gly Ser Ser Asp Glu Trp Ser Asp Val Gln Asp Ile Ile
        310                 315                 320

gac tcc acc cca gag ctg gat gtg tgt cct gaa acc cgt ctg gag cgc 1123
Asp Ser Thr Pro Glu Leu Asp Val Cys Pro Glu Thr Arg Leu Glu Arg
    325                 330                 335

aca gga agc agc cca acc cag gga att gta aac aaa gct ttt gga atc 1171
Thr Gly Ser Ser Pro Thr Gln Gly Ile Val Asn Lys Ala Phe Gly Ile
340                 345                 350                 355

aac act gac tcc ttg tat cac gaa ctc tcc acg gcg gga tct gag gtc 1219
Asn Thr Asp Ser Leu Tyr His Glu Leu Ser Thr Ala Gly Ser Glu Val
                360                 365                 370

atc ggg gat gtg gac gag gga gct gat ctc cta ggg gag ttt tca gtg 1267
Ile Gly Asp Val Asp Glu Gly Ala Asp Leu Leu Gly Glu Phe Ser Val
            375                 380                 385

cgc gat gat ttt ttt gga atg ggc aaa gaa gtg ggg aac ctg ctg ctg 1315
Arg Asp Asp Phe Phe Gly Met Gly Lys Glu Val Gly Asn Leu Leu Leu
        390                 395                 400

gag aac tca cag ctt cta gag aca aaa aat gct tta aat gta gtg aag 1363
Glu Asn Ser Gln Leu Leu Glu Thr Lys Asn Ala Leu Asn Val Val Lys
    405                 410                 415

aat gac ctc att gct aag gtt gac caa ctg tca gga gaa cag gag gtc 1411
Asn Asp Leu Ile Ala Lys Val Asp Gln Leu Ser Gly Glu Gln Glu Val
420                 425                 430                 435

ctg aag ggt gag ctg gaa gca gcc aag caa gcg aaa gtc aag ctg gag 1459

Leu Lys Gly Glu Leu Glu Ala Ala Lys Gln Ala Lys Val Lys Leu Glu
                440                 445                 450

aac cga atc aaa gag ctt gaa gaa gaa ctg aag aga gtc aag tca gag 1507
Asn Arg Ile Lys Glu Leu Glu Glu Glu Leu Lys Arg Val Lys Ser Glu
            455                 460                 465

gca gta act gcc cgc cgt gag ccc aga gaa gag gtg gag gat gta agc 1555
Ala Val Thr Ala Arg Arg Glu Pro Arg Glu Glu Val Glu Asp Val Ser
        470                 475                 480

agc tat ctc tgt aca gaa ttg gac aaa atc ccc atg gcc cag cgc cga 1603
Ser Tyr Leu Cys Thr Glu Leu Asp Lys Ile Pro Met Ala Gln Arg Arg
    485                 490                 495

cgc ttc aca cgg gtg gag atg gcc cga gtg ctc atg gaa cgc aac cag 1651
Arg Phe Thr Arg Val Glu Met Ala Arg Val Leu Met Glu Arg Asn Gln
500                 505                 510                 515

tac aag gaa cgc ctc atg gag ctg cag gag gct gtg agg tgg act gaa 1699
Tyr Lys Glu Arg Leu Met Glu Leu Gln Glu Ala Val Arg Trp Thr Glu
                520                 525                 530

atg atc aga gca tca agg gaa cac cca tct gtc cag gag aag aag aag 1747
Met Ile Arg Ala Ser Arg Glu His Pro Ser Val Gln Glu Lys Lys Lys
            535                 540                 545

tcc acc atc tgg cag ttc ttt agt cgc ctc ttc agc tcc tca tct agc 1795
Ser Thr Ile Trp Gln Phe Phe Ser Arg Leu Phe Ser Ser Ser Ser Ser
        550                 555                 560

ccc cct ccg gcc aaa cga tcc tac cca tct gtg aac att cac tac aag 1843
Pro Pro Pro Ala Lys Arg Ser Tyr Pro Ser Val Asn Ile His Tyr Lys
    565                 570                 575

tca ccc act gca gct ggc ttt agc cag cgt cgc agc cat gct ttg tgc 1891
Ser Pro Thr Ala Ala Gly Phe Ser Gln Arg Arg Ser His Ala Leu Cys
580                 585                 590                 595

cag atc tca gcc ggc agc agg ccc ctg gag ttc ttc cct gat gat gac 1939
Gln Ile Ser Ala Gly Ser Arg Pro Leu Glu Phe Phe Pro Asp Asp Asp
                600                 605                 610

tgc acc tct tct gcc cgg cgg gag cag aag cgg gag cag tac cgc cag 1987
Cys Thr Ser Ser Ala Arg Arg Glu Gln Lys Arg Glu Gln Tyr Arg Gln
            615                 620                 625

gtt cgt gaa cac gtg cgc aat gat gac ggg agg ctg cag gcc tgt ggg 2035
Val Arg Glu His Val Arg Asn Asp Asp Gly Arg Leu Gln Ala Cys Gly
        630                 635                 640

tgg agc ctg cct gcc aag tac aag cag ctg agc ccc aat gga ggc cag 2083

Trp Ser Leu Pro Ala Lys Tyr Lys Gln Leu Ser Pro Asn Gly Gly Gln
    645                 650                 655

gaa gac acc cgg atg aaa aat gtg cct gtc cct gtg tac tgt cgc cct 2131
Glu Asp Thr Arg Met Lys Asn Val Pro Val Pro Val Tyr Cys Arg Pro
660                 665                 670                 675

ctg gtg gag aag gac cct tcg aca aag ctg tgg tgt gct gct ggt gtc 2179
Leu Val Glu Lys Asp Pro Ser Thr Lys Leu Trp Cys Ala Ala Gly Val
                680                 685                 690

aac ctg agt ggg tgg aag cca cat gaa gag gac tct agc aat gga ccc 2227
Asn Leu Ser Gly Trp Lys Pro His Glu Glu Asp Ser Ser Asn Gly Pro
            695                 700                 705

aag cct gta cca ggt cga gac cct ctg acc tgt gac cgg gaa gga gaa 2275
Lys Pro Val Pro Gly Arg Asp Pro Leu Thr Cys Asp Arg Glu Gly Glu
        710                 715                 720

ggc gaa ccc aag agc aca cac cca tca cct gag aag aag aag gca aag 2323
Gly Glu Pro Lys Ser Thr His Pro Ser Pro Glu Lys Lys Lys Ala Lys
    725                 730                 735

gaa acc cct gag gca gat gct acc tcc agt cgg gta tgg atc ctc acc 2371
Glu Thr Pro Glu Ala Asp Ala Thr Ser Ser Arg Val Trp Ile Leu Thr
740                 745                 750                 755

agc acc ctg aca acc agc aag gtg gtg atc att gat gcc aac cag cca 2419
Ser Thr Leu Thr Thr Ser Lys Val Val Ile Ile Asp Ala Asn Gln Pro
                760                 765                 770

ggc aca att gtg gat cag ttc aca gtc tgc aat gcc cac gtc ctg tgt 2467
Gly Thr Ile Val Asp Gln Phe Thr Val Cys Asn Ala His Val Leu Cys
            775                 780                 785

atc tcc agc att cct gcg gcc agt gac agt gac tat ccc cct ggg gag 2515
Ile Ser Ser Ile Pro Ala Ala Ser Asp Ser Asp Tyr Pro Pro Gly Glu
        790                 795                 800

atg ttc cta gac agt gat gtg aac cct gaa gat tca ggt gct gat ggt 2563
Met Phe Leu Asp Ser Asp Val Asn Pro Glu Asp Ser Gly Ala Asp Gly
    805                 810                 815

gtg ctg gct ggc atc acc ctg gtg ggg tgt gct acc cgc tgc aat gtt 2611
Val Leu Ala Gly Ile Thr Leu Val Gly Cys Ala Thr Arg Cys Asn Val
820                 825                 830                 835

cca cgt agc aac tgt tcc tca cga gga gac acc cca gta ctg gac aag 2659
Pro Arg Ser Asn Cys Ser Ser Arg Gly Asp Thr Pro Val Leu Asp Lys
                840                 845                 850

ggg cag ggg gat gtg gcg acc act gcc aat ggg aag gtc aac ccg tcc 2707

Gly Gln Gly Asp Val Ala Thr Thr Ala Asn Gly Lys Val Asn Pro Ser
            855                 860                 865

caa tcc aca gaa gaa gcc aca gaa gcc aca gag gtg cca gac cct ggt 2755
Gln Ser Thr Glu Glu Ala Thr Glu Ala Thr Glu Val Pro Asp Pro Gly
        870                 875                 880

ccc agc gag tca gaa gca acg aca gtc cgg ccc ggg cct ctc aca gag 2803
Pro Ser Glu Ser Glu Ala Thr Thr Val Arg Pro Gly Pro Leu Thr Glu
    885                 890                 895

cat gtc ttt act gac cca gca ccc acc cca tcc tcc agc acc cag cct 2851
His Val Phe Thr Asp Pro Ala Pro Thr Pro Ser Ser Ser Thr Gln Pro
900                 905                 910                 915

gcc agt gag aat ggg tca gaa tcc aat ggc acc att gta cag cct cag 2899
Ala Ser Glu Asn Gly Ser Glu Ser Asn Gly Thr Ile Val Gln Pro Gln
                920                 925                 930

gtg gag ccc agt ggg gaa ctc tca aca aca acc agt agc gct gca ccc 2947
Val Glu Pro Ser Gly Glu Leu Ser Thr Thr Thr Ser Ser Ala Ala Pro
            935                 940                 945

act atg tgg cta gga gcc cag aat ggc tgg ctc tat gtg cat tca gcg 2995
Thr Met Trp Leu Gly Ala Gln Asn Gly Trp Leu Tyr Val His Ser Ala
        950                 955                 960

gta gcc aac tgg aag aag tgt ctg cac tcc atc aag cta aaa gac tct 3043
Val Ala Asn Trp Lys Lys Cys Leu His Ser Ile Lys Leu Lys Asp Ser
    965                 970                 975

gtg ctg agc ctg gtg cat gtc aaa ggc cga gtg ctg gta gct ctt gca 3091
Val Leu Ser Leu Val His Val Lys Gly Arg Val Leu Val Ala Leu Ala
980                 985                 990                 995

gat ggg acc ctg gct atc ttc cat cgt gga gag gat ggc cag tgg gac 3139
Asp Gly Thr Leu Ala Ile Phe His Arg Gly Glu Asp Gly Gln Trp Asp
                1000                1005                1010

ctg agc aac tac cac cta atg gac ctg ggc cac cca cac cac tcc atc 3187
Leu Ser Asn Tyr His Leu Met Asp Leu Gly His Pro His His Ser Ile
            1015                1020                1025

cgc tgc atg gct gtt gtg aat gac cga gtt tgg tgt ggc tac aag aac 3235
Arg Cys Met Ala Val Val Asn Asp Arg Val Trp Cys Gly Tyr Lys Asn
        1030                1035                1040

aag gtg cat gtt atc cag ccc aag aca atg cag att gag aaa tca ttt 3283
Lys Val His Val Ile Gln Pro Lys Thr Met Gln Ile Glu Lys Ser Phe
    1045                1050                1055

gat gcc cac cca agg cgg gaa agc cag gta cgt cag ctg gcc tgg atc 3331

Asp Ala His Pro Arg Arg Glu Ser Gln Val Arg Gln Leu Ala Trp Ile
1060                1065                1070                1075

ggt gat gga gtg tgg gtc tct att cgc ttg gat tct acc ctt cgg ctc    3379
Gly Asp Gly Val Trp Val Ser Ile Arg Leu Asp Ser Thr Leu Arg Leu
                1080                1085                1090

tac cat gct cac acc cac cag cac ctg cag gat gtg gac att gag ccc    3427
Tyr His Ala His Thr His Gln His Leu Gln Asp Val Asp Ile Glu Pro
            1095                1100                1105

tat gtt agc aag atg cta gga acc ggc aag ctg ggc ttc tcc ttc gtg    3475
Tyr Val Ser Lys Met Leu Gly Thr Gly Lys Leu Gly Phe Ser Phe Val
        1110                1115                1120

cgc atc aca gcc tta ctc att gca ggc aac cgt ctg tgg gtg ggc act    3523
Arg Ile Thr Ala Leu Leu Ile Ala Gly Asn Arg Leu Trp Val Gly Thr
    1125                1130                1135

ggc aat ggg gtt gtc atc tcc atc ccc ttg act gag act gtg gtc ctg    3571
Gly Asn Gly Val Val Ile Ser Ile Pro Leu Thr Glu Thr Val Val Leu
1140                1145                1150                1155

cat cga ggc cag ctc cta ggg ctc cga gcc aac aag aca tcc cca aca    3619
His Arg Gly Gln Leu Leu Gly Leu Arg Ala Asn Lys Thr Ser Pro Thr
                1160                1165                1170

tct ggg gag ggg acc cgc cca ggg ggc atc atc cat gtg tat ggg gac 3667
Ser Gly Glu Gly Thr Arg Pro Gly Gly Ile Ile His Val Tyr Gly Asp
            1175                1180                1185

gac agc agt gac aag gcc gcc agt agt ttc atc ccc tac tgc tcc atg 3715
Asp Ser Ser Asp Lys Ala Ala Ser Ser Phe Ile Pro Tyr Cys Ser Met
        1190                1195                1200

gca cag gct cag ctt tgc ttc cat ggg cac cgt gat gct gtc aaa ttc 3763
Ala Gln Ala Gln Leu Cys Phe His Gly His Arg Asp Ala Val Lys Phe
    1205                1210                1215

ttt gtc tct gtg cca gga aat gtg ctg gcc act ctc aat ggc agt gtg 3811
Phe Val Ser Val Pro Gly Asn Val Leu Ala Thr Leu Asn Gly Ser Val
1220                1225                1230                1235

cta gac agc cca tca gag ggc cct ggg cct gct gca ccc gct gca gat 3859
Leu Asp Ser Pro Ser Glu Gly Pro Gly Pro Ala Ala Pro Ala Ala Asp
                1240                1245                1250

gct gag ggc cag aag ttg aag aat gca ctg gtg ctg agt ggt ggt gaa 3907
Ala Glu Gly Gln Lys Leu Lys Asn Ala Leu Val Leu Ser Gly Gly Glu
            1255                1260                1265

ggt tac att gac ttc cgt atc gga gac gga gag gat gat gaa act gag 3955

Gly Tyr Ile Asp Phe Arg Ile Gly Asp Gly Glu Asp Asp Glu Thr Glu
        1270                1275                1280

gaa tgt gcc ggg gac gtg aac cag aca aag ccc tcg ttg tcc aag gct 4003
Glu Cys Ala Gly Asp Val Asn Gln Thr Lys Pro Ser Leu Ser Lys Ala
    1285                1290                1295

gag cgc agc cac atc atc gtg tgg cag gtg tcc tac acc cct gag 4043
Glu Arg Ser His Ile Ile Val Trp Gln Val Ser Tyr Thr Pro Glu
1300                1305                1310

tgagaccctg tcctacctga tgccaactgt acataggacc ctacctgcct gcctccccgc 4102

ctgttccctg gggcagccag gttcgtccat ccccttttaa cctctcaact tgcagctttt 4162

gcctgaggtc cagcccctag ctgttagaga gg 4200

<210> 3
<211> 4269
<212> DNA
<213> Mouse

<220>
<221> CDS
<222> (107)..(4117)

<400> 3
   ggcctgggcg gcggcacatc ctaaggtagc ggctgcctga ggtgacagct gcccgtggat 60
   tcgggccccg gaacgagccg cgctggcggc ggcggcggta gccgcg atg atg gag 115
   Met Met Glu
   1
   atc cag atg gac gag gga gga ggt gtg gtg gtg tac caa gac gac tac 163
   Ile Gln Met Asp Glu Gly Gly Gly Val Val Val Tyr Gln Asp Asp Tyr
   5 10 15
   tgc tcg ggc tcg gtc atg tcg gag cgt gtg tcg ggc ctg gcg ggc tcc 211
   Cys Ser Gly Ser Val Met Ser Glu Arg Val Ser Gly Leu Ala Gly Ser
   20 25 30 35
   atc tac cgc gag ttc gag cgc ctc att cac tgc tat gac gag gag gtg 259
   Ile Tyr Arg Glu Phe Glu Arg Leu Ile His Cys Tyr Asp Glu Glu Val
   40 45 50
   gtc aag gag ctc atg ccg ctg gtg gtg aac gtg ctg gag aac ctt gac 307
   Val Lys Glu Leu Met Pro Leu Val Val Asn Val Leu Glu Asn Leu Asp
   55 60 65
   tcg gtg ctg agc gag aac cag gag cac gag gtg gag ctg gag ctc cta 355
   Ser Val Leu Ser Glu Asn Gln Glu His Glu Val Glu Leu Glu Leu Leu
   70 75 80
   cgc gag gac aac gag cag ctg ctc acg caa tac gag cgc gag aag gcg 403
   Arg Glu Asp Asn Glu Gln Leu Leu Thr Gln Tyr Glu Arg Glu Lys Ala
   85 90 95
   ctg cgc aaa cag gcc gag gag aaa ttc atc gaa ttt gaa gat gcc ttg 451
   Leu Arg Lys Gln Ala Glu Glu Lys Phe Ile Glu Phe Glu Asp Ala Leu
   100 105 110 115
   gaa caa gag aag aaa gaa ctc cag atc cag gta gaa cat tat gag ttt 499
   Glu Gln Glu Lys Lys Glu Leu Gln Ile Gln Val Glu His Tyr Glu Phe
   120 125 130
   cag aca cgc cag ctg gag cta aag gcc aaa aac tat gca gat cag att 547
   Gln Thr Arg Gln Leu Glu Leu Lys Ala Lys Asn Tyr Ala Asp Gln Ile
   135 140 145
   tcc cga ctg gag gaa cga gaa tcg gag atg aag aag gaa tac aat gcc 595
   Ser Arg Leu Glu Glu Arg Glu Ser Glu Met Lys Lys Glu Tyr Asn Ala
   150 155 160
   ctg cac cag cgg cac aca gag atg atc cag acc tat gtg gaa cac att 643
   Leu His Gln Arg His Thr Glu Met Ile Gln Thr Tyr Val Glu His Ile
   165 170 175
   gaa aga tcc aag atg cag caa gtt ggg ggt agc ggc caa aca gaa agc 691
   Glu Arg Ser Lys Met Gln Gln Val Gly Gly Ser Gly Gln Thr Glu Ser
   180 185 190 195
   agc ctg ccc ggg cgg agc agg aag gag cgt ccc acc tct ctg aat gtc 739
   Ser Leu Pro Gly Arg Ser Arg Lys Glu Arg Pro Thr Ser Leu Asn Val
   200 205 210
   ttc ccc ctg gct gat ggc atg gta cgt gca cag atg ggg ggc aag ctc 787
   Phe Pro Leu Ala Asp Gly Met Val Arg Ala Gln Met Gly Gly Lys Leu
   215 220 225
   gtg cct gcg ggg gac cac tgg cac ctg agt gac ctc ggc cag cta cag 835
   Val Pro Ala Gly Asp His Trp His Leu Ser Asp Leu Gly Gln Leu Gln
   230 235 240
   tcc agc tcc agc tac cag tgt cca aac gat gag atg tct gag tca ggc 883
   Ser Ser Ser Ser Tyr Gln Cys Pro Asn Asp Glu Met Ser Glu Ser Gly
   245 250 255
   cag tcc tca gca gct gca aca ccc agt acc aca ggt acc aag tcc aac 931
   Gln Ser Ser Ala Ala Ala Thr Pro Ser Thr Thr Gly Thr Lys Ser Asn
   260 265 270 275
   aca ccc acg tcc tcc gtg ccc tca gca gca gtc acg cca ctc aac gag 979
   Thr Pro Thr Ser Ser Val Pro Ser Ala Ala Val Thr Pro Leu Asn Glu
   280 285 290
   agc cta cag ccc ctg ggg gac tat gtc agt gtc aca aag aac aac aag 1027
   Ser Leu Gln Pro Leu Gly Asp Tyr Val Ser Val Thr Lys Asn Asn Lys
   295 300 305
   cag gcc cga gag aag cgc aat agc cgt aac atg gag gtc cag gtc acc 1075
   Gln Ala Arg Glu Lys Arg Asn Ser Arg Asn Met Glu Val Gln Val Thr
   310 315 320
   caa gag atg cgg aac gtc agt atc ggc atg ggc agc agt gac gag tgg 1123
   Gln Glu Met Arg Asn Val Ser Ile Gly Met Gly Ser Ser Asp Glu Trp
   325 330 335
   tcc gat gtt cag gac att atc gac tcc acc cca gag ctg gat gtg tgt 1171
   Ser Asp Val Gln Asp Ile Ile Asp Ser Thr Pro Glu Leu Asp Val Cys
   340 345 350 355
   cct gaa acc cgt ctg gag cgc aca gga agc agc cca acc cag gga att 1219
   Pro Glu Thr Arg Leu Glu Arg Thr Gly Ser Ser Pro Thr Gln Gly Ile
   360 365 370
   gta aac aaa gct ttt gga atc aac act gac tcc ttg tat cac gaa ctc 1267
   Val Asn Lys Ala Phe Gly Ile Asn Thr Asp Ser Leu Tyr His Glu Leu
   375 380 385
   tcc acg gcg gga tct gag gtc atc ggg gat gtg gac gag gga gct gat 1315
   Ser Thr Ala Gly Ser Glu Val Ile Gly Asp Val Asp Glu Gly Ala Asp
   390 395 400
   ctc cta ggg gag ttt tca gtg cgc gat gat ttt ttt gga atg ggc aaa 1363
   Leu Leu Gly Glu Phe Ser Val Arg Asp Asp Phe Phe Gly Met Gly Lys
   405 410 415
   gaa gtg ggg aac ctg ctg ctg gag aac tca cag ctt cta gag aca aaa 1411
   Glu Val Gly Asn Leu Leu Leu Glu Asn Ser Gln Leu Leu Glu Thr Lys
   420 425 430 435
   aat gct tta aat gta gtg aag aat gac ctc att gct aag gtt gac caa 1459
   Asn Ala Leu Asn Val Val Lys Asn Asp Leu Ile Ala Lys Val Asp Gln
   440 445 450
   ctg tca gga gaa cag gag gtc ctg aag ggt gag ctg gaa gca gcc aag 1507
   Leu Ser Gly Glu Gln Glu Val Leu Lys Gly Glu Leu Glu Ala Ala Lys
   455 460 465
   caa gcg aaa gtc aag ctg gag aac cga atc aaa gag ctt gaa gaa gaa 1555
   Gln Ala Lys Val Lys Leu Glu Asn Arg Ile Lys Glu Leu Glu Glu Glu
   470 475 480
   ctg aag aga gtc aag tca gag gca gta act gcc cgc cgt gag ccc aga 1603
   Leu Lys Arg Val Lys Ser Glu Ala Val Thr Ala Arg Arg Glu Pro Arg
   485 490 495
   gaa gag gtg gag gat gta agc agc tat ctc tgt aca gaa ttg gac aaa 1651
   Glu Glu Val Glu Asp Val Ser Ser Tyr Leu Cys Thr Glu Leu Asp Lys
   500 505 510 515
   atc ccc atg gcc cag cgc cga cgc ttc aca cgg gtg gag atg gcc cga 1699
   Ile Pro Met Ala Gln Arg Arg Arg Phe Thr Arg Val Glu Met Ala Arg
   520 525 530
   gtg ctc atg gaa cgc aac cag tac aag gaa cgc ctc atg gag ctg cag 1747
   Val Leu Met Glu Arg Asn Gln Tyr Lys Glu Arg Leu Met Glu Leu Gln
   535 540 545
   gag gct gtg agg tgg act gaa atg atc aga gca tca agg gaa cac cca 1795
   Glu Ala Val Arg Trp Thr Glu Met Ile Arg Ala Ser Arg Glu His Pro
   550 555 560
   tct gtc cag gag aag aag aag tcc acc atc tgg cag ttc ttt agt cgc 1843
   Ser Val Gln Glu Lys Lys Lys Ser Thr Ile Trp Gln Phe Phe Ser Arg
   565 570 575
   ctc ttc agc tcc tca tct agc ccc cct ccg gcc aaa cga tcc tac cca 1891
   Leu Phe Ser Ser Ser Ser Ser Pro Pro Pro Ala Lys Arg Ser Tyr Pro
   580 585 590 595
   tct gtg aac att cac tac aag tca ccc act gca gct ggc ttt agc cag 1939
   Ser Val Asn Ile His Tyr Lys Ser Pro Thr Ala Ala Gly Phe Ser Gln
   600 605 610
   cgt cgc agc cat gct ttg tgc cag atc tca gcc ggc agc agg ccc ctg 1987
   Arg Arg Ser His Ala Leu Cys Gln Ile Ser Ala Gly Ser Arg Pro Leu
   615 620 625
   gag ttc ttc cct gat gat gac tgc acc tct tct gcc cgg cgg gag cag 2035
   Glu Phe Phe Pro Asp Asp Asp Cys Thr Ser Ser Ala Arg Arg Glu Gln
   630 635 640
   aag cgg gag cag tac cgc cag gtt cgt gaa cac gtg cgc aat gat gac 2083
   Lys Arg Glu Gln Tyr Arg Gln Val Arg Glu His Val Arg Asn Asp Asp
   645 650 655
   ggg agg ctg cag gcc tgt ggg tgg agc ctg cct gcc aag tac aag cag 2131
   Gly Arg Leu Gln Ala Cys Gly Trp Ser Leu Pro Ala Lys Tyr Lys Gln
   660 665 670 675
   ctg agc ccc aat gga ggc cag gaa gac acc cgg atg aaa aat gtg cct 2179
   Leu Ser Pro Asn Gly Gly Gln Glu Asp Thr Arg Met Lys Asn Val Pro
   680 685 690
   gtc cct gtg tac tgt cgc cct ctg gtg gag aag gac cct tcg aca aag 2227
   Val Pro Val Tyr Cys Arg Pro Leu Val Glu Lys Asp Pro Ser Thr Lys
   695 700 705
   ctg tgg tgt gct gct ggt gtc aac ctg agt ggg tgg aag cca cat gaa 2275
   Leu Trp Cys Ala Ala Gly Val Asn Leu Ser Gly Trp Lys Pro His Glu
   710 715 720
   gag gac tct agc aat gga ccc aag cct gta cca ggt cga gac cct ctg 2323
   Glu Asp Ser Ser Asn Gly Pro Lys Pro Val Pro Gly Arg Asp Pro Leu
   725 730 735
   acc tgt gac cgg gaa gga gaa ggc gaa ccc aag agc aca cac cca tca 2371
   Thr Cys Asp Arg Glu Gly Glu Gly Glu Pro Lys Ser Thr His Pro Ser
   740 745 750 755
   cct gag aag aag aag gca aag gaa acc cct gag gca gat gct acc tcc 2419
   Pro Glu Lys Lys Lys Ala Lys Glu Thr Pro Glu Ala Asp Ala Thr Ser
   760 765 770
   agt cgg gta tgg atc ctc acc agc acc ctg aca acc agc aag gtg gtg 2467
   Ser Arg Val Trp Ile Leu Thr Ser Thr Leu Thr Thr Ser Lys Val Val
   775 780 785
   atc att gat gcc aac cag cca ggc aca att gtg gat cag ttc aca gtc 2515
   Ile Ile Asp Ala Asn Gln Pro Gly Thr Ile Val Asp Gln Phe Thr Val
   790 795 800
   tgc aat gcc cac gtc ctg tgt atc tcc agc att cct gcg gcc agt gac 2563
   Cys Asn Ala His Val Leu Cys Ile Ser Ser Ile Pro Ala Ala Ser Asp
   805 810 815
   agt gac tat ccc cct ggg gag atg ttc cta gac agt gat gtg aac cct 2611
   Ser Asp Tyr Pro Pro Gly Glu Met Phe Leu Asp Ser Asp Val Asn Pro
   820 825 830 835
   gaa gat tca ggt gct gat ggt gtg ctg gct ggc atc acc ctg gtg ggg 2659
   Glu Asp Ser Gly Ala Asp Gly Val Leu Ala Gly Ile Thr Leu Val Gly
   840 845 850
   tgt gct acc cgc tgc aat gtt cca cgt agc aac tgt tcc tca cga gga 2707
   Cys Ala Thr Arg Cys Asn Val Pro Arg Ser Asn Cys Ser Ser Arg Gly
   855 860 865
   gac acc cca gta ctg gac aag ggg cag ggg gat gtg gcg acc act gcc 2755
   Asp Thr Pro Val Leu Asp Lys Gly Gln Gly Asp Val Ala Thr Thr Ala
   870 875 880
   aat ggg aag gtc aac ccg tcc caa tcc aca gaa gaa gcc aca gaa gcc 2803
   Asn Gly Lys Val Asn Pro Ser Gln Ser Thr Glu Glu Ala Thr Glu Ala
   885 890 895
   aca gag gtg cca gac cct ggt ccc agc gag tca gaa gca acg aca gtc 2851
   Thr Glu Val Pro Asp Pro Gly Pro Ser Glu Ser Glu Ala Thr Thr Val
   900                 905                 910                 915
   cgg ccc ggg cct ctc aca gag cat gtc ttt act gac cca gca ccc acc    2899
   Arg Pro Gly Pro Leu Thr Glu His Val Phe Thr Asp Pro Ala Pro Thr
                   920                 925                 930
   cca tcc tcc agc acc cag cct gcc agt gag aat ggg tca gaa tcc aat    2947
   Pro Ser Ser Ser Thr Gln Pro Ala Ser Glu Asn Gly Ser Glu Ser Asn
               935                 940                 945
   ggc acc att gta cag cct cag gtg gag ccc agt ggg gaa ctc tca aca    2995
   Gly Thr Ile Val Gln Pro Gln Val Glu Pro Ser Gly Glu Leu Ser Thr
           950                 955                 960
   aca acc agt agc gct gca ccc act atg tgg cta gga gcc cag aat ggc    3043
   Thr Thr Ser Ser Ala Ala Pro Thr Met Trp Leu Gly Ala Gln Asn Gly
       965                 970                 975
   tgg ctc tat gtg cat tca gcg gta gcc aac tgg aag aag tgt ctg cac    3091
   Trp Leu Tyr Val His Ser Ala Val Ala Asn Trp Lys Lys Cys Leu His
   980                 985                 990                 995
   tcc atc aag cta aaa gac tct gtg ctg agc ctg gtg cat gtc aaa ggc    3139
   Ser Ile Lys Leu Lys Asp Ser Val Leu Ser Leu Val His Val Lys Gly
                   1000                1005                1010
   cga gtg ctg gta gct ctt gca gat ggg acc ctg gct atc ttc cat cgt 3187
   Arg Val Leu Val Ala Leu Ala Asp Gly Thr Leu Ala Ile Phe His Arg
   1015 1020 1025
   gga gag gat ggc cag tgg gac ctg agc aac tac cac cta atg gac ctg 3235
   Gly Glu Asp Gly Gln Trp Asp Leu Ser Asn Tyr His Leu Met Asp Leu
   1030 1035 1040
   ggc cac cca cac cac tcc atc cgc tgc atg gct gtt gtg aat gac cga 3283
   Gly His Pro His His Ser Ile Arg Cys Met Ala Val Val Asn Asp Arg
   1045 1050 1055
   gtt tgg tgt ggc tac aag aac aag gtg cat gtt atc cag ccc aag aca 3331
   Val Trp Cys Gly Tyr Lys Asn Lys Val His Val Ile Gln Pro Lys Thr
   1060 1065 1070 1075
   atg cag att gag aaa tca ttt gat gcc cac cca agg cgg gaa agc cag 3379
   Met Gln Ile Glu Lys Ser Phe Asp Ala His Pro Arg Arg Glu Ser Gln
   1080 1085 1090
   gta cgt cag ctg gcc tgg atc ggt gat gga gtg tgg gtc tct att cgc 3427
   Val Arg Gln Leu Ala Trp Ile Gly Asp Gly Val Trp Val Ser Ile Arg
   1095 1100 1105
   ttg gat tct acc ctt cgg ctc tac cat gct cac acc cac cag cac ctg 3475
   Leu Asp Ser Thr Leu Arg Leu Tyr His Ala His Thr His Gln His Leu
   1110                1115                1120
   cag gat gtg gac att gag ccc tat gtt agc aag atg cta gga acc ggc    3523
   Gln Asp Val Asp Ile Glu Pro Tyr Val Ser Lys Met Leu Gly Thr Gly
       1125                1130                1135
   aag ctg ggc ttc tcc ttc gtg cgc atc aca gcc tta ctc att gca ggc    3571
   Lys Leu Gly Phe Ser Phe Val Arg Ile Thr Ala Leu Leu Ile Ala Gly
   1140                1145                1150                1155
   aac cgt ctg tgg gtg ggc act ggc aat ggg gtt gtc atc tcc atc ccc    3619
   Asn Arg Leu Trp Val Gly Thr Gly Asn Gly Val Val Ile Ser Ile Pro
                   1160                1165                1170
   ttg act gag act gtg gtc ctg cat cga ggc cag ctc cta ggg ctc cga    3667
   Leu Thr Glu Thr Val Val Leu His Arg Gly Gln Leu Leu Gly Leu Arg
               1175                1180                1185
   gcc aac aag aca tcc cca aca tct ggg gag ggg acc cgc cca ggg ggc    3715
   Ala Asn Lys Thr Ser Pro Thr Ser Gly Glu Gly Thr Arg Pro Gly Gly
           1190                1195                1200
   atc atc cat gtg tat ggg gac gac agc agt gac aag gcc gcc agt agt    3763
   Ile Ile His Val Tyr Gly Asp Asp Ser Ser Asp Lys Ala Ala Ser Ser
       1205                1210                1215
   ttc atc ccc tac tgc tcc atg gca cag gct cag ctt tgc ttc cat ggg 3811
   Phe Ile Pro Tyr Cys Ser Met Ala Gln Ala Gln Leu Cys Phe His Gly
   1220 1225 1230 1235
   cac cgt gat gct gtc aaa ttc ttt gtc tct gtg cca gga aat gtg ctg 3859
   His Arg Asp Ala Val Lys Phe Phe Val Ser Val Pro Gly Asn Val Leu
   1240 1245 1250
   gcc act ctc aat ggc agt gtg cta gac agc cca tca gag ggc cct ggg 3907
   Ala Thr Leu Asn Gly Ser Val Leu Asp Ser Pro Ser Glu Gly Pro Gly
   1255 1260 1265
   cct gct gca ccc gct gca gat gct gag ggc cag aag ttg aag aat gca 3955
   Pro Ala Ala Pro Ala Ala Asp Ala Glu Gly Gln Lys Leu Lys Asn Ala
   1270 1275 1280
   ctg gtg ctg agt ggt ggt gaa ggt tac att gac ttc cgt atc gga gac 4003
   Leu Val Leu Ser Gly Gly Glu Gly Tyr Ile Asp Phe Arg Ile Gly Asp
   1285 1290 1295
   gga gag gat gat gaa act gag gaa tgt gcc ggg gac gtg aac cag aca 4051
   Gly Glu Asp Asp Glu Thr Glu Glu Cys Ala Gly Asp Val Asn Gln Thr
   1300 1305 1310 1315
   aag ccc tcg ttg tcc aag gct gag cgc agc cac atc atc gtg tgg cag 4099
   Lys Pro Ser Leu Ser Lys Ala Glu Arg Ser His Ile Ile Val Trp Gln
   1320                1325                1330
   gtg tcc tac acc cct gag tgagaccctg tcctacctga tgccaactgt          4147
   Val Ser Tyr Thr Pro Glu
           1335
   acataggacc ctacctgcct gcctccccgc ctgttccctg gggcagccag gttcgtccat 4207
   ccccttttaa cctctcaact tgcagctttt gcctgaggtc cagcccctag ctgttagaga 4267
   gg                                                                4269

<210> 4
<211> 4266
<212> DNA
<213> Mouse

<220>
<221> CDS
<222> (107)..(4114)

<400> 4
ggcctgggcg gcggcacatc ctaaggtagc ggctgcctga ggtgacagct gcccgtggat 60

tcgggccccg gaacgagccg cgctggcggc ggcggcggta gccgcg atg atg gag    115

Met Met Glu
1
atc cag atg gac gag gga gga ggt gtg gtg gtg tac caa gac gac tac 163
Ile Gln Met Asp Glu Gly Gly Gly Val Val Val Tyr Gln Asp Asp Tyr
    5                   10                  15
tgc tcg ggc tcg gtc atg tcg gag cgt gtg tcg ggc ctg gcg ggc tcc 211
Cys Ser Gly Ser Val Met Ser Glu Arg Val Ser Gly Leu Ala Gly Ser
20                  25                  30                  35
atc tac cgc gag ttc gag cgc ctc att cac tgc tat gac gag gag gtg 259
Ile Tyr Arg Glu Phe Glu Arg Leu Ile His Cys Tyr Asp Glu Glu Val
                40                  45                  50
gtc aag gag ctc atg ccg ctg gtg gtg aac gtg ctg gag aac ctt gac 307
Val Lys Glu Leu Met Pro Leu Val Val Asn Val Leu Glu Asn Leu Asp
            55                  60                  65
tcg gtg ctg agc gag aac cag gag cac gag gtg gag ctg gag ctc cta 355
Ser Val Leu Ser Glu Asn Gln Glu His Glu Val Glu Leu Glu Leu Leu
        70                  75                  80
cgc gag gac aac gag cag ctg ctc acg caa tac gag cgc gag aag gcg 403
Arg Glu Asp Asn Glu Gln Leu Leu Thr Gln Tyr Glu Arg Glu Lys Ala
    85                  90                  95

ctg cgc aaa cag gcc gag gag aaa ttc atc gaa ttt gaa gat gcc ttg 451
Leu Arg Lys Gln Ala Glu Glu Lys Phe Ile Glu Phe Glu Asp Ala Leu
100                 105                 110                 115

gaa caa gag aag aaa gaa ctc cag atc cag gta gaa cat tat gag ttt 499
Glu Gln Glu Lys Lys Glu Leu Gln Ile Gln Val Glu His Tyr Glu Phe
                120                 125                 130

cag aca cgc cag ctg gag cta aag gcc aaa aac tat gca gat cag att 547
Gln Thr Arg Gln Leu Glu Leu Lys Ala Lys Asn Tyr Ala Asp Gln Ile
            135                 140                 145

tcc cga ctg gag gaa cga gaa tcg gag atg aag aag gaa tac aat gcc 595
Ser Arg Leu Glu Glu Arg Glu Ser Glu Met Lys Lys Glu Tyr Asn Ala
        150                 155                 160

ctg cac cag cgg cac aca gag atg atc cag acc tat gtg gaa cac att 643
Leu His Gln Arg His Thr Glu Met Ile Gln Thr Tyr Val Glu His Ile
    165                 170                 175

gaa aga tcc aag atg cag caa gtt ggg ggt agc ggc caa aca gaa agc 691
Glu Arg Ser Lys Met Gln Gln Val Gly Gly Ser Gly Gln Thr Glu Ser
180                 185                 190                 195

agc ctg ccc ggg cgg agg aag gag cgt ccc acc tct ctg aat gtc ttc 739

Ser Leu Pro Gly Arg Arg Lys Glu Arg Pro Thr Ser Leu Asn Val Phe
                200                 205                 210

ccc ctg gct gat ggc atg gta cgt gca cag atg ggg ggc aag ctc gtg 787
Pro Leu Ala Asp Gly Met Val Arg Ala Gln Met Gly Gly Lys Leu Val
            215                 220                 225

cct gcg ggg gac cac tgg cac ctg agt gac ctc ggc cag cta cag tcc 835
Pro Ala Gly Asp His Trp His Leu Ser Asp Leu Gly Gln Leu Gln Ser
        230                 235                 240

agc tcc agc tac cag tgt cca aac gat gag atg tct gag tca ggc cag 883
Ser Ser Ser Tyr Gln Cys Pro Asn Asp Glu Met Ser Glu Ser Gly Gln
    245                 250                 255

tcc tca gca gct gca aca ccc agt acc aca ggt acc aag tcc aac aca 931
Ser Ser Ala Ala Ala Thr Pro Ser Thr Thr Gly Thr Lys Ser Asn Thr
260                 265                 270                 275

ccc acg tcc tcc gtg ccc tca gca gca gtc acg cca ctc aac gag agc 979
Pro Thr Ser Ser Val Pro Ser Ala Ala Val Thr Pro Leu Asn Glu Ser
                280                 285                 290

cta cag ccc ctg ggg gac tat gtc agt gtc aca aag aac aac aag cag 1027
Leu Gln Pro Leu Gly Asp Tyr Val Ser Val Thr Lys Asn Asn Lys Gln
            295                 300                 305

gcc cga gag aag cgc aat agc cgt aac atg gag gtc cag gtc acc caa 1075
Ala Arg Glu Lys Arg Asn Ser Arg Asn Met Glu Val Gln Val Thr Gln
        310                 315                 320

gag atg cgg aac gtc agt atc ggc atg ggc agc agt gac gag tgg tcc 1123
Glu Met Arg Asn Val Ser Ile Gly Met Gly Ser Ser Asp Glu Trp Ser
    325                 330                 335

gat gtt cag gac att atc gac tcc acc cca gag ctg gat gtg tgt cct 1171
Asp Val Gln Asp Ile Ile Asp Ser Thr Pro Glu Leu Asp Val Cys Pro
340                 345                 350                 355

gaa acc cgt ctg gag cgc aca gga agc agc cca acc cag gga att gta 1219
Glu Thr Arg Leu Glu Arg Thr Gly Ser Ser Pro Thr Gln Gly Ile Val
                360                 365                 370

aac aaa gct ttt gga atc aac act gac tcc ttg tat cac gaa ctc tcc 1267
Asn Lys Ala Phe Gly Ile Asn Thr Asp Ser Leu Tyr His Glu Leu Ser
            375                 380                 385

acg gcg gga tct gag gtc atc ggg gat gtg gac gag gga gct gat ctc 1315
Thr Ala Gly Ser Glu Val Ile Gly Asp Val Asp Glu Gly Ala Asp Leu
        390                 395                 400

cta ggg gag ttt tca gtg cgc gat gat ttt ttt gga atg ggc aaa gaa 1363

Leu Gly Glu Phe Ser Val Arg Asp Asp Phe Phe Gly Met Gly Lys Glu
    405                 410                 415

gtg ggg aac ctg ctg ctg gag aac tca cag ctt cta gag aca aaa aat 1411
Val Gly Asn Leu Leu Leu Glu Asn Ser Gln Leu Leu Glu Thr Lys Asn
420                 425                 430                 435

gct tta aat gta gtg aag aat gac ctc att gct aag gtt gac caa ctg 1459
Ala Leu Asn Val Val Lys Asn Asp Leu Ile Ala Lys Val Asp Gln Leu
                440                 445                 450

tca gga gaa cag gag gtc ctg aag ggt gag ctg gaa gca gcc aag caa 1507
Ser Gly Glu Gln Glu Val Leu Lys Gly Glu Leu Glu Ala Ala Lys Gln
            455                 460                 465

gcg aaa gtc aag ctg gag aac cga atc aaa gag ctt gaa gaa gaa ctg 1555
Ala Lys Val Lys Leu Glu Asn Arg Ile Lys Glu Leu Glu Glu Glu Leu
        470                 475                 480

aag aga gtc aag tca gag gca gta act gcc cgc cgt gag ccc aga gaa 1603
Lys Arg Val Lys Ser Glu Ala Val Thr Ala Arg Arg Glu Pro Arg Glu
    485                 490                 495

gag gtg gag gat gta agc agc tat ctc tgt aca gaa ttg gac aaa atc 1651
Glu Val Glu Asp Val Ser Ser Tyr Leu Cys Thr Glu Leu Asp Lys Ile
500                 505                 510                 515

ccc atg gcc cag cgc cga cgc ttc aca cgg gtg gag atg gcc cga gtg 1699
Pro Met Ala Gln Arg Arg Arg Phe Thr Arg Val Glu Met Ala Arg Val
                520                 525                 530

ctc atg gaa cgc aac cag tac aag gaa cgc ctc atg gag ctg cag gag 1747
Leu Met Glu Arg Asn Gln Tyr Lys Glu Arg Leu Met Glu Leu Gln Glu
            535                 540                 545

gct gtg agg tgg act gaa atg atc aga gca tca agg gaa cac cca tct 1795
Ala Val Arg Trp Thr Glu Met Ile Arg Ala Ser Arg Glu His Pro Ser
        550                 555                 560

gtc cag gag aag aag aag tcc acc atc tgg cag ttc ttt agt cgc ctc 1843
Val Gln Glu Lys Lys Lys Ser Thr Ile Trp Gln Phe Phe Ser Arg Leu
    565                 570                 575

ttc agc tcc tca tct agc ccc cct ccg gcc aaa cga tcc tac cca tct 1891
Phe Ser Ser Ser Ser Ser Pro Pro Pro Ala Lys Arg Ser Tyr Pro Ser
580                 585                 590                 595

gtg aac att cac tac aag tca ccc act gca gct ggc ttt agc cag cgt 1939
Val Asn Ile His Tyr Lys Ser Pro Thr Ala Ala Gly Phe Ser Gln Arg
                600                 605                 610

cgc agc cat gct ttg tgc cag atc tca gcc ggc agc agg ccc ctg gag 1987

Arg Ser His Ala Leu Cys Gln Ile Ser Ala Gly Ser Arg Pro Leu Glu
            615                 620                 625

ttc ttc cct gat gat gac tgc acc tct tct gcc cgg cgg gag cag aag 2035
Phe Phe Pro Asp Asp Asp Cys Thr Ser Ser Ala Arg Arg Glu Gln Lys
        630                 635                 640

cgg gag cag tac cgc cag gtt cgt gaa cac gtg cgc aat gat gac ggg 2083
Arg Glu Gln Tyr Arg Gln Val Arg Glu His Val Arg Asn Asp Asp Gly
    645                 650                 655

agg ctg cag gcc tgt ggg tgg agc ctg cct gcc aag tac aag cag ctg 2131
Arg Leu Gln Ala Cys Gly Trp Ser Leu Pro Ala Lys Tyr Lys Gln Leu
660                 665                 670                 675

agc ccc aat gga ggc cag gaa gac acc cgg atg aaa aat gtg cct gtc 2179
Ser Pro Asn Gly Gly Gln Glu Asp Thr Arg Met Lys Asn Val Pro Val
                680                 685                 690

cct gtg tac tgt cgc cct ctg gtg gag aag gac cct tcg aca aag ctg 2227
Pro Val Tyr Cys Arg Pro Leu Val Glu Lys Asp Pro Ser Thr Lys Leu
            695                 700                 705

tgg tgt gct gct ggt gtc aac ctg agt ggg tgg aag cca cat gaa gag 2275
Trp Cys Ala Ala Gly Val Asn Leu Ser Gly Trp Lys Pro His Glu Glu
        710                 715                 720

gac tct agc aat gga ccc aag cct gta cca ggt cga gac cct ctg acc 2323
Asp Ser Ser Asn Gly Pro Lys Pro Val Pro Gly Arg Asp Pro Leu Thr
    725                 730                 735

tgt gac cgg gaa gga gaa ggc gaa ccc aag agc aca cac cca tca cct 2371
Cys Asp Arg Glu Gly Glu Gly Glu Pro Lys Ser Thr His Pro Ser Pro
740                 745                 750                 755

gag aag aag aag gca aag gaa acc cct gag gca gat gct acc tcc agt 2419
Glu Lys Lys Lys Ala Lys Glu Thr Pro Glu Ala Asp Ala Thr Ser Ser
                760                 765                 770

cgg gta tgg atc ctc acc agc acc ctg aca acc agc aag gtg gtg atc 2467
Arg Val Trp Ile Leu Thr Ser Thr Leu Thr Thr Ser Lys Val Val Ile
            775                 780                 785

att gat gcc aac cag cca ggc aca att gtg gat cag ttc aca gtc tgc 2515
Ile Asp Ala Asn Gln Pro Gly Thr Ile Val Asp Gln Phe Thr Val Cys
        790                 795                 800

aat gcc cac gtc ctg tgt atc tcc agc att cct gcg gcc agt gac agt 2563
Asn Ala His Val Leu Cys Ile Ser Ser Ile Pro Ala Ala Ser Asp Ser
    805                 810                 815

gac tat ccc cct ggg gag atg ttc cta gac agt gat gtg aac cct gaa 2611

Asp Tyr Pro Pro Gly Glu Met Phe Leu Asp Ser Asp Val Asn Pro Glu
820                 825                 830                 835

gat tca ggt gct gat ggt gtg ctg gct ggc atc acc ctg gtg ggg tgt 2659
Asp Ser Gly Ala Asp Gly Val Leu Ala Gly Ile Thr Leu Val Gly Cys
                840                 845                 850

gct acc cgc tgc aat gtt cca cgt agc aac tgt tcc tca cga gga gac 2707
Ala Thr Arg Cys Asn Val Pro Arg Ser Asn Cys Ser Ser Arg Gly Asp
            855                 860                 865

acc cca gta ctg gac aag ggg cag ggg gat gtg gcg acc act gcc aat 2755
Thr Pro Val Leu Asp Lys Gly Gln Gly Asp Val Ala Thr Thr Ala Asn
        870                 875                 880

ggg aag gtc aac ccg tcc caa tcc aca gaa gaa gcc aca gaa gcc aca 2803
Gly Lys Val Asn Pro Ser Gln Ser Thr Glu Glu Ala Thr Glu Ala Thr
    885                 890                 895

gag gtg cca gac cct ggt ccc agc gag tca gaa gca acg aca gtc cgg 2851
Glu Val Pro Asp Pro Gly Pro Ser Glu Ser Glu Ala Thr Thr Val Arg
900                 905                 910                 915

ccc ggg cct ctc aca gag cat gtc ttt act gac cca gca ccc acc cca 2899
Pro Gly Pro Leu Thr Glu His Val Phe Thr Asp Pro Ala Pro Thr Pro
                920                 925                 930

tcc tcc agc acc cag cct gcc agt gag aat ggg tca gaa tcc aat ggc 2947
Ser Ser Ser Thr Gln Pro Ala Ser Glu Asn Gly Ser Glu Ser Asn Gly
            935                 940                 945

acc att gta cag cct cag gtg gag ccc agt ggg gaa ctc tca aca aca 2995
Thr Ile Val Gln Pro Gln Val Glu Pro Ser Gly Glu Leu Ser Thr Thr
        950                 955                 960

acc agt agc gct gca ccc act atg tgg cta gga gcc cag aat ggc tgg 3043
Thr Ser Ser Ala Ala Pro Thr Met Trp Leu Gly Ala Gln Asn Gly Trp
    965                 970                 975

ctc tat gtg cat tca gcg gta gcc aac tgg aag aag tgt ctg cac tcc 3091
Leu Tyr Val His Ser Ala Val Ala Asn Trp Lys Lys Cys Leu His Ser
980                 985                 990                 995

atc aag cta aaa gac tct gtg ctg agc ctg gtg cat gtc aaa ggc cga 3139
Ile Lys Leu Lys Asp Ser Val Leu Ser Leu Val His Val Lys Gly Arg
                1000                1005                1010

gtg ctg gta gct ctt gca gat ggg acc ctg gct atc ttc cat cgt gga 3187
Val Leu Val Ala Leu Ala Asp Gly Thr Leu Ala Ile Phe His Arg Gly
            1015                1020                1025

gag gat ggc cag tgg gac ctg agc aac tac cac cta atg gac ctg ggc 3235

Glu Asp Gly Gln Trp Asp Leu Ser Asn Tyr His Leu Met Asp Leu Gly
        1030                1035                1040

cac cca cac cac tcc atc cgc tgc atg gct gtt gtg aat gac cga gtt 3283
His Pro His His Ser Ile Arg Cys Met Ala Val Val Asn Asp Arg Val
    1045                1050                1055

tgg tgt ggc tac aag aac aag gtg cat gtt atc cag ccc aag aca atg 3331
Trp Cys Gly Tyr Lys Asn Lys Val His Val Ile Gln Pro Lys Thr Met
1060                1065                1070                1075

cag att gag aaa tca ttt gat gcc cac cca agg cgg gaa agc cag gta 3379
Gln Ile Glu Lys Ser Phe Asp Ala His Pro Arg Arg Glu Ser Gln Val
                1080                1085                1090

cgt cag ctg gcc tgg atc ggt gat gga gtg tgg gtc tct att cgc ttg 3427
Arg Gln Leu Ala Trp Ile Gly Asp Gly Val Trp Val Ser Ile Arg Leu
            1095                1100                1105

gat tct acc ctt cgg ctc tac cat gct cac acc cac cag cac ctg cag 3475
Asp Ser Thr Leu Arg Leu Tyr His Ala His Thr His Gln His Leu Gln
        1110                1115                1120

gat gtg gac att gag ccc tat gtt agc aag atg cta gga acc ggc aag 3523
Asp Val Asp Ile Glu Pro Tyr Val Ser Lys Met Leu Gly Thr Gly Lys
    1125                1130                1135

ctg ggc ttc tcc ttc gtg cgc atc aca gcc tta ctc att gca ggc aac 3571
Leu Gly Phe Ser Phe Val Arg Ile Thr Ala Leu Leu Ile Ala Gly Asn
1140                1145                1150                1155

cgt ctg tgg gtg ggc act ggc aat ggg gtt gtc atc tcc atc ccc ttg 3619
Arg Leu Trp Val Gly Thr Gly Asn Gly Val Val Ile Ser Ile Pro Leu
                1160                1165                1170

act gag act gtg gtc ctg cat cga ggc cag ctc cta ggg ctc cga gcc 3667
Thr Glu Thr Val Val Leu His Arg Gly Gln Leu Leu Gly Leu Arg Ala
            1175                1180                1185

aac aag aca tcc cca aca tct ggg gag ggg acc cgc cca ggg ggc atc 3715
Asn Lys Thr Ser Pro Thr Ser Gly Glu Gly Thr Arg Pro Gly Gly Ile
        1190                1195                1200

atc cat gtg tat ggg gac gac agc agt gac aag gcc gcc agt agt ttc 3763
Ile His Val Tyr Gly Asp Asp Ser Ser Asp Lys Ala Ala Ser Ser Phe
    1205                1210                1215

atc ccc tac tgc tcc atg gca cag gct cag ctt tgc ttc cat ggg cac 3811
Ile Pro Tyr Cys Ser Met Ala Gln Ala Gln Leu Cys Phe His Gly His
1220                1225                1230                1235

cgt gat gct gtc aaa ttc ttt gtc tct gtg cca gga aat gtg ctg gcc 3859

Arg Asp Ala Val Lys Phe Phe Val Ser Val Pro Gly Asn Val Leu Ala
                1240                1245                1250

act ctc aat ggc agt gtg cta gac agc cca tca gag ggc cct ggg cct 3907
Thr Leu Asn Gly Ser Val Leu Asp Ser Pro Ser Glu Gly Pro Gly Pro
            1255                1260                1265

gct gca ccc gct gca gat gct gag ggc cag aag ttg aag aat gca ctg 3955
Ala Ala Pro Ala Ala Asp Ala Glu Gly Gln Lys Leu Lys Asn Ala Leu
        1270                1275                1280

gtg ctg agt ggt ggt gaa ggt tac att gac ttc cgt atc gga gac gga 4003
Val Leu Ser Gly Gly Glu Gly Tyr Ile Asp Phe Arg Ile Gly Asp Gly
    1285                1290                1295

gag gat gat gaa act gag gaa tgt gcc ggg gac gtg aac cag aca aag 4051
Glu Asp Asp Glu Thr Glu Glu Cys Ala Gly Asp Val Asn Gln Thr Lys
1300                1305                1310                1315

ccc tcg ttg tcc aag gct gag cgc agc cac atc atc gtg tgg cag gtg 4099
Pro Ser Leu Ser Lys Ala Glu Arg Ser His Ile Ile Val Trp Gln Val
                1320                1325                1330

tcc tac acc cct gag tgagaccctg tcctacctga tgccaactgt acataggacc 4154
Ser Tyr Thr Pro Glu
            1335

ctacctgcct gcctccccgc ctgttccctg gggcagccag gttcgtccat ccccttttaa 4214

cctctcaact tgcagctttt gcctgaggtc cagcccctag ctgttagaga gg 4266

<210> 5
<211> 1450
<212> DNA
<213> Mouse

<220>
<221> CDS
<222> (41)..(1330)

<400> 5
ggcgaattca tggtcgtgga aaccccgccc caattaagca atg tca ggc gtc cga 55
Met Ser Gly Val Arg
1               5

cct ccc atc atg aac ggg ccc atg cac ccc cgg ccc ctg gtg gcg ctg 103
Pro Pro Ile Met Asn Gly Pro Met His Pro Arg Pro Leu Val Ala Leu
                10                  15                  20

ctg gat ggc cgg gac tgc aca gtg gag atg cct atc ctg aag gat gtg 151
Leu Asp Gly Arg Asp Cys Thr Val Glu Met Pro Ile Leu Lys Asp Val

            25                  30                  35

gcc aca gta gcc ttc tgt gat gca cag tcc aca cag gag atc cat gag 199
Ala Thr Val Ala Phe Cys Asp Ala Gln Ser Thr Gln Glu Ile His Glu
        40                  45                  50

aag gta ctg aat gag gct gtg ggt gcc ctg atg tac cat acc atc aca 247
Lys Val Leu Asn Glu Ala Val Gly Ala Leu Met Tyr His Thr Ile Thr
    55                  60                  65

ctg acc aga gaa gat ctg gag aag ttt aaa gct ctt aga atc atc gtc 295
Leu Thr Arg Glu Asp Leu Glu Lys Phe Lys Ala Leu Arg Ile Ile Val
70                  75                  80                  85

cga att ggc agc ggg ttt gac aat atc gac atc aag tca gct ggg gat 343
Arg Ile Gly Ser Gly Phe Asp Asn Ile Asp Ile Lys Ser Ala Gly Asp
                90                  95                  100

cta ggc atc gca gtg tgc aat gtg ccg gca gca tct gtg gaa gaa acg 391
Leu Gly Ile Ala Val Cys Asn Val Pro Ala Ala Ser Val Glu Glu Thr
            105                 110                 115

gca gac tcc acc ctg tgc cac atc ctg aac ctg tac cga cga acc acc 439
Ala Asp Ser Thr Leu Cys His Ile Leu Asn Leu Tyr Arg Arg Thr Thr
        120                 125                 130

tgg cta cac cag gcc ctt cgg gaa ggc act cga gtc cag agt gta gag 487
Trp Leu His Gln Ala Leu Arg Glu Gly Thr Arg Val Gln Ser Val Glu
    135                 140                 145

cag atc cga gag gtg gct tca gga gct gcc agg atc cgt gga gag acc 535
Gln Ile Arg Glu Val Ala Ser Gly Ala Ala Arg Ile Arg Gly Glu Thr
150                 155                 160                 165

ttg ggc atc att gga cta ggt cgt gtg ggc cag gcg gtg gca ctt cgg 583
Leu Gly Ile Ile Gly Leu Gly Arg Val Gly Gln Ala Val Ala Leu Arg
                170                 175                 180

gca aag gct ttt ggc ttc aac gtc ctc ttc tat gat cca tac cta tct 631
Ala Lys Ala Phe Gly Phe Asn Val Leu Phe Tyr Asp Pro Tyr Leu Ser
            185                 190                 195

gat gga atc gag cgg gcc ctg ggg cta cag cgc gtg agc acg ctg cag 679
Asp Gly Ile Glu Arg Ala Leu Gly Leu Gln Arg Val Ser Thr Leu Gln
        200                 205                 210

gac ctg ctc ttc cac agt gac tgc gtt acc ctg cat tgc ggc ctc aat 727
Asp Leu Leu Phe His Ser Asp Cys Val Thr Leu His Cys Gly Leu Asn
    215                 220                 225

gag cac aac cac cac ctc atc aat gac ttt act gtc aag cag atg aga 775
Glu His Asn His His Leu Ile Asn Asp Phe Thr Val Lys Gln Met Arg

230                 235                 240                 245

caa gga gcc ttt ctg gtg aac aca gcc cgt ggt ggc ctg gtg gat gag    823
Gln Gly Ala Phe Leu Val Asn Thr Ala Arg Gly Gly Leu Val Asp Glu
                250                 255                 260

aag gca gtg gcc cag gcc ctg aag gaa ggg cgg atc cgt ggc gca gcg    871
Lys Ala Val Ala Gln Ala Leu Lys Glu Gly Arg Ile Arg Gly Ala Ala
            265                 270                 275

ctg gac gtg cat gag tca gag ccc ttc agc ttt agc cag gga ccc tta    919
Leu Asp Val His Glu Ser Glu Pro Phe Ser Phe Ser Gln Gly Pro Leu
        280                 285                 290

aag gat gca ccc aac ctc atc tgc aca ccc cat gct gca tgg tac agt    967
Lys Asp Ala Pro Asn Leu Ile Cys Thr Pro His Ala Ala Trp Tyr Ser
    295                 300                 305

gag cag gcg tcc att gag atg aga gag gag gca gcc cgg gaa atc cgg    1015
Glu Gln Ala Ser Ile Glu Met Arg Glu Glu Ala Ala Arg Glu Ile Arg
310                 315                 320                 325

cga gcc atc aca ggc cgg atc cca gat agc ttg aaa aac tgt gtc aac    1063
Arg Ala Ile Thr Gly Arg Ile Pro Asp Ser Leu Lys Asn Cys Val Asn
                330                 335                 340

aag gac cac ctg aca gcc gcc acg cac tgg gcc agc atg gac cct gct 1111
Lys Asp His Leu Thr Ala Ala Thr His Trp Ala Ser Met Asp Pro Ala
            345                 350                 355

gtg gtg cac cct gag ctc aat ggg gct gcc tac agc agg tac cct cca 1159
Val Val His Pro Glu Leu Asn Gly Ala Ala Tyr Ser Arg Tyr Pro Pro
        360                 365                 370

ggc gtc gtg agt gtg gcc ccc act ggc atc cca gct gct gtg gaa ggg 1207
Gly Val Val Ser Val Ala Pro Thr Gly Ile Pro Ala Ala Val Glu Gly
    375                 380                 385

att gtt ccc agt gcc atg tcc ctg tct cat ggc ctg ccc cct gtg gcc 1255
Ile Val Pro Ser Ala Met Ser Leu Ser His Gly Leu Pro Pro Val Ala
390                 395                 400                 405

cac cca ccc cac gct ccc tct cct ggc cag act gtc aag cct gag gcg 1303
His Pro Pro His Ala Pro Ser Pro Gly Gln Thr Val Lys Pro Glu Ala
                410                 415                 420

gat aga gac cat acg agt gac cag ttg tagcctgtga ggagctgtcc 1350
Asp Arg Asp His Thr Ser Asp Gln Leu
            425                 430

agccttggca cctggtggag ggtgctgcac gctcttggac ccaagtgtgc agaggtggca 1410

tccagtgtgg gccctagcac tccagagact ggcccgggcg 1450

<210> 6
<211> 4684
<212> DNA
<213> Mouse

<220>
<221> CDS
<222> (150)..(4673)

<400> 6
gaagacacct actgtgatga taaggttgct tctgccatct caggccagtg aaagtgcata 60

tccgaagcct gtgttgaacg agaaggcagt accgctgttg agacaagtcc aaaggctagg 120

ccagggactg tccttagggg ctcctcgtt atg atg gct gga gaa ggg tca acc 173
Met Met Ala Gly Glu Gly Ser Thr
1               5

att act agc cgt atc aag aac ttg ctg agg tct cca tcc atc aaa tta 221
Ile Thr Ser Arg Ile Lys Asn Leu Leu Arg Ser Pro Ser Ile Lys Leu
    10                  15                  20

cgc aga agt aaa gca gga aac cgg aga gag gac ctc agc tcc aag gta 269

Arg Arg Ser Lys Ala Gly Asn Arg Arg Glu Asp Leu Ser Ser Lys Val
25                  30                  35                  40

acc ttg gag aag gtc ctg gga gtg aca gta tct gga gga aga gga ctt    317
Thr Leu Glu Lys Val Leu Gly Val Thr Val Ser Gly Gly Arg Gly Leu
                45                  50                  55

gct tgt gag ccc cga tct ggc tta gtt gcc tac cca gca ggg tgt gtg    365
Ala Cys Glu Pro Arg Ser Gly Leu Val Ala Tyr Pro Ala Gly Cys Val
            60                  65                  70

gtc gtc ctc ttc aat ccc cgg aag cac aaa cag cac cac atc ctc aac    413
Val Val Leu Phe Asn Pro Arg Lys His Lys Gln His His Ile Leu Asn
        75                  80                  85

agc tcc agg aaa acc att act gcc ctt gcc ttc tcc cct gat ggc aag    461
Ser Ser Arg Lys Thr Ile Thr Ala Leu Ala Phe Ser Pro Asp Gly Lys
    90                  95                  100

tac ttg gtc act gga gag agt ggg cac atg cct gcc gtg cgg gtt tgg    509
Tyr Leu Val Thr Gly Glu Ser Gly His Met Pro Ala Val Arg Val Trp
105                 110                 115                 120

gat gtg gct gaa cgt agc cag gtg gca gag cta cag gag cat aag tat    557
Asp Val Ala Glu Arg Ser Gln Val Ala Glu Leu Gln Glu His Lys Tyr
                125                 130                 135

ggt gtg gct tgt gtg gct ttc tcc cca agt gcc aag tac att gtg tct 605
Gly Val Ala Cys Val Ala Phe Ser Pro Ser Ala Lys Tyr Ile Val Ser
            140                 145                 150

gtg ggc tac cag cat gac atg att gtc aac gtg tgg gcc tgg aag aaa 653
Val Gly Tyr Gln His Asp Met Ile Val Asn Val Trp Ala Trp Lys Lys
        155                 160                 165

aac att gta gtg gcc tcc aac aaa gta tcc agt cgg gta acc gca gtg 701
Asn Ile Val Val Ala Ser Asn Lys Val Ser Ser Arg Val Thr Ala Val
    170                 175                 180

tcc ttt tct gaa gac tgc agc tac ttt gtc act gca ggc aac cgg cac 749
Ser Phe Ser Glu Asp Cys Ser Tyr Phe Val Thr Ala Gly Asn Arg His
185                 190                 195                 200

atc aaa ttc tgg tac ctg gat gac agt aag acc tca aag gtg aac gcc 797
Ile Lys Phe Trp Tyr Leu Asp Asp Ser Lys Thr Ser Lys Val Asn Ala
                205                 210                 215

act gtg ccc ctg ctg ggc cgc tcg ggg ctg ctg ggg gag ctg agg aac 845
Thr Val Pro Leu Leu Gly Arg Ser Gly Leu Leu Gly Glu Leu Arg Asn
            220                 225                 230

aac ctg ttc act gat gtg gcc tgt ggc cga ggg gaa aag gct gat agc 893

Asn Leu Phe Thr Asp Val Ala Cys Gly Arg Gly Glu Lys Ala Asp Ser
        235                 240                 245

act ttc tgt atc acg tcg tcg ggg ctg ctg tgc gag ttc agc gat cgc 941
Thr Phe Cys Ile Thr Ser Ser Gly Leu Leu Cys Glu Phe Ser Asp Arg
    250                 255                 260

agg ctt ctg gac aaa tgg gtg gag cta agg aac aca gac agc ttc aca 989
Arg Leu Leu Asp Lys Trp Val Glu Leu Arg Asn Thr Asp Ser Phe Thr
265                 270                 275                 280

acc act gtg gcc cac tgc atc tct gtc acc caa gaa tac atc ttc tgt 1037
Thr Thr Val Ala His Cys Ile Ser Val Thr Gln Glu Tyr Ile Phe Cys
                285                 290                 295

ggc tgt gct gat ggc acg gtg cgc ctt ttc aat cct tcc aac ctg cac 1085
Gly Cys Ala Asp Gly Thr Val Arg Leu Phe Asn Pro Ser Asn Leu His
            300                 305                 310

ttc ctc agt acc cta ccc aga ccc cat gct ctt gga aca gac att gcc 1133
Phe Leu Ser Thr Leu Pro Arg Pro His Ala Leu Gly Thr Asp Ile Ala
        315                 320                 325

agc atc act gag gcc agt cgc ctc ttt tct gga ggg gtc aat gca agg 1181
Ser Ile Thr Glu Ala Ser Arg Leu Phe Ser Gly Gly Val Asn Ala Arg
    330                 335                 340

tac cca gac acc att gcc ttg acc ttc gat cca act aat cag tgg cta    1229
Tyr Pro Asp Thr Ile Ala Leu Thr Phe Asp Pro Thr Asn Gln Trp Leu
345                 350                 355                 360

tct tgt gta tac aac gac cac agc ata tat gtt tgg gat gtg agg gac    1277
Ser Cys Val Tyr Asn Asp His Ser Ile Tyr Val Trp Asp Val Arg Asp
                365                 370                 375

ccc aag aaa gtg ggg aag gtg tac tcc gct ctg tat cac tcc tcc tgt    1325
Pro Lys Lys Val Gly Lys Val Tyr Ser Ala Leu Tyr His Ser Ser Cys
            380                 385                 390

gtc tgg agt gtg gag gtc tac cct gag atc aag gac agt cac cag gcc    1373
Val Trp Ser Val Glu Val Tyr Pro Glu Ile Lys Asp Ser His Gln Ala
        395                 400                 405

tgt ctt ccc ctc agt tcc ttt att act tgc tcc tca gac aac acc atc    1421
Cys Leu Pro Leu Ser Ser Phe Ile Thr Cys Ser Ser Asp Asn Thr Ile
    410                 415                 420

cgc ctg tgg aac aca gag agc tct ggg gta cat ggc tct acc ctg cac    1469
Arg Leu Trp Asn Thr Glu Ser Ser Gly Val His Gly Ser Thr Leu His
425                 430                 435                 440

cgt aac atc ctc agc aat gat ctc att aag atc atc tat gtg gat ggg    1517

Arg Asn Ile Leu Ser Asn Asp Leu Ile Lys Ile Ile Tyr Val Asp Gly
                445                 450                 455

aac act cag gct ttg ttg gac act gag ctg cct gga gga gac aaa gct 1565
Asn Thr Gln Ala Leu Leu Asp Thr Glu Leu Pro Gly Gly Asp Lys Ala
            460                 465                 470

gat ggg tcg ctg atg gac ccc cga gtg ggc atc cgg tcc gtg tgt att 1613
Asp Gly Ser Leu Met Asp Pro Arg Val Gly Ile Arg Ser Val Cys Ile
        475                 480                 485

agc ccc aat gga cag cac ctg gcc tcg gga gac cgc atg ggg aca ctt 1661
Ser Pro Asn Gly Gln His Leu Ala Ser Gly Asp Arg Met Gly Thr Leu
    490                 495                 500

agg ata cat gaa ctg cag tcc ctg agt gag atg ctg aaa gtg gag gcc 1709
Arg Ile His Glu Leu Gln Ser Leu Ser Glu Met Leu Lys Val Glu Ala
505                 510                 515                 520

cac gac tct gag atc ttg tgc ctg gag tac tct aag cca gac aca ggt 1757
His Asp Ser Glu Ile Leu Cys Leu Glu Tyr Ser Lys Pro Asp Thr Gly
                525                 530                 535

ttg aaa ctg cta gca tcg gca agc cgg gac cgt ctg atc cac gag ctg 1805
Leu Lys Leu Leu Ala Ser Ala Ser Arg Asp Arg Leu Ile His Glu Leu
            540                 545                 550

gat gct ggc cgg gaa tat agt cta cag cag aca ctg gat gag cat tca 1853
Asp Ala Gly Arg Glu Tyr Ser Leu Gln Gln Thr Leu Asp Glu His Ser
        555                 560                 565

tct tcc atc act gct gtc aag ttt gca gcc agt gat ggg caa gtg cga 1901
Ser Ser Ile Thr Ala Val Lys Phe Ala Ala Ser Asp Gly Gln Val Arg
    570                 575                 580

atg atc agc tgt ggt gca gac aag agc att tac ttc cga act gca cag 1949
Met Ile Ser Cys Gly Ala Asp Lys Ser Ile Tyr Phe Arg Thr Ala Gln
585                 590                 595                 600

aag tct gga gaa gga gta cag ttt aca cga acg cac cac gtg gta cgg 1997
Lys Ser Gly Glu Gly Val Gln Phe Thr Arg Thr His His Val Val Arg
                605                 610                 615

aag aca act ctc tat gac atg gat gtg gag ccc agc tgg aag tac acg 2045
Lys Thr Thr Leu Tyr Asp Met Asp Val Glu Pro Ser Trp Lys Tyr Thr
            620                 625                 630

gcc atc ggc tgc caa gac cgg aat att cgg atc ttt aac att agc agc 2093
Ala Ile Gly Cys Gln Asp Arg Asn Ile Arg Ile Phe Asn Ile Ser Ser
        635                 640                 645

gga aag cag aaa aag ctg ttt aaa ggg tca cag ggt gaa gat ggc act 2141

Gly Lys Gln Lys Lys Leu Phe Lys Gly Ser Gln Gly Glu Asp Gly Thr
    650                 655                 660

ctc att aag gtg cag aca gac ccc tca ggg atc tac att gcc act agc 2189
Leu Ile Lys Val Gln Thr Asp Pro Ser Gly Ile Tyr Ile Ala Thr Ser
665                 670                 675                 680

tgt tcc gat aag aat ctc tcc att ttt gac ttc tcc tca ggc gag tgt 2237
Cys Ser Asp Lys Asn Leu Ser Ile Phe Asp Phe Ser Ser Gly Glu Cys
                685                 690                 695

gtg gcc acc atg ttt ggc cac tca gag att gtc act ggc atg aaa ttt 2285
Val Ala Thr Met Phe Gly His Ser Glu Ile Val Thr Gly Met Lys Phe
            700                 705                 710

agt aac gat tgc aaa cat ctc atc tct gtg tca ggg gac agc tgc atc 2333
Ser Asn Asp Cys Lys His Leu Ile Ser Val Ser Gly Asp Ser Cys Ile
        715                 720                 725

ttt gtc tgg cgt ctg agc tct gag atg acc atc agc atg agg cag cgc 2381
Phe Val Trp Arg Leu Ser Ser Glu Met Thr Ile Ser Met Arg Gln Arg
    730                 735                 740

ctg cgt gag cgg cgg cag cgc cag cga ggg atc aag cag caa gga cca 2429
Leu Arg Glu Arg Arg Gln Arg Gln Arg Gly Ile Lys Gln Gln Gly Pro
745                 750                 755                 760

acg tct ccc cag agg gct tct gga gcc aag cag cac cat gct cca gtg 2477
Thr Ser Pro Gln Arg Ala Ser Gly Ala Lys Gln His His Ala Pro Val
                765                 770                 775

gta ccc cct tct gga cca gct ctt tcc tca gac agt gac aag gag gga 2525
Val Pro Pro Ser Gly Pro Ala Leu Ser Ser Asp Ser Asp Lys Glu Gly
            780                 785                 790

gaa gat gag ggt act gaa gaa gaa gaa ttg cca gct ctg ccc atc ctt 2573
Glu Asp Glu Gly Thr Glu Glu Glu Glu Leu Pro Ala Leu Pro Ile Leu
        795                 800                 805

agc aag agc acc aag aaa gaa cta gcc tca ggc tct agt cca gcc ttg 2621
Ser Lys Ser Thr Lys Lys Glu Leu Ala Ser Gly Ser Ser Pro Ala Leu
    810                 815                 820

ctc cga agc ctg tcc cac tgg gaa atg agt cgg gca caa gag acc atg 2669
Leu Arg Ser Leu Ser His Trp Glu Met Ser Arg Ala Gln Glu Thr Met
825                 830                 835                 840

gag tac ctg gac cca gct cct gta gct aac aca gga cct aaa aga aga 2717
Glu Tyr Leu Asp Pro Ala Pro Val Ala Asn Thr Gly Pro Lys Arg Arg
                845                 850                 855

ggg cgc tgg gct cag cca ggc gtg gag ctg agt gtt cgc tcc atg ttg 2765

Gly Arg Trp Ala Gln Pro Gly Val Glu Leu Ser Val Arg Ser Met Leu
            860                 865                 870

gac ctg aga cag ata gag acc tta gcc cca agc cct cga ggc ccc agc 2813
Asp Leu Arg Gln Ile Glu Thr Leu Ala Pro Ser Pro Arg Gly Pro Ser
        875                 880                 885

cag gac tca ctg gct gtg tcc cca gct ggt cct ggg aag cat ggt cca 2861
Gln Asp Ser Leu Ala Val Ser Pro Ala Gly Pro Gly Lys His Gly Pro
    890                 895                 900

cag gcc cct gag ctg tca tgt gtc agt cag aat gaa agg gcc cct cgg 2909
Gln Ala Pro Glu Leu Ser Cys Val Ser Gln Asn Glu Arg Ala Pro Arg
905                 910                 915                 920

ctt cag acc tcc caa ccc tgc tcc tgc ccc gac att atc caa ttg ttg 2957
Leu Gln Thr Ser Gln Pro Cys Ser Cys Pro Asp Ile Ile Gln Leu Leu
                925                 930                 935

tca caa gag gaa gga gtc ttt gcc caa gat ctg gag cct gca ccc att 3005
Ser Gln Glu Glu Gly Val Phe Ala Gln Asp Leu Glu Pro Ala Pro Ile
            940                 945                 950

gaa gat ggt att gtc tac ccg gaa ccc agt gac agc cct acc atg gat 3053
Glu Asp Gly Ile Val Tyr Pro Glu Pro Ser Asp Ser Pro Thr Met Asp
        955                 960                 965

acc agt gcg ttt cag gtg cag gct cca acc gga gga tcc cta gga aga 3101
Thr Ser Ala Phe Gln Val Gln Ala Pro Thr Gly Gly Ser Leu Gly Arg
    970                 975                 980

atg tac cca ggc agc agg ggc tca gaa aag cac agt cct gac agt gca 3149
Met Tyr Pro Gly Ser Arg Gly Ser Glu Lys His Ser Pro Asp Ser Ala
985                 990                 995                 1000

tgc tct gtg gat tac agc agc agc cgg ctt tcc agc cct gaa cac cct 3197
Cys Ser Val Asp Tyr Ser Ser Ser Arg Leu Ser Ser Pro Glu His Pro
                1005                1010                1015

aat gaa gac tct gag agc aca gag ccc cta agt gtg gat ggc atc tcc 3245
Asn Glu Asp Ser Glu Ser Thr Glu Pro Leu Ser Val Asp Gly Ile Ser
            1020                1025                1030

tca gac ctg gaa gag cca gcc gag ggt gat gaa gac gag gaa gaa gag 3293
Ser Asp Leu Glu Glu Pro Ala Glu Gly Asp Glu Asp Glu Glu Glu Glu
        1035                1040                1045

gga ggc act ggc ctc tgt ggg cta cag gaa ggc ggc cct cgt acc cca 3341
Gly Gly Thr Gly Leu Cys Gly Leu Gln Glu Gly Gly Pro Arg Thr Pro
    1050                1055                1060

gat cag gaa cag ttt cta aaa cag ctc ttt gag act ctg gcc aat ggg 3389

Asp Gln Glu Gln Phe Leu Lys Gln Leu Phe Glu Thr Leu Ala Asn Gly
1065                1070                1075                1080

act gct cca ggg ggc cca gca cgg gtg cta gag agg aca gag tct cgg    3437
Thr Ala Pro Gly Gly Pro Ala Arg Val Leu Glu Arg Thr Glu Ser Arg
                1085                1090                1095

agc atc tca tca cga ttc ctt ctg caa gtg cag acc ctc cca ctc agg    3485
Ser Ile Ser Ser Arg Phe Leu Leu Gln Val Gln Thr Leu Pro Leu Arg
            1100                1105                1110

gaa cca tcc cta tcc tcc tca ggc ttg gcc ctg acg tcc aga cct gac    3533
Glu Pro Ser Leu Ser Ser Ser Gly Leu Ala Leu Thr Ser Arg Pro Asp
        1115                1120                1125

cag gta tca cag gtg tct ggt gag cag ctg aaa ggc agt ggt gcc act    3581
Gln Val Ser Gln Val Ser Gly Glu Gln Leu Lys Gly Ser Gly Ala Thr
    1130                1135                1140

cct cca gga gca ccc cca gaa atg gaa ccc tct tct ggc aac tct ggc    3629
Pro Pro Gly Ala Pro Pro Glu Met Glu Pro Ser Ser Gly Asn Ser Gly
1145                1150                1155                1160

ccc aag cag gtg gct cct gtg ctg ttg aca cga cgg cgt aac aac ttg    3677
Pro Lys Gln Val Ala Pro Val Leu Leu Thr Arg Arg Arg Asn Asn Leu
                1165                1170                1175

gac aac agc tgg gcc tcc aag aaa atg gct gca acc cgg cct tta gct    3725
Asp Asn Ser Trp Ala Ser Lys Lys Met Ala Ala Thr Arg Pro Leu Ala
            1180                1185                1190

gga ctc cag aaa gcc cag tct gtg cat agt ttg gta cca cag gat gag    3773
Gly Leu Gln Lys Ala Gln Ser Val His Ser Leu Val Pro Gln Asp Glu
        1195                1200                1205

gtg cct tca tca cgt cca ctg ctc ttc cgg gag gca gag acc cag ggc    3821
Val Pro Ser Ser Arg Pro Leu Leu Phe Arg Glu Ala Glu Thr Gln Gly
    1210                1215                1220

agc tta gga tcc ctg cca caa gct ggt ggc tgc tca tct cag ccc cac    3869
Ser Leu Gly Ser Leu Pro Gln Ala Gly Gly Cys Ser Ser Gln Pro His
1225                1230                1235                1240

tcc tac cag aac cac acc acc agt tct atg gcc aag cta gcg cgt agt    3917
Ser Tyr Gln Asn His Thr Thr Ser Ser Met Ala Lys Leu Ala Arg Ser
                1245                1250                1255

att tct gtt ggc gag aat ccg ggc ctg gca act gaa cct caa gct cct    3965
Ile Ser Val Gly Glu Asn Pro Gly Leu Ala Thr Glu Pro Gln Ala Pro
            1260                1265                1270

gca ccg atc cga atc tca cca ttc aac aaa cta gct ctg cct agc agg    4013

Ala Pro Ile Arg Ile Ser Pro Phe Asn Lys Leu Ala Leu Pro Ser Arg
        1275                1280                1285

gct cac ctt gtc ctg gac atc ccc aaa cca ctt cct gac cgt cct act 4061
Ala His Leu Val Leu Asp Ile Pro Lys Pro Leu Pro Asp Arg Pro Thr
    1290                1295                1300

ctg acc aca ttc tca cct gta tcc aag ggc ctg acc cac aat gaa aca 4109
Leu Thr Thr Phe Ser Pro Val Ser Lys Gly Leu Thr His Asn Glu Thr
1305                1310                1315                1320

gaa caa tcg ggg ccc ctt cgt gag cct agg aag gct cat act aca gtt 4157
Glu Gln Ser Gly Pro Leu Arg Glu Pro Arg Lys Ala His Thr Thr Val
                1325                1330                1335

gaa aag cac tcc tgt tta ggg gag ggt act act cat aaa tct agg aca 4205
Glu Lys His Ser Cys Leu Gly Glu Gly Thr Thr His Lys Ser Arg Thr
            1340                1345                1350

gag tgc cag gct tat cct gga ccc aac cac ccc tgt cgc cag caa ctg 4253
Glu Cys Gln Ala Tyr Pro Gly Pro Asn His Pro Cys Arg Gln Gln Leu
        1355                1360                1365

cca gtc aac aac ctt ctc caa gct gag agc ttg cag ccc ctg tcc cct 4301
Pro Val Asn Asn Leu Leu Gln Ala Glu Ser Leu Gln Pro Leu Ser Pro
    1370                1375                1380

gag aag act cgt aac ccc gtg gaa agc agc agg cca ggg gta gcc ctg    4349
Glu Lys Thr Arg Asn Pro Val Glu Ser Ser Arg Pro Gly Val Ala Leu
1385                1390                1395                1400

agc cag gac tca gaa ctg gcc ttg agt ctg caa cag tgt gaa cag ctc    4397
Ser Gln Asp Ser Glu Leu Ala Leu Ser Leu Gln Gln Cys Glu Gln Leu
                1405                1410                1415

gtg gca gag ctc cag ggg aat gta cgc cag gca gtg gag ctc tac cgc    4445
Val Ala Glu Leu Gln Gly Asn Val Arg Gln Ala Val Glu Leu Tyr Arg
            1420                1425                1430

gcg gtg acc agc tgt aag aca cct tcg gca gag caa agt cac atc acc    4493
Ala Val Thr Ser Cys Lys Thr Pro Ser Ala Glu Gln Ser His Ile Thr
        1435                1440                1445

cgt ctc ctg aga gac acc ttc tct ccg gtg cga cag gag ctc gag gtt    4541
Arg Leu Leu Arg Asp Thr Phe Ser Pro Val Arg Gln Glu Leu Glu Val
    1450                1455                1460

ctg gct ggg gca gtg ctg tcc agc cca ggt ggc agc cct ggg gct gtg    4589
Leu Ala Gly Ala Val Leu Ser Ser Pro Gly Gly Ser Pro Gly Ala Val
1465                1470                1475                1480

gcg gct gag cag acg cag gcc ctg ttg gag caa tac tcc gag cta ctg    4637

Ala Ala Glu Gln Thr Gln Ala Leu Leu Glu Gln Tyr Ser Glu Leu Leu
                1485                1490                1495

cta aga gct gtg gag cgg cgc atg gag cgc aga ctc tgagctcctg a 4684
Leu Arg Ala Val Glu Arg Arg Met Glu Arg Arg Leu
            1500                1505

<210> 7
<211> 734
<212> DNA
<213> Mouse

<220>
<221> CDS
<222> (1)..(732)

<400> 7
agt agc aaa tat tca aac gag tcg aga agc cag gcg gac tct ggc ttc 48
Ser Ser Lys Tyr Ser Asn Glu Ser Arg Ser Gln Ala Asp Ser Gly Phe
1               5                   10                  15

ctg ggg ctg cgg ccg acc tcg gtg gat ccc gct ctg agg cgg cgg cgg 96
Leu Gly Leu Arg Pro Thr Ser Val Asp Pro Ala Leu Arg Arg Arg Arg
            20                  25                  30

cgg ggc ccc aga aac aag aag cgc ggc tgg agg agg ctc gcc gag gag 144
Arg Gly Pro Arg Asn Lys Lys Arg Gly Trp Arg Arg Leu Ala Glu Glu
        35                  40                  45

ccg ctg ggg tta gag gtc gac cag ttc ctg gaa gac gtc cgg cta cag 192
Pro Leu Gly Leu Glu Val Asp Gln Phe Leu Glu Asp Val Arg Leu Gln
    50                  55                  60

gag cgc acg acc ggt ggc ttg ttg gca gag gcc cca aac gaa aag ctc 240
Glu Arg Thr Thr Gly Gly Leu Leu Ala Glu Ala Pro Asn Glu Lys Leu
65                  70                  75                  80

ttc ttc gtg gac aca gga ttc aag aga aaa gaa cca aga aag aag agg 288
Phe Phe Val Asp Thr Gly Phe Lys Arg Lys Glu Pro Arg Lys Lys Arg
                85                  90                  95

acc ttg gtc cag aag aag tca cag cgt ctc cag aaa ccc tta cgg gtt 336
Thr Leu Val Gln Lys Lys Ser Gln Arg Leu Gln Lys Pro Leu Arg Val
            100                 105                 110

gac ctt gcc ctt gag aat cat tct aag atc cct gct ccc aaa gac atc 384
Asp Leu Ala Leu Glu Asn His Ser Lys Ile Pro Ala Pro Lys Asp Ile
        115                 120                 125

ctc gca cat cag gtc cct aat gcc aag aag ctc agg cga aag gag gag 432
Leu Ala His Gln Val Pro Asn Ala Lys Lys Leu Arg Arg Lys Glu Glu

    130                 135                 140

tta tgg gag aaa ctg gca aag cag ggc gaa ctg ccc agg gat gtg cgc 480
Leu Trp Glu Lys Leu Ala Lys Gln Gly Glu Leu Pro Arg Asp Val Arg
145                 150                 155                 160

aag gca cag gcc cga ctc ctt agc cct ccc aca cca aag gcc aaa cct 528
Lys Ala Gln Ala Arg Leu Leu Ser Pro Pro Thr Pro Lys Ala Lys Pro
                165                 170                 175

ggg ccc cag gac atc att gag cga ccc ttc tat gac ctc tgg aac cca 576
Gly Pro Gln Asp Ile Ile Glu Arg Pro Phe Tyr Asp Leu Trp Asn Pro
            180                 185                 190

gac aac cct ctg gac acg cct ttg att ggt cag gat gca ttt ttt ctg 624
Asp Asn Pro Leu Asp Thr Pro Leu Ile Gly Gln Asp Ala Phe Phe Leu
        195                 200                 205

gaa cag acc aag aag aaa ggc gtg agg cgg cca caa cgc ctc cac atc 672
Glu Gln Thr Lys Lys Lys Gly Val Arg Arg Pro Gln Arg Leu His Ile
    210                 215                 220

aag cct tcc cag gtg cct gca gtg gag gtg att cct gca gga gcc tcc 720
Lys Pro Ser Gln Val Pro Ala Val Glu Val Ile Pro Ala Gly Ala Ser
225                 230                 235                 240

tac aac cca acc tt                                                    734
Tyr Asn Pro Thr

<210> 8
<211> 1469
<212> DNA
<213> Mouse

<220>
<221> CDS
<222> (7)..(1458)

<400> 8
aagaag atg gct gcg ggt ggt aac agg gac ggt gag aag cgc ggc tcg aga 51
       Met Ala Ala Gly Gly Asn Arg Asp Gly Glu Lys Arg Gly Ser Arg
       1               5                   10                  15

agc cag gcg gac tct ggc ttc ctg ggg ctg cgg ccg acc tcg gtg gat     99
Ser Gln Ala Asp Ser Gly Phe Leu Gly Leu Arg Pro Thr Ser Val Asp
                20                  25                  30

ccc gct ctg agg cgg cgg cgg cgg ggc ccc aga aac aag aag cgc ggc     147
Pro Ala Leu Arg Arg Arg Arg Arg Gly Pro Arg Asn Lys Lys Arg Gly
            35                  40                  45

tgg agg agg ctc gcc gag gag ccg ctg ggg tta gag gtc gac cag ttc     195
Trp Arg Arg Leu Ala Glu Glu Pro Leu Gly Leu Glu Val Asp Gln Phe
        50                  55                  60

ctg gaa gac gtc cgg cta cag gag cgc acg acc ggt ggc ttg ttg gca     243

Leu Glu Asp Val Arg Leu Gln Glu Arg Thr Thr Gly Gly Leu Leu Ala
    65                  70                  75

gag gcc cca aac gaa aag ctc ttc ttc gtg gac aca gga ttc aag aga 291
Glu Ala Pro Asn Glu Lys Leu Phe Phe Val Asp Thr Gly Phe Lys Arg
80                  85                  90                  95

aaa gaa cca aga aag aag agg acc ttg gtc cag aag aag tca cag cgt 339
Lys Glu Pro Arg Lys Lys Arg Thr Leu Val Gln Lys Lys Ser Gln Arg
                100                 105                 110

ctc cag aaa ccc tta cgg gtt gac ctt gcc ctt gag aat cat tct aag 387
Leu Gln Lys Pro Leu Arg Val Asp Leu Ala Leu Glu Asn His Ser Lys
            115                 120                 125

atc cct gct ccc aaa gac atc ctc gca cat cag gtc cct aat gcc aag 435
Ile Pro Ala Pro Lys Asp Ile Leu Ala His Gln Val Pro Asn Ala Lys
        130                 135                 140

aag ctc agg cga aag gag gag tta tgg gag aaa ctg gca aag cag ggc 483
Lys Leu Arg Arg Lys Glu Glu Leu Trp Glu Lys Leu Ala Lys Gln Gly
    145                 150                 155

gaa ctg ccc agg gat gtg cgc aag gca cag gcc cga ctc ctt agc cct 531
Glu Leu Pro Arg Asp Val Arg Lys Ala Gln Ala Arg Leu Leu Ser Pro
160                 165                 170                 175

ccc aca cca aag gcc aaa cct ggg ccc cag gac atc att gag cga ccc 579
Pro Thr Pro Lys Ala Lys Pro Gly Pro Gln Asp Ile Ile Glu Arg Pro
                180                 185                 190

ttc tat gac ctc tgg aac cca gac aac cct ctg gac acg cct ttg att 627
Phe Tyr Asp Leu Trp Asn Pro Asp Asn Pro Leu Asp Thr Pro Leu Ile
            195                 200                 205

ggt cag gat gca ttt ttt ctg gaa cag acc aag aag aaa ggc gtg agg 675
Gly Gln Asp Ala Phe Phe Leu Glu Gln Thr Lys Lys Lys Gly Val Arg

        210                 215                 220

cgg cca caa cgc ctc cac atc aag cct tcc cag gtg cct gca gtg gag 723
Arg Pro Gln Arg Leu His Ile Lys Pro Ser Gln Val Pro Ala Val Glu
    225                 230                 235

gtg att cct gca gga gcc tcc tac aac cca acc ttt gaa gat cac cag 771
Val Ile Pro Ala Gly Ala Ser Tyr Asn Pro Thr Phe Glu Asp His Gln
240                 245                 250                 255

gcc ctg ctt cga gag gcc cat gag gtg gag ctg cag cgt gag aaa gag 819
Ala Leu Leu Arg Glu Ala His Glu Val Glu Leu Gln Arg Glu Lys Glu
                260                 265                 270

gca gaa aag ctg gag cga cag ctg gcc ctg ccc acc tca gag caa gct 867
Ala Glu Lys Leu Glu Arg Gln Leu Ala Leu Pro Thr Ser Glu Gln Ala
            275                 280                 285

gcc acc cag gag tcc gtg ttt cgg gag atg tgt gag ggc ctg ctg gag 915
Ala Thr Gln Glu Ser Val Phe Arg Glu Met Cys Glu Gly Leu Leu Glu
        290                 295                 300

gag tct gat ggt gag gat gag cat gag gca ggc cgt gcc ggg cag cca 963
Glu Ser Asp Gly Glu Asp Glu His Glu Ala Gly Arg Ala Gly Gln Pro
    305                 310                 315

gag gct ggt gat ggg acc acc gag atc tca ccc act ggt gct gct ggt 1011
Glu Ala Gly Asp Gly Thr Thr Glu Ile Ser Pro Thr Gly Ala Ala Gly
320                 325                 330                 335

cct gag aag agg atg gag aag aag acg gag cag cag cgg cgg cgg gag 1059
Pro Glu Lys Arg Met Glu Lys Lys Thr Glu Gln Gln Arg Arg Arg Glu
                340                 345                 350

aaa gct gct cgc aag ctg cgg gtg cag cag gct gca ctg agg gca gcc 1107
Lys Ala Ala Arg Lys Leu Arg Val Gln Gln Ala Ala Leu Arg Ala Ala
            355                 360                 365

cgg ctt cag cac caa gaa ctt ttc agg ctg cgt ggg atc aag gcc cag 1155
Arg Leu Gln His Gln Glu Leu Phe Arg Leu Arg Gly Ile Lys Ala Gln
        370                 375                 380

gtg gcc cga agg ctg gca gaa ctg gca cgc cgg agg gag cag cgg cgc 1203
Val Ala Arg Arg Leu Ala Glu Leu Ala Arg Arg Arg Glu Gln Arg Arg
    385                 390                 395

ata cgg cga ctg gca gag gct gac aag ccc cga agg ctg gga cgg ctc 1251
Ile Arg Arg Leu Ala Glu Ala Asp Lys Pro Arg Arg Leu Gly Arg Leu
400                 405                 410                 415

aag tac cag gct cct gac att gat gtg cag ctc agc tct gag ttg tct 1299
Lys Tyr Gln Ala Pro Asp Ile Asp Val Gln Leu Ser Ser Glu Leu Ser
                420                 425                 430

ggc tca ctc agg aca ctg aag cca gaa ggt cac att ctc cga gac agg 1347
Gly Ser Leu Arg Thr Leu Lys Pro Glu Gly His Ile Leu Arg Asp Arg
            435                 440                 445

ttc aag agc ttc cag aag aga aat atg att gag ccc cga gaa cga gcc 1395
Phe Lys Ser Phe Gln Lys Arg Asn Met Ile Glu Pro Arg Glu Arg Ala
        450                 455                 460

aag ttc aag cgc aaa tac aaa gtg aag ctg gtg gag aag cgg gcc tac 1443
Lys Phe Lys Arg Lys Tyr Lys Val Lys Leu Val Glu Lys Arg Ala Tyr
    465                 470                 475

cgt gag att cag ttg tag ctgtgcagat g 1469
Arg Glu Ile Gln Leu
480

<210> 9

<211> 1305

<212> PRT

<213> Mouse

<400> 9

Met Met Glu Ile Gln Met Asp Glu Gly Gly Gly Val Val Val Tyr Gln

1               5                   10                  15

Asp Asp Tyr Cys Ser Gly Ser Val Met Ser Glu Arg Val Ser Gly Leu

            20                  25                  30

Ala Gly Ser Ile Tyr Arg Glu Phe Glu Arg Leu Ile His Cys Tyr Asp

        35                  40                  45

Glu Glu Val Val Lys Glu Leu Met Pro Leu Val Val Asn Val Leu Glu

    50                  55                  60

Asn Leu Asp Ser Val Leu Ser Glu Asn Gln Glu His Glu Val Glu Leu

65                  70                  75                  80

Glu Leu Leu Arg Glu Asp Asn Glu Gln Leu Leu Thr Gln Tyr Glu Arg

                85                  90                  95

Glu Lys Ala Leu Arg Lys Gln Ala Glu Glu Lys Phe Ile Glu Phe Glu

            100                 105                 110

Asp Ala Leu Glu Gln Glu Lys Lys Glu Leu Gln Ile Gln Val Glu His

        115                 120                 125

Tyr Glu Phe Gln Thr Arg Gln Leu Glu Leu Lys Ala Lys Asn Tyr Ala
    130                 135                 140

Asp Gln Ile Ser Arg Leu Glu Glu Arg Glu Ser Glu Met Lys Lys Glu
145                 150                 155                 160

Tyr Asn Ala Leu His Gln Arg His Thr Glu Met Ile Gln Thr Tyr Val
                165                 170                 175

Glu His Ile Glu Arg Ser Lys Met Gln Gln Val Gly Gly Ser Gly Gln
            180                 185                 190

Thr Glu Ser Ser Leu Pro Gly Arg Arg Lys Glu Arg Pro Thr Ser Leu
        195                 200                 205

Asn Val Phe Pro Leu Ala Asp Gly Met Cys Pro Asn Asp Glu Met Ser
    210                 215                 220

Glu Ser Gly Gln Ser Ser Ala Ala Ala Thr Pro Ser Thr Thr Gly Thr
225                 230                 235                 240

Lys Ser Asn Thr Pro Thr Ser Ser Val Pro Ser Ala Ala Val Thr Pro
                245                 250                 255

Leu Asn Glu Ser Leu Gln Pro Leu Gly Asp Tyr Val Ser Val Thr Lys
            260                 265                 270
Asn Asn Lys Gln Ala Arg Glu Lys Arg Asn Ser Arg Asn Met Glu Val
        275                 280                 285
Gln Val Thr Gln Glu Met Arg Asn Val Ser Ile Gly Met Gly Ser Ser
    290                 295                 300
Asp Glu Trp Ser Asp Val Gln Asp Ile Ile Asp Ser Thr Pro Glu Leu
305                 310                 315                 320
Asp Val Cys Pro Glu Thr Arg Leu Glu Arg Thr Gly Ser Ser Pro Thr
                325                 330                 335
Gln Gly Ile Val Asn Lys Ala Phe Gly Ile Asn Thr Asp Ser Leu Tyr
            340                 345                 350
His Glu Leu Ser Thr Ala Gly Ser Glu Val Ile Gly Asp Val Asp Glu
        355                 360                 365
Gly Ala Asp Leu Leu Gly Glu Phe Ser Val Arg Asp Asp Phe Phe Gly
    370                 375                 380
Met Gly Lys Glu Val Gly Asn Leu Leu Leu Glu Asn Ser Gln Leu Leu
385                 390                 395                 400

Glu Thr Lys Asn Ala Leu Asn Val Val Lys Asn Asp Leu Ile Ala Lys
                405                 410                 415
Val Asp Gln Leu Ser Gly Glu Gln Glu Val Leu Lys Gly Glu Leu Glu
            420                 425                 430
Ala Ala Lys Gln Ala Lys Val Lys Leu Glu Asn Arg Ile Lys Glu Leu
        435                 440                 445
Glu Glu Glu Leu Lys Arg Val Lys Ser Glu Ala Val Thr Ala Arg Arg
    450                 455                 460
Glu Pro Arg Glu Glu Val Glu Asp Val Ser Ser Tyr Leu Cys Thr Glu
465                 470                 475                 480
Leu Asp Lys Ile Pro Met Ala Gln Arg Arg Arg Phe Thr Arg Val Glu
                485                 490                 495
Met Ala Arg Val Leu Met Glu Arg Asn Gln Tyr Lys Glu Arg Leu Met
            500                 505                 510
Glu Leu Gln Glu Ala Val Arg Trp Thr Glu Met Ile Arg Ala Ser Arg
        515                 520                 525
Glu His Pro Ser Val Gln Glu Lys Lys Lys Ser Thr Ile Trp Gln Phe

    530                 535                 540

Phe Ser Arg Leu Phe Ser Ser Ser Ser Ser Pro Pro Pro Ala Lys Arg
545                 550                 555                 560

Ser Tyr Pro Ser Val Asn Ile His Tyr Lys Ser Pro Thr Ala Ala Gly
                565                 570                 575

Phe Ser Gln Arg Arg Ser His Ala Leu Cys Gln Ile Ser Ala Gly Ser
            580                 585                 590

Arg Pro Leu Glu Phe Phe Pro Asp Asp Asp Cys Thr Ser Ser Ala Arg
        595                 600                 605

Arg Glu Gln Lys Arg Glu Gln Tyr Arg Gln Val Arg Glu His Val Arg
    610                 615                 620

Asn Asp Asp Gly Arg Leu Gln Ala Cys Gly Trp Ser Leu Pro Ala Lys
625                 630                 635                 640

Tyr Lys Gln Leu Ser Pro Asn Gly Gly Gln Glu Asp Thr Arg Met Lys
                645                 650                 655

Asn Val Pro Val Pro Val Tyr Cys Arg Pro Leu Val Glu Lys Asp Pro
            660                 665                 670

Ser Thr Lys Leu Trp Cys Ala Ala Gly Val Asn Leu Ser Gly Trp Lys
        675                 680                 685

Pro His Glu Glu Asp Ser Ser Asn Gly Pro Lys Pro Val Pro Gly Arg
    690                 695                 700

Asp Pro Leu Thr Cys Asp Arg Glu Gly Glu Gly Glu Pro Lys Ser Thr
705                 710                 715                 720

His Pro Ser Pro Glu Lys Lys Lys Ala Lys Glu Thr Pro Glu Ala Asp
                725                 730                 735

Ala Thr Ser Ser Arg Val Trp Ile Leu Thr Ser Thr Leu Thr Thr Ser
            740                 745                 750

Lys Val Val Ile Ile Asp Ala Asn Gln Pro Gly Thr Ile Val Asp Gln
        755                 760                 765

Phe Thr Val Cys Asn Ala His Val Leu Cys Ile Ser Ser Ile Pro Ala
    770                 775                 780

Ala Ser Asp Ser Asp Tyr Pro Pro Gly Glu Met Phe Leu Asp Ser Asp
785                 790                 795                 800

Val Asn Pro Glu Asp Ser Gly Ala Asp Gly Val Leu Ala Gly Ile Thr
                805                 810                 815

Leu Val Gly Cys Ala Thr Arg Cys Asn Val Pro Arg Ser Asn Cys Ser
            820                 825                 830
Ser Arg Gly Asp Thr Pro Val Leu Asp Lys Gly Gln Gly Asp Val Ala
        835                 840                 845
Thr Thr Ala Asn Gly Lys Val Asn Pro Ser Gln Ser Thr Glu Glu Ala
    850                 855                 860
Thr Glu Ala Thr Glu Val Pro Asp Pro Gly Pro Ser Glu Ser Glu Ala
865                 870                 875                 880
Thr Thr Val Arg Pro Gly Pro Leu Thr Glu His Val Phe Thr Asp Pro
                885                 890                 895
Ala Pro Thr Pro Ser Ser Ser Thr Gln Pro Ala Ser Glu Asn Gly Ser
            900                 905                 910
Glu Ser Asn Gly Thr Ile Val Gln Pro Gln Val Glu Pro Ser Gly Glu
        915                 920                 925
Leu Ser Thr Thr Thr Ser Ser Ala Ala Pro Thr Met Trp Leu Gly Ala
    930                 935                 940
Gln Asn Gly Trp Leu Tyr Val His Ser Ala Val Ala Asn Trp Lys Lys

945                 950                 955                 960

Cys Leu His Ser Ile Lys Leu Lys Asp Ser Val Leu Ser Leu Val His

                965                 970                 975

Val Lys Gly Arg Val Leu Val Ala Leu Ala Asp Gly Thr Leu Ala Ile

            980                 985                 990

Phe His Arg Gly Glu Asp Gly Gln Trp Asp Leu Ser Asn Tyr His Leu

        995                 1000                1005

Met Asp Leu Gly His Pro His His Ser Ile Arg Cys Met Ala Val Val

    1010                1015                1020

Asn Asp Arg Val Trp Cys Gly Tyr Lys Asn Lys Val His Val Ile Gln

025 1030 1035 1040

Pro Lys Thr Met Gln Ile Glu Lys Ser Phe Asp Ala His Pro Arg Arg

                1045                1050                1055

Glu Ser Gln Val Arg Gln Leu Ala Trp Ile Gly Asp Gly Val Trp Val

            1060                1065                1070

Ser Ile Arg Leu Asp Ser Thr Leu Arg Leu Tyr His Ala His Thr His

        1075                1080                1085

Gln His Leu Gln Asp Val Asp Ile Glu Pro Tyr Val Ser Lys Met Leu
    1090                1095                1100

Gly Thr Gly Lys Leu Gly Phe Ser Phe Val Arg Ile Thr Ala Leu Leu
105 1110 1115 1120

Ile Ala Gly Asn Arg Leu Trp Val Gly Thr Gly Asn Gly Val Val Ile
                1125                1130                1135

Ser Ile Pro Leu Thr Glu Thr Val Val Leu His Arg Gly Gln Leu Leu
            1140                1145                1150

Gly Leu Arg Ala Asn Lys Thr Ser Pro Thr Ser Gly Glu Gly Thr Arg
        1155                1160                1165

Pro Gly Gly Ile Ile His Val Tyr Gly Asp Asp Ser Ser Asp Lys Ala
    1170                1175                1180

Ala Ser Ser Phe Ile Pro Tyr Cys Ser Met Ala Gln Ala Gln Leu Cys
185 1190 1195 1200

Phe His Gly His Arg Asp Ala Val Lys Phe Phe Val Ser Val Pro Gly
                1205                1210                1215

Asn Val Leu Ala Thr Leu Asn Gly Ser Val Leu Asp Ser Pro Ser Glu
            1220                1225                1230

Gly Pro Gly Pro Ala Ala Pro Ala Ala Asp Ala Glu Gly Gln Lys Leu
        1235                1240                1245
Lys Asn Ala Leu Val Leu Ser Gly Gly Glu Gly Tyr Ile Asp Phe Arg
    1250                1255                1260
Ile Gly Asp Gly Glu Asp Asp Glu Thr Glu Glu Cys Ala Gly Asp Val
265 1270 1275 1280
Asn Gln Thr Lys Pro Ser Leu Ser Lys Ala Glu Arg Ser His Ile Ile
                1285                1290                1295
Val Trp Gln Val Ser Tyr Thr Pro Glu
            1300                1305

<210> 10
<211> 1314
<212> PRT
<213> Mouse

<400> 10
Met Met Glu Ile Gln Met Asp Glu Gly Gly Gly Val Val Val Tyr Gln
1               5                   10                  15

Asp Asp Tyr Cys Ser Gly Ser Val Met Ser Glu Arg Val Ser Gly Leu
            20                  25                  30
Ala Gly Ser Ile Tyr Arg Glu Phe Glu Arg Leu Ile His Cys Tyr Asp
        35                  40                  45
Glu Glu Val Val Lys Glu Leu Met Pro Leu Val Val Asn Val Leu Glu
    50                  55                  60
Asn Leu Asp Ser Val Leu Ser Glu Asn Gln Glu His Glu Val Glu Leu
65                  70                  75                  80
Glu Leu Leu Arg Glu Asp Asn Glu Gln Leu Leu Thr Gln Tyr Glu Arg
                85                  90                  95
Glu Lys Ala Leu Arg Lys Gln Ala Glu Glu Lys Phe Ile Glu Phe Glu
            100                 105                 110
Asp Ala Leu Glu Gln Glu Lys Lys Glu Leu Gln Ile Gln Val Glu His
        115                 120                 125
Tyr Glu Phe Gln Thr Arg Gln Leu Glu Leu Lys Ala Lys Asn Tyr Ala
    130                 135                 140
Asp Gln Ile Ser Arg Leu Glu Glu Arg Glu Ser Glu Met Lys Lys Glu
145                 150                 155                 160

Tyr Asn Ala Leu His Gln Arg His Thr Glu Met Ile Gln Thr Tyr Val
                165                 170                 175
Glu His Ile Glu Arg Ser Lys Met Gln Gln Val Gly Gly Ser Gly Gln
            180                 185                 190
Thr Glu Ser Ser Leu Pro Gly Arg Ser Pro Arg Gln Ser Trp Arg Lys
        195                 200                 205
Ser Arg Lys Glu Arg Pro Thr Ser Leu Asn Val Phe Pro Leu Ala Asp
    210                 215                 220
Gly Met Cys Pro Asn Asp Glu Met Ser Glu Ser Gly Gln Ser Ser Ala
225                 230                 235                 240
Ala Ala Thr Pro Ser Thr Thr Gly Thr Lys Ser Asn Thr Pro Thr Ser
                245                 250                 255
Ser Val Pro Ser Ala Ala Val Thr Pro Leu Asn Glu Ser Leu Gln Pro
            260                 265                 270
Leu Gly Asp Tyr Val Ser Val Thr Lys Asn Asn Lys Gln Ala Arg Glu
        275                 280                 285
Lys Arg Asn Ser Arg Asn Met Glu Val Gln Val Thr Gln Glu Met Arg

    290                 295                 300

Asn Val Ser Ile Gly Met Gly Ser Ser Asp Glu Trp Ser Asp Val Gln
305                 310                 315                 320

Asp Ile Ile Asp Ser Thr Pro Glu Leu Asp Val Cys Pro Glu Thr Arg
                325                 330                 335

Leu Glu Arg Thr Gly Ser Ser Pro Thr Gln Gly Ile Val Asn Lys Ala
            340                 345                 350

Phe Gly Ile Asn Thr Asp Ser Leu Tyr His Glu Leu Ser Thr Ala Gly
        355                 360                 365

Ser Glu Val Ile Gly Asp Val Asp Glu Gly Ala Asp Leu Leu Gly Glu
    370                 375                 380

Phe Ser Val Arg Asp Asp Phe Phe Gly Met Gly Lys Glu Val Gly Asn
385                 390                 395                 400

Leu Leu Leu Glu Asn Ser Gln Leu Leu Glu Thr Lys Asn Ala Leu Asn
                405                 410                 415

Val Val Lys Asn Asp Leu Ile Ala Lys Val Asp Gln Leu Ser Gly Glu
            420                 425                 430

Gln Glu Val Leu Lys Gly Glu Leu Glu Ala Ala Lys Gln Ala Lys Val
        435                 440                 445

Lys Leu Glu Asn Arg Ile Lys Glu Leu Glu Glu Glu Leu Lys Arg Val
    450                 455                 460

Lys Ser Glu Ala Val Thr Ala Arg Arg Glu Pro Arg Glu Glu Val Glu
465                 470                 475                 480

Asp Val Ser Ser Tyr Leu Cys Thr Glu Leu Asp Lys Ile Pro Met Ala
                485                 490                 495

Gln Arg Arg Arg Phe Thr Arg Val Glu Met Ala Arg Val Leu Met Glu
            500                 505                 510

Arg Asn Gln Tyr Lys Glu Arg Leu Met Glu Leu Gln Glu Ala Val Arg
        515                 520                 525

Trp Thr Glu Met Ile Arg Ala Ser Arg Glu His Pro Ser Val Gln Glu
    530                 535                 540

Lys Lys Lys Ser Thr Ile Trp Gln Phe Phe Ser Arg Leu Phe Ser Ser
545                 550                 555                 560

Ser Ser Ser Pro Pro Pro Ala Lys Arg Ser Tyr Pro Ser Val Asn Ile
                565                 570                 575

His Tyr Lys Ser Pro Thr Ala Ala Gly Phe Ser Gln Arg Arg Ser His
            580                 585                 590

Ala Leu Cys Gln Ile Ser Ala Gly Ser Arg Pro Leu Glu Phe Phe Pro
        595                 600                 605

Asp Asp Asp Cys Thr Ser Ser Ala Arg Arg Glu Gln Lys Arg Glu Gln
    610                 615                 620

Tyr Arg Gln Val Arg Glu His Val Arg Asn Asp Asp Gly Arg Leu Gln
625                 630                 635                 640

Ala Cys Gly Trp Ser Leu Pro Ala Lys Tyr Lys Gln Leu Ser Pro Asn
                645                 650                 655

Gly Gly Gln Glu Asp Thr Arg Met Lys Asn Val Pro Val Pro Val Tyr
            660                 665                 670

Cys Arg Pro Leu Val Glu Lys Asp Pro Ser Thr Lys Leu Trp Cys Ala
        675                 680                 685

Ala Gly Val Asn Leu Ser Gly Trp Lys Pro His Glu Glu Asp Ser Ser
    690                 695                 700

Asn Gly Pro Lys Pro Val Pro Gly Arg Asp Pro Leu Thr Cys Asp Arg

705                 710                 715                 720

Glu Gly Glu Gly Glu Pro Lys Ser Thr His Pro Ser Pro Glu Lys Lys

                725                 730                 735

Lys Ala Lys Glu Thr Pro Glu Ala Asp Ala Thr Ser Ser Arg Val Trp

            740                 745                 750

Ile Leu Thr Ser Thr Leu Thr Thr Ser Lys Val Val Ile Ile Asp Ala

        755                 760                 765

Asn Gln Pro Gly Thr Ile Val Asp Gln Phe Thr Val Cys Asn Ala His

    770                 775                 780

Val Leu Cys Ile Ser Ser Ile Pro Ala Ala Ser Asp Ser Asp Tyr Pro
785                 790                 795                 800

Pro Gly Glu Met Phe Leu Asp Ser Asp Val Asn Pro Glu Asp Ser Gly

                805                 810                 815

Ala Asp Gly Val Leu Ala Gly Ile Thr Leu Val Gly Cys Ala Thr Arg

            820                 825                 830

Cys Asn Val Pro Arg Ser Asn Cys Ser Ser Arg Gly Asp Thr Pro Val

        835                 840                 845

Leu Asp Lys Gly Gln Gly Asp Val Ala Thr Thr Ala Asn Gly Lys Val
    850                 855                 860

Asn Pro Ser Gln Ser Thr Glu Glu Ala Thr Glu Ala Thr Glu Val Pro
865                 870                 875                 880

Asp Pro Gly Pro Ser Glu Ser Glu Ala Thr Thr Val Arg Pro Gly Pro
                885                 890                 895

Leu Thr Glu His Val Phe Thr Asp Pro Ala Pro Thr Pro Ser Ser Ser
            900                 905                 910

Thr Gln Pro Ala Ser Glu Asn Gly Ser Glu Ser Asn Gly Thr Ile Val
        915                 920                 925

Gln Pro Gln Val Glu Pro Ser Gly Glu Leu Ser Thr Thr Thr Ser Ser
    930                 935                 940

Ala Ala Pro Thr Met Trp Leu Gly Ala Gln Asn Gly Trp Leu Tyr Val
945                 950                 955                 960

His Ser Ala Val Ala Asn Trp Lys Lys Cys Leu His Ser Ile Lys Leu
                965                 970                 975

Lys Asp Ser Val Leu Ser Leu Val His Val Lys Gly Arg Val Leu Val
            980                 985                 990

Ala Leu Ala Asp Gly Thr Leu Ala Ile Phe His Arg Gly Glu Asp Gly
        995                 1000                1005
Gln Trp Asp Leu Ser Asn Tyr His Leu Met Asp Leu Gly His Pro His
    1010                1015                1020
His Ser Ile Arg Cys Met Ala Val Val Asn Asp Arg Val Trp Cys Gly
025 1030 1035 1040
Tyr Lys Asn Lys Val His Val Ile Gln Pro Lys Thr Met Gln Ile Glu
                1045                1050                1055
Lys Ser Phe Asp Ala His Pro Arg Arg Glu Ser Gln Val Arg Gln Leu
            1060                1065                1070
Ala Trp Ile Gly Asp Gly Val Trp Val Ser Ile Arg Leu Asp Ser Thr
        1075                1080                1085
Leu Arg Leu Tyr His Ala His Thr His Gln His Leu Gln Asp Val Asp
    1090                1095                1100
Ile Glu Pro Tyr Val Ser Lys Met Leu Gly Thr Gly Lys Leu Gly Phe
105 1110 1115 1120
Ser Phe Val Arg Ile Thr Ala Leu Leu Ile Ala Gly Asn Arg Leu Trp

                1125                1130                1135

Val Gly Thr Gly Asn Gly Val Val Ile Ser Ile Pro Leu Thr Glu Thr

            1140                1145                1150

Val Val Leu His Arg Gly Gln Leu Leu Gly Leu Arg Ala Asn Lys Thr

        1155                1160                1165

Ser Pro Thr Ser Gly Glu Gly Thr Arg Pro Gly Gly Ile Ile His Val

    1170                1175                1180

Tyr Gly Asp Asp Ser Ser Asp Lys Ala Ala Ser Ser Phe Ile Pro Tyr

185 1190 1195 1200

Cys Ser Met Ala Gln Ala Gln Leu Cys Phe His Gly His Arg Asp Ala

                1205                1210                1215

Val Lys Phe Phe Val Ser Val Pro Gly Asn Val Leu Ala Thr Leu Asn

            1220                1225                1230

Gly Ser Val Leu Asp Ser Pro Ser Glu Gly Pro Gly Pro Ala Ala Pro

        1235                1240                1245

Ala Ala Asp Ala Glu Gly Gln Lys Leu Lys Asn Ala Leu Val Leu Ser

    1250                1255                1260

Gly Gly Glu Gly Tyr Ile Asp Phe Arg Ile Gly Asp Gly Glu Asp Asp
265                 1270                1275                1280

Glu Thr Glu Glu Cys Ala Gly Asp Val Asn Gln Thr Lys Pro Ser Leu
                1285                1290                1295

Ser Lys Ala Glu Arg Ser His Ile Ile Val Trp Gln Val Ser Tyr Thr
            1300                1305                1310

Pro Glu

<210> 11
<211> 1337
<212> PRT
<213> Mouse

<400> 11
Met Met Glu Ile Gln Met Asp Glu Gly Gly Gly Val Val Val Tyr Gln
  1               5                  10                  15

Asp Asp Tyr Cys Ser Gly Ser Val Met Ser Glu Arg Val Ser Gly Leu
                 20                  25                  30

Ala Gly Ser Ile Tyr Arg Glu Phe Glu Arg Leu Ile His Cys Tyr Asp

        35                  40                  45

Glu Glu Val Val Lys Glu Leu Met Pro Leu Val Val Asn Val Leu Glu

    50                  55                  60

Asn Leu Asp Ser Val Leu Ser Glu Asn Gln Glu His Glu Val Glu Leu

65                  70                  75                  80

Glu Leu Leu Arg Glu Asp Asn Glu Gln Leu Leu Thr Gln Tyr Glu Arg

                85                  90                  95

Glu Lys Ala Leu Arg Lys Gln Ala Glu Glu Lys Phe Ile Glu Phe Glu

            100                 105                 110

Asp Ala Leu Glu Gln Glu Lys Lys Glu Leu Gln Ile Gln Val Glu His

        115                 120                 125

Tyr Glu Phe Gln Thr Arg Gln Leu Glu Leu Lys Ala Lys Asn Tyr Ala

    130                 135                 140

Asp Gln Ile Ser Arg Leu Glu Glu Arg Glu Ser Glu Met Lys Lys Glu

145                 150                 155                 160

Tyr Asn Ala Leu His Gln Arg His Thr Glu Met Ile Gln Thr Tyr Val

                165                 170                 175

Glu His Ile Glu Arg Ser Lys Met Gln Gln Val Gly Gly Ser Gly Gln
            180                 185                 190

Thr Glu Ser Ser Leu Pro Gly Arg Ser Arg Lys Glu Arg Pro Thr Ser
        195                 200                 205

Leu Asn Val Phe Pro Leu Ala Asp Gly Met Val Arg Ala Gln Met Gly
    210                 215                 220

Gly Lys Leu Val Pro Ala Gly Asp His Trp His Leu Ser Asp Leu Gly
225                 230                 235                 240

Gln Leu Gln Ser Ser Ser Ser Tyr Gln Cys Pro Asn Asp Glu Met Ser
                245                 250                 255

Glu Ser Gly Gln Ser Ser Ala Ala Ala Thr Pro Ser Thr Thr Gly Thr
            260                 265                 270

Lys Ser Asn Thr Pro Thr Ser Ser Val Pro Ser Ala Ala Val Thr Pro
        275                 280                 285

Leu Asn Glu Ser Leu Gln Pro Leu Gly Asp Tyr Val Ser Val Thr Lys
    290                 295                 300

Asn Asn Lys Gln Ala Arg Glu Lys Arg Asn Ser Arg Asn Met Glu Val
305                 310                 315                 320

Gln Val Thr Gln Glu Met Arg Asn Val Ser Ile Gly Met Gly Ser Ser
                325                 330                 335
Asp Glu Trp Ser Asp Val Gln Asp Ile Ile Asp Ser Thr Pro Glu Leu
            340                 345                 350
Asp Val Cys Pro Glu Thr Arg Leu Glu Arg Thr Gly Ser Ser Pro Thr
        355                 360                 365
Gln Gly Ile Val Asn Lys Ala Phe Gly Ile Asn Thr Asp Ser Leu Tyr
    370                 375                 380
His Glu Leu Ser Thr Ala Gly Ser Glu Val Ile Gly Asp Val Asp Glu
385                 390                 395                 400
Gly Ala Asp Leu Leu Gly Glu Phe Ser Val Arg Asp Asp Phe Phe Gly
                405                 410                 415
Met Gly Lys Glu Val Gly Asn Leu Leu Leu Glu Asn Ser Gln Leu Leu
            420                 425                 430
Glu Thr Lys Asn Ala Leu Asn Val Val Lys Asn Asp Leu Ile Ala Lys
        435                 440                 445
Val Asp Gln Leu Ser Gly Glu Gln Glu Val Leu Lys Gly Glu Leu Glu

    450                 455                 460

Ala Ala Lys Gln Ala Lys Val Lys Leu Glu Asn Arg Ile Lys Glu Leu
465                 470                 475                 480

Glu Glu Glu Leu Lys Arg Val Lys Ser Glu Ala Val Thr Ala Arg Arg
                485                 490                 495

Glu Pro Arg Glu Glu Val Glu Asp Val Ser Ser Tyr Leu Cys Thr Glu
            500                 505                 510

Leu Asp Lys Ile Pro Met Ala Gln Arg Arg Arg Phe Thr Arg Val Glu
        515                 520                 525

Met Ala Arg Val Leu Met Glu Arg Asn Gln Tyr Lys Glu Arg Leu Met
    530                 535                 540

Glu Leu Gln Glu Ala Val Arg Trp Thr Glu Met Ile Arg Ala Ser Arg
545                 550                 555                 560

Glu His Pro Ser Val Gln Glu Lys Lys Lys Ser Thr Ile Trp Gln Phe
                565                 570                 575

Phe Ser Arg Leu Phe Ser Ser Ser Ser Ser Pro Pro Pro Ala Lys Arg
            580                 585                 590

Ser Tyr Pro Ser Val Asn Ile His Tyr Lys Ser Pro Thr Ala Ala Gly
        595                 600                 605

Phe Ser Gln Arg Arg Ser His Ala Leu Cys Gln Ile Ser Ala Gly Ser
    610                 615                 620

Arg Pro Leu Glu Phe Phe Pro Asp Asp Asp Cys Thr Ser Ser Ala Arg
625                 630                 635                 640

Arg Glu Gln Lys Arg Glu Gln Tyr Arg Gln Val Arg Glu His Val Arg
                645                 650                 655

Asn Asp Asp Gly Arg Leu Gln Ala Cys Gly Trp Ser Leu Pro Ala Lys
            660                 665                 670

Tyr Lys Gln Leu Ser Pro Asn Gly Gly Gln Glu Asp Thr Arg Met Lys
        675                 680                 685

Asn Val Pro Val Pro Val Tyr Cys Arg Pro Leu Val Glu Lys Asp Pro
    690                 695                 700

Ser Thr Lys Leu Trp Cys Ala Ala Gly Val Asn Leu Ser Gly Trp Lys
705                 710                 715                 720

Pro His Glu Glu Asp Ser Ser Asn Gly Pro Lys Pro Val Pro Gly Arg
                725                 730                 735

Asp Pro Leu Thr Cys Asp Arg Glu Gly Glu Gly Glu Pro Lys Ser Thr
            740                 745                 750
His Pro Ser Pro Glu Lys Lys Lys Ala Lys Glu Thr Pro Glu Ala Asp
        755                 760                 765
Ala Thr Ser Ser Arg Val Trp Ile Leu Thr Ser Thr Leu Thr Thr Ser
    770                 775                 780
Lys Val Val Ile Ile Asp Ala Asn Gln Pro Gly Thr Ile Val Asp Gln
785                 790                 795                 800
Phe Thr Val Cys Asn Ala His Val Leu Cys Ile Ser Ser Ile Pro Ala
                805                 810                 815
Ala Ser Asp Ser Asp Tyr Pro Pro Gly Glu Met Phe Leu Asp Ser Asp
            820                 825                 830
Val Asn Pro Glu Asp Ser Gly Ala Asp Gly Val Leu Ala Gly Ile Thr
        835                 840                 845
Leu Val Gly Cys Ala Thr Arg Cys Asn Val Pro Arg Ser Asn Cys Ser
    850                 855                 860
Ser Arg Gly Asp Thr Pro Val Leu Asp Lys Gly Gln Gly Asp Val Ala

865                 870                 875                 880

Thr Thr Ala Asn Gly Lys Val Asn Pro Ser Gln Ser Thr Glu Glu Ala
                885                 890                 895

Thr Glu Ala Thr Glu Val Pro Asp Pro Gly Pro Ser Glu Ser Glu Ala
            900                 905                 910

Thr Thr Val Arg Pro Gly Pro Leu Thr Glu His Val Phe Thr Asp Pro
        915                 920                 925

Ala Pro Thr Pro Ser Ser Ser Thr Gln Pro Ala Ser Glu Asn Gly Ser
    930                 935                 940

Glu Ser Asn Gly Thr Ile Val Gln Pro Gln Val Glu Pro Ser Gly Glu
945                 950                 955                 960

Leu Ser Thr Thr Thr Ser Ser Ala Ala Pro Thr Met Trp Leu Gly Ala
                965                 970                 975

Gln Asn Gly Trp Leu Tyr Val His Ser Ala Val Ala Asn Trp Lys Lys
            980                 985                 990

Cys Leu His Ser Ile Lys Leu Lys Asp Ser Val Leu Ser Leu Val His
        995                 1000                1005

Val Lys Gly Arg Val Leu Val Ala Leu Ala Asp Gly Thr Leu Ala Ile
    1010                1015                1020
Phe His Arg Gly Glu Asp Gly Gln Trp Asp Leu Ser Asn Tyr His Leu
025 1030 1035 1040
Met Asp Leu Gly His Pro His His Ser Ile Arg Cys Met Ala Val Val
                1045                1050                1055
Asn Asp Arg Val Trp Cys Gly Tyr Lys Asn Lys Val His Val Ile Gln
            1060                1065                1070
Pro Lys Thr Met Gln Ile Glu Lys Ser Phe Asp Ala His Pro Arg Arg
        1075                1080                1085
Glu Ser Gln Val Arg Gln Leu Ala Trp Ile Gly Asp Gly Val Trp Val
    1090                1095                1100
Ser Ile Arg Leu Asp Ser Thr Leu Arg Leu Tyr His Ala His Thr His
105 1110 1115 1120
Gln His Leu Gln Asp Val Asp Ile Glu Pro Tyr Val Ser Lys Met Leu
                1125                1130                1135
Gly Thr Gly Lys Leu Gly Phe Ser Phe Val Arg Ile Thr Ala Leu Leu
            1140                1145                1150

Ile Ala Gly Asn Arg Leu Trp Val Gly Thr Gly Asn Gly Val Val Ile
        1155                1160                1165
Ser Ile Pro Leu Thr Glu Thr Val Val Leu His Arg Gly Gln Leu Leu
    1170                1175                1180
Gly Leu Arg Ala Asn Lys Thr Ser Pro Thr Ser Gly Glu Gly Thr Arg
185 1190 1195 1200
Pro Gly Gly Ile Ile His Val Tyr Gly Asp Asp Ser Ser Asp Lys Ala
                1205                1210                1215
Ala Ser Ser Phe Ile Pro Tyr Cys Ser Met Ala Gln Ala Gln Leu Cys
            1220                1225                1230
Phe His Gly His Arg Asp Ala Val Lys Phe Phe Val Ser Val Pro Gly
        1235                1240                1245
Asn Val Leu Ala Thr Leu Asn Gly Ser Val Leu Asp Ser Pro Ser Glu
    1250                1255                1260
Gly Pro Gly Pro Ala Ala Pro Ala Ala Asp Ala Glu Gly Gln Lys Leu
265 1270 1275 1280
Lys Asn Ala Leu Val Leu Ser Gly Gly Glu Gly Tyr Ile Asp Phe Arg

1285                    1290                    1295

Ile Gly Asp Gly Glu Asp Asp Glu Thr Glu Glu Cys Ala Gly Asp Val

        1300                    1305                    1310

Asn Gln Thr Lys Pro Ser Leu Ser Lys Ala Glu Arg Ser His Ile Ile

    1315                    1320                    1325

Val Trp Gln Val Ser Tyr Thr Pro Glu

    1330                    1335

<210> 12

<211> 1336

<212> PRT

<213> Mouse

<400> 12

Met Met Glu Ile Gln Met Asp Glu Gly Gly Gly Val Val Val Tyr Gln

1               5                   10                  15

Asp Asp Tyr Cys Ser Gly Ser Val Met Ser Glu Arg Val Ser Gly Leu

            20                  25                  30

Ala Gly Ser Ile Tyr Arg Glu Phe Glu Arg Leu Ile His Cys Tyr Asp

        35                  40                  45

Glu Glu Val Val Lys Glu Leu Met Pro Leu Val Val Asn Val Leu Glu
    50                  55                  60
Asn Leu Asp Ser Val Leu Ser Glu Asn Gln Glu His Glu Val Glu Leu
65                  70                  75                  80
Glu Leu Leu Arg Glu Asp Asn Glu Gln Leu Leu Thr Gln Tyr Glu Arg
                85                  90                  95
Glu Lys Ala Leu Arg Lys Gln Ala Glu Glu Lys Phe Ile Glu Phe Glu
            100                 105                 110
Asp Ala Leu Glu Gln Glu Lys Lys Glu Leu Gln Ile Gln Val Glu His
        115                 120                 125
Tyr Glu Phe Gln Thr Arg Gln Leu Glu Leu Lys Ala Lys Asn Tyr Ala
    130                 135                 140
Asp Gln Ile Ser Arg Leu Glu Glu Arg Glu Ser Glu Met Lys Lys Glu
145                 150                 155                 160
Tyr Asn Ala Leu His Gln Arg His Thr Glu Met Ile Gln Thr Tyr Val
                165                 170                 175
Glu His Ile Glu Arg Ser Lys Met Gln Gln Val Gly Gly Ser Gly Gln

            180                 185                 190

Thr Glu Ser Ser Leu Pro Gly Arg Arg Lys Glu Arg Pro Thr Ser Leu

        195                 200                 205

Asn Val Phe Pro Leu Ala Asp Gly Met Val Arg Ala Gln Met Gly Gly

    210                 215                 220

Lys Leu Val Pro Ala Gly Asp His Trp His Leu Ser Asp Leu Gly Gln

225                 230                 235                 240

Leu Gln Ser Ser Ser Ser Tyr Gln Cys Pro Asn Asp Glu Met Ser Glu

                245                 250                 255

Ser Gly Gln Ser Ser Ala Ala Ala Thr Pro Ser Thr Thr Gly Thr Lys

            260                 265                 270

Ser Asn Thr Pro Thr Ser Ser Val Pro Ser Ala Ala Val Thr Pro Leu

        275                 280                 285

Asn Glu Ser Leu Gln Pro Leu Gly Asp Tyr Val Ser Val Thr Lys Asn

    290                 295                 300

Asn Lys Gln Ala Arg Glu Lys Arg Asn Ser Arg Asn Met Glu Val Gln

305                 310                 315                 320

Val Thr Gln Glu Met Arg Asn Val Ser Ile Gly Met Gly Ser Ser Asp
                325                 330                 335
Glu Trp Ser Asp Val Gln Asp Ile Ile Asp Ser Thr Pro Glu Leu Asp
            340                 345                 350
Val Cys Pro Glu Thr Arg Leu Glu Arg Thr Gly Ser Ser Pro Thr Gln
        355                 360                 365
Gly Ile Val Asn Lys Ala Phe Gly Ile Asn Thr Asp Ser Leu Tyr His
    370                 375                 380
Glu Leu Ser Thr Ala Gly Ser Glu Val Ile Gly Asp Val Asp Glu Gly
385                 390                 395                 400
Ala Asp Leu Leu Gly Glu Phe Ser Val Arg Asp Asp Phe Phe Gly Met
                405                 410                 415
Gly Lys Glu Val Gly Asn Leu Leu Leu Glu Asn Ser Gln Leu Leu Glu
            420                 425                 430
Thr Lys Asn Ala Leu Asn Val Val Lys Asn Asp Leu Ile Ala Lys Val
        435                 440                 445
Asp Gln Leu Ser Gly Glu Gln Glu Val Leu Lys Gly Glu Leu Glu Ala
    450                 455                 460

Ala Lys Gln Ala Lys Val Lys Leu Glu Asn Arg Ile Lys Glu Leu Glu
465                 470                 475                 480
Glu Glu Leu Lys Arg Val Lys Ser Glu Ala Val Thr Ala Arg Arg Glu
                485                 490                 495
Pro Arg Glu Glu Val Glu Asp Val Ser Ser Tyr Leu Cys Thr Glu Leu
            500                 505                 510
Asp Lys Ile Pro Met Ala Gln Arg Arg Arg Phe Thr Arg Val Glu Met
        515                 520                 525
Ala Arg Val Leu Met Glu Arg Asn Gln Tyr Lys Glu Arg Leu Met Glu
    530                 535                 540
Leu Gln Glu Ala Val Arg Trp Thr Glu Met Ile Arg Ala Ser Arg Glu
545                 550                 555                 560
His Pro Ser Val Gln Glu Lys Lys Lys Ser Thr Ile Trp Gln Phe Phe
                565                 570                 575
Ser Arg Leu Phe Ser Ser Ser Ser Ser Pro Pro Pro Ala Lys Arg Ser
            580                 585                 590
Tyr Pro Ser Val Asn Ile His Tyr Lys Ser Pro Thr Ala Ala Gly Phe

        595                 600                 605

Ser Gln Arg Arg Ser His Ala Leu Cys Gln Ile Ser Ala Gly Ser Arg
    610                 615                 620

Pro Leu Glu Phe Phe Pro Asp Asp Asp Cys Thr Ser Ser Ala Arg Arg
625                 630                 635                 640

Glu Gln Lys Arg Glu Gln Tyr Arg Gln Val Arg Glu His Val Arg Asn
                645                 650                 655

Asp Asp Gly Arg Leu Gln Ala Cys Gly Trp Ser Leu Pro Ala Lys Tyr
            660                 665                 670

Lys Gln Leu Ser Pro Asn Gly Gly Gln Glu Asp Thr Arg Met Lys Asn
        675                 680                 685

Val Pro Val Pro Val Tyr Cys Arg Pro Leu Val Glu Lys Asp Pro Ser
    690                 695                 700

Thr Lys Leu Trp Cys Ala Ala Gly Val Asn Leu Ser Gly Trp Lys Pro
705                 710                 715                 720

His Glu Glu Asp Ser Ser Asn Gly Pro Lys Pro Val Pro Gly Arg Asp
                725                 730                 735

Pro Leu Thr Cys Asp Arg Glu Gly Glu Gly Glu Pro Lys Ser Thr His
            740                 745                 750

Pro Ser Pro Glu Lys Lys Lys Ala Lys Glu Thr Pro Glu Ala Asp Ala
        755                 760                 765

Thr Ser Ser Arg Val Trp Ile Leu Thr Ser Thr Leu Thr Thr Ser Lys
    770                 775                 780

Val Val Ile Ile Asp Ala Asn Gln Pro Gly Thr Ile Val Asp Gln Phe
785                 790                 795                 800

Thr Val Cys Asn Ala His Val Leu Cys Ile Ser Ser Ile Pro Ala Ala
                805                 810                 815

Ser Asp Ser Asp Tyr Pro Pro Gly Glu Met Phe Leu Asp Ser Asp Val
            820                 825                 830

Asn Pro Glu Asp Ser Gly Ala Asp Gly Val Leu Ala Gly Ile Thr Leu
        835                 840                 845

Val Gly Cys Ala Thr Arg Cys Asn Val Pro Arg Ser Asn Cys Ser Ser
    850                 855                 860

Arg Gly Asp Thr Pro Val Leu Asp Lys Gly Gln Gly Asp Val Ala Thr
865                 870                 875                 880

Thr Ala Asn Gly Lys Val Asn Pro Ser Gln Ser Thr Glu Glu Ala Thr
                885                 890                 895

Glu Ala Thr Glu Val Pro Asp Pro Gly Pro Ser Glu Ser Glu Ala Thr
            900                 905                 910

Thr Val Arg Pro Gly Pro Leu Thr Glu His Val Phe Thr Asp Pro Ala
        915                 920                 925

Pro Thr Pro Ser Ser Ser Thr Gln Pro Ala Ser Glu Asn Gly Ser Glu
    930                 935                 940

Ser Asn Gly Thr Ile Val Gln Pro Gln Val Glu Pro Ser Gly Glu Leu
945                 950                 955                 960

Ser Thr Thr Thr Ser Ser Ala Ala Pro Thr Met Trp Leu Gly Ala Gln
                965                 970                 975

Asn Gly Trp Leu Tyr Val His Ser Ala Val Ala Asn Trp Lys Lys Cys
            980                 985                 990

Leu His Ser Ile Lys Leu Lys Asp Ser Val Leu Ser Leu Val His Val
        995                 1000                1005

Lys Gly Arg Val Leu Val Ala Leu Ala Asp Gly Thr Leu Ala Ile Phe

1010                1015                1020

His Arg Gly Glu Asp Gly Gln Trp Asp Leu Ser Asn Tyr His Leu Met
025                1030                1035                1040

Asp Leu Gly His Pro His His Ser Ile Arg Cys Met Ala Val Val Asn
                1045                1050                1055

Asp Arg Val Trp Cys Gly Tyr Lys Asn Lys Val His Val Ile Gln Pro
            1060                1065                1070

Lys Thr Met Gln Ile Glu Lys Ser Phe Asp Ala His Pro Arg Arg Glu
        1075                1080                1085

Ser Gln Val Arg Gln Leu Ala Trp Ile Gly Asp Gly Val Trp Val Ser
    1090                1095                1100

Ile Arg Leu Asp Ser Thr Leu Arg Leu Tyr His Ala His Thr His Gln
105                1110                1115                1120

His Leu Gln Asp Val Asp Ile Glu Pro Tyr Val Ser Lys Met Leu Gly
                1125                1130                1135

Thr Gly Lys Leu Gly Phe Ser Phe Val Arg Ile Thr Ala Leu Leu Ile
            1140                1145                1150

Ala Gly Asn Arg Leu Trp Val Gly Thr Gly Asn Gly Val Val Ile Ser
        1155                1160                1165

Ile Pro Leu Thr Glu Thr Val Val Leu His Arg Gly Gln Leu Leu Gly
    1170                1175                1180

Leu Arg Ala Asn Lys Thr Ser Pro Thr Ser Gly Glu Gly Thr Arg Pro
185 1190 1195 1200

Gly Gly Ile Ile His Val Tyr Gly Asp Asp Ser Ser Asp Lys Ala Ala
                1205                1210                1215

Ser Ser Phe Ile Pro Tyr Cys Ser Met Ala Gln Ala Gln Leu Cys Phe
            1220                1225                1230

His Gly His Arg Asp Ala Val Lys Phe Phe Val Ser Val Pro Gly Asn
        1235                1240                1245

Val Leu Ala Thr Leu Asn Gly Ser Val Leu Asp Ser Pro Ser Glu Gly
    1250                1255                1260

Pro Gly Pro Ala Ala Pro Ala Ala Asp Ala Glu Gly Gln Lys Leu Lys
265 1270 1275 1280

Asn Ala Leu Val Leu Ser Gly Gly Glu Gly Tyr Ile Asp Phe Arg Ile
                1285                1290                1295

Gly Asp Gly Glu Asp Asp Glu Thr Glu Glu Cys Ala Gly Asp Val Asn
            1300                1305                1310

Gln Thr Lys Pro Ser Leu Ser Lys Ala Glu Arg Ser His Ile Ile Val
        1315                1320                1325

Trp Gln Val Ser Tyr Thr Pro Glu
    1330                1335

<210> 13
<211> 430
<212> PRT
<213> Mouse

<400> 13
Met Ser Gly Val Arg Pro Pro Ile Met Asn Gly Pro Met His Pro Arg
1               5                   10                  15

Pro Leu Val Ala Leu Leu Asp Gly Arg Asp Cys Thr Val Glu Met Pro
            20                  25                  30

Ile Leu Lys Asp Val Ala Thr Val Ala Phe Cys Asp Ala Gln Ser Thr
        35                  40                  45

Gln Glu Ile His Glu Lys Val Leu Asn Glu Ala Val Gly Ala Leu Met
    50                  55                  60

Tyr His Thr Ile Thr Leu Thr Arg Glu Asp Leu Glu Lys Phe Lys Ala
65                  70                  75                  80

Leu Arg Ile Ile Val Arg Ile Gly Ser Gly Phe Asp Asn Ile Asp Ile
                85                  90                  95

Lys Ser Ala Gly Asp Leu Gly Ile Ala Val Cys Asn Val Pro Ala Ala
            100                 105                 110

Ser Val Glu Glu Thr Ala Asp Ser Thr Leu Cys His Ile Leu Asn Leu
        115                 120                 125

Tyr Arg Arg Thr Thr Trp Leu His Gln Ala Leu Arg Glu Gly Thr Arg
    130                 135                 140

Val Gln Ser Val Glu Gln Ile Arg Glu Val Ala Ser Gly Ala Ala Arg
145                 150                 155                 160

Ile Arg Gly Glu Thr Leu Gly Ile Ile Gly Leu Gly Arg Val Gly Gln
                165                 170                 175

Ala Val Ala Leu Arg Ala Lys Ala Phe Gly Phe Asn Val Leu Phe Tyr
            180                 185                 190

Asp Pro Tyr Leu Ser Asp Gly Ile Glu Arg Ala Leu Gly Leu Gln Arg
        195                 200                 205
Val Ser Thr Leu Gln Asp Leu Leu Phe His Ser Asp Cys Val Thr Leu
    210                 215                 220
His Cys Gly Leu Asn Glu His Asn His His Leu Ile Asn Asp Phe Thr
225                 230                 235                 240
Val Lys Gln Met Arg Gln Gly Ala Phe Leu Val Asn Thr Ala Arg Gly
                245                 250                 255
Gly Leu Val Asp Glu Lys Ala Val Ala Gln Ala Leu Lys Glu Gly Arg
            260                 265                 270
Ile Arg Gly Ala Ala Leu Asp Val His Glu Ser Glu Pro Phe Ser Phe
        275                 280                 285
Ser Gln Gly Pro Leu Lys Asp Ala Pro Asn Leu Ile Cys Thr Pro His
    290                 295                 300
Ala Ala Trp Tyr Ser Glu Gln Ala Ser Ile Glu Met Arg Glu Glu Ala
305                 310                 315                 320
Ala Arg Glu Ile Arg Arg Ala Ile Thr Gly Arg Ile Pro Asp Ser Leu

                325                 330                 335

Lys Asn Cys Val Asn Lys Asp His Leu Thr Ala Ala Thr His Trp Ala

            340                 345                 350

Ser Met Asp Pro Ala Val Val His Pro Glu Leu Asn Gly Ala Ala Tyr

        355                 360                 365

Ser Arg Tyr Pro Pro Gly Val Val Ser Val Ala Pro Thr Gly Ile Pro

    370                 375                 380

Ala Ala Val Glu Gly Ile Val Pro Ser Ala Met Ser Leu Ser His Gly

385                 390                 395                 400

Leu Pro Pro Val Ala His Pro Pro His Ala Pro Ser Pro Gly Gln Thr

                405                 410                 415

Val Lys Pro Glu Ala Asp Arg Asp His Thr Ser Asp Gln Leu

            420                 425                 430

<210> 14

<211> 1508

<212> PRT

<213> Mouse

<400> 14

Met Met Ala Gly Glu Gly Ser Thr Ile Thr Ser Arg Ile Lys Asn Leu

1               5                   10                  15

Leu Arg Ser Pro Ser Ile Lys Leu Arg Arg Ser Lys Ala Gly Asn Arg

            20                  25                  30

Arg Glu Asp Leu Ser Ser Lys Val Thr Leu Glu Lys Val Leu Gly Val

        35                  40                  45

Thr Val Ser Gly Gly Arg Gly Leu Ala Cys Glu Pro Arg Ser Gly Leu

    50                  55                  60

Val Ala Tyr Pro Ala Gly Cys Val Val Val Leu Phe Asn Pro Arg Lys

65                  70                  75                  80

His Lys Gln His His Ile Leu Asn Ser Ser Arg Lys Thr Ile Thr Ala

                85                  90                  95

Leu Ala Phe Ser Pro Asp Gly Lys Tyr Leu Val Thr Gly Glu Ser Gly

            100                 105                 110

His Met Pro Ala Val Arg Val Trp Asp Val Ala Glu Arg Ser Gln Val

        115                 120                 125

Ala Glu Leu Gln Glu His Lys Tyr Gly Val Ala Cys Val Ala Phe Ser

    130                 135                 140

Pro Ser Ala Lys Tyr Ile Val Ser Val Gly Tyr Gln His Asp Met Ile
145                 150                 155                 160

Val Asn Val Trp Ala Trp Lys Lys Asn Ile Val Val Ala Ser Asn Lys
                165                 170                 175

Val Ser Ser Arg Val Thr Ala Val Ser Phe Ser Glu Asp Cys Ser Tyr
            180                 185                 190

Phe Val Thr Ala Gly Asn Arg His Ile Lys Phe Trp Tyr Leu Asp Asp
        195                 200                 205

Ser Lys Thr Ser Lys Val Asn Ala Thr Val Pro Leu Leu Gly Arg Ser
    210                 215                 220

Gly Leu Leu Gly Glu Leu Arg Asn Asn Leu Phe Thr Asp Val Ala Cys
225                 230                 235                 240

Gly Arg Gly Glu Lys Ala Asp Ser Thr Phe Cys Ile Thr Ser Ser Gly
                245                 250                 255

Leu Leu Cys Glu Phe Ser Asp Arg Arg Leu Leu Asp Lys Trp Val Glu
            260                 265                 270

Leu Arg Asn Thr Asp Ser Phe Thr Thr Thr Val Ala His Cys Ile Ser
        275                 280                 285
Val Thr Gln Glu Tyr Ile Phe Cys Gly Cys Ala Asp Gly Thr Val Arg
    290                 295                 300
Leu Phe Asn Pro Ser Asn Leu His Phe Leu Ser Thr Leu Pro Arg Pro
305                 310                 315                 320
His Ala Leu Gly Thr Asp Ile Ala Ser Ile Thr Glu Ala Ser Arg Leu
                325                 330                 335
Phe Ser Gly Gly Val Asn Ala Arg Tyr Pro Asp Thr Ile Ala Leu Thr
            340                 345                 350
Phe Asp Pro Thr Asn Gln Trp Leu Ser Cys Val Tyr Asn Asp His Ser
        355                 360                 365
Ile Tyr Val Trp Asp Val Arg Asp Pro Lys Lys Val Gly Lys Val Tyr
    370                 375                 380
Ser Ala Leu Tyr His Ser Ser Cys Val Trp Ser Val Glu Val Tyr Pro
385                 390                 395                 400
Glu Ile Lys Asp Ser His Gln Ala Cys Leu Pro Leu Ser Ser Phe Ile
                405                 410                 415

Thr Cys Ser Ser Asp Asn Thr Ile Arg Leu Trp Asn Thr Glu Ser Ser
            420                 425                 430
Gly Val His Gly Ser Thr Leu His Arg Asn Ile Leu Ser Asn Asp Leu
        435                 440                 445
Ile Lys Ile Ile Tyr Val Asp Gly Asn Thr Gln Ala Leu Leu Asp Thr
    450                 455                 460
Glu Leu Pro Gly Gly Asp Lys Ala Asp Gly Ser Leu Met Asp Pro Arg
465                 470                 475                 480
Val Gly Ile Arg Ser Val Cys Ile Ser Pro Asn Gly Gln His Leu Ala
                485                 490                 495
Ser Gly Asp Arg Met Gly Thr Leu Arg Ile His Glu Leu Gln Ser Leu
            500                 505                 510
Ser Glu Met Leu Lys Val Glu Ala His Asp Ser Glu Ile Leu Cys Leu
        515                 520                 525
Glu Tyr Ser Lys Pro Asp Thr Gly Leu Lys Leu Leu Ala Ser Ala Ser
    530                 535                 540
Arg Asp Arg Leu Ile His Glu Leu Asp Ala Gly Arg Glu Tyr Ser Leu

545                 550                 555                 560

Gln Gln Thr Leu Asp Glu His Ser Ser Ser Ile Thr Ala Val Lys Phe

                565                 570                 575

Ala Ala Ser Asp Gly Gln Val Arg Met Ile Ser Cys Gly Ala Asp Lys

            580                 585                 590

Ser Ile Tyr Phe Arg Thr Ala Gln Lys Ser Gly Glu Gly Val Gln Phe

        595                 600                 605

Thr Arg Thr His His Val Val Arg Lys Thr Thr Leu Tyr Asp Met Asp

    610                 615                 620

Val Glu Pro Ser Trp Lys Tyr Thr Ala Ile Gly Cys Gln Asp Arg Asn

625                 630                 635                 640

Ile Arg Ile Phe Asn Ile Ser Ser Gly Lys Gln Lys Lys Leu Phe Lys

                645                 650                 655

Gly Ser Gln Gly Glu Asp Gly Thr Leu Ile Lys Val Gln Thr Asp Pro

            660                 665                 670

Ser Gly Ile Tyr Ile Ala Thr Ser Cys Ser Asp Lys Asn Leu Ser Ile

        675                 680                 685

Phe Asp Phe Ser Ser Gly Glu Cys Val Ala Thr Met Phe Gly His Ser
    690                 695                 700
Glu Ile Val Thr Gly Met Lys Phe Ser Asn Asp Cys Lys His Leu Ile
705                 710                 715                 720
Ser Val Ser Gly Asp Ser Cys Ile Phe Val Trp Arg Leu Ser Ser Glu
                725                 730                 735
Met Thr Ile Ser Met Arg Gln Arg Leu Arg Glu Arg Arg Gln Arg Gln
            740                 745                 750
Arg Gly Ile Lys Gln Gln Gly Pro Thr Ser Pro Gln Arg Ala Ser Gly
        755                 760                 765
Ala Lys Gln His His Ala Pro Val Val Pro Pro Ser Gly Pro Ala Leu
    770                 775                 780
Ser Ser Asp Ser Asp Lys Glu Gly Glu Asp Glu Gly Thr Glu Glu Glu
785                 790                 795                 800
Glu Leu Pro Ala Leu Pro Ile Leu Ser Lys Ser Thr Lys Lys Glu Leu
                805                 810                 815
Ala Ser Gly Ser Ser Pro Ala Leu Leu Arg Ser Leu Ser His Trp Glu
            820                 825                 830

Met Ser Arg Ala Gln Glu Thr Met Glu Tyr Leu Asp Pro Ala Pro Val
        835                 840                 845
Ala Asn Thr Gly Pro Lys Arg Arg Gly Arg Trp Ala Gln Pro Gly Val
    850                 855                 860
Glu Leu Ser Val Arg Ser Met Leu Asp Leu Arg Gln Ile Glu Thr Leu
865                 870                 875                 880
Ala Pro Ser Pro Arg Gly Pro Ser Gln Asp Ser Leu Ala Val Ser Pro
                885                 890                 895
Ala Gly Pro Gly Lys His Gly Pro Gln Ala Pro Glu Leu Ser Cys Val
            900                 905                 910
Ser Gln Asn Glu Arg Ala Pro Arg Leu Gln Thr Ser Gln Pro Cys Ser
        915                 920                 925
Cys Pro Asp Ile Ile Gln Leu Leu Ser Gln Glu Glu Gly Val Phe Ala
    930                 935                 940
Gln Asp Leu Glu Pro Ala Pro Ile Glu Asp Gly Ile Val Tyr Pro Glu
945                 950                 955                 960
Pro Ser Asp Ser Pro Thr Met Asp Thr Ser Ala Phe Gln Val Gln Ala

                965                 970                 975

Pro Thr Gly Gly Ser Leu Gly Arg Met Tyr Pro Gly Ser Arg Gly Ser

            980                 985                 990

Glu Lys His Ser Pro Asp Ser Ala Cys Ser Val Asp Tyr Ser Ser Ser

        995                 1000                1005

Arg Leu Ser Ser Pro Glu His Pro Asn Glu Asp Ser Glu Ser Thr Glu

    1010                1015                1020

Pro Leu Ser Val Asp Gly Ile Ser Ser Asp Leu Glu Glu Pro Ala Glu

025 1030 1035 1040

Gly Asp Glu Asp Glu Glu Glu Glu Gly Gly Thr Gly Leu Cys Gly Leu

                1045                1050                1055

Gln Glu Gly Gly Pro Arg Thr Pro Asp Gln Glu Gln Phe Leu Lys Gln

            1060                1065                1070

Leu Phe Glu Thr Leu Ala Asn Gly Thr Ala Pro Gly Gly Pro Ala Arg

        1075                1080                1085

Val Leu Glu Arg Thr Glu Ser Arg Ser Ile Ser Ser Arg Phe Leu Leu

    1090                1095                1100

Gln Val Gln Thr Leu Pro Leu Arg Glu Pro Ser Leu Ser Ser Ser Gly
105                 1110                1115                1120

Leu Ala Leu Thr Ser Arg Pro Asp Gln Val Ser Gln Val Ser Gly Glu
                1125                1130                1135

Gln Leu Lys Gly Ser Gly Ala Thr Pro Pro Gly Ala Pro Pro Glu Met
            1140                1145                1150

Glu Pro Ser Ser Gly Asn Ser Gly Pro Lys Gln Val Ala Pro Val Leu
        1155                1160                1165

Leu Thr Arg Arg Arg Asn Asn Leu Asp Asn Ser Trp Ala Ser Lys Lys
    1170                1175                1180

Met Ala Ala Thr Arg Pro Leu Ala Gly Leu Gln Lys Ala Gln Ser Val
185                 1190                1195                1200

His Ser Leu Val Pro Gln Asp Glu Val Pro Ser Ser Arg Pro Leu Leu
                1205                1210                1215

Phe Arg Glu Ala Glu Thr Gln Gly Ser Leu Gly Ser Leu Pro Gln Ala
            1220                1225                1230

Gly Gly Cys Ser Ser Gln Pro His Ser Tyr Gln Asn His Thr Thr Ser
        1235                1240                1245

Ser Met Ala Lys Leu Ala Arg Ser Ile Ser Val Gly Glu Asn Pro Gly
    1250                1255                1260
Leu Ala Thr Glu Pro Gln Ala Pro Ala Pro Ile Arg Ile Ser Pro Phe
265 1270 1275 1280
Asn Lys Leu Ala Leu Pro Ser Arg Ala His Leu Val Leu Asp Ile Pro
                1285                1290                1295
Lys Pro Leu Pro Asp Arg Pro Thr Leu Thr Thr Phe Ser Pro Val Ser
            1300                1305                1310
Lys Gly Leu Thr His Asn Glu Thr Glu Gln Ser Gly Pro Leu Arg Glu
        1315                1320                1325
Pro Arg Lys Ala His Thr Thr Val Glu Lys His Ser Cys Leu Gly Glu
    1330                1335                1340
Gly Thr Thr His Lys Ser Arg Thr Glu Cys Gln Ala Tyr Pro Gly Pro
345 1350 1355 1360
Asn His Pro Cys Arg Gln Gln Leu Pro Val Asn Asn Leu Leu Gln Ala
                1365                1370                1375
Glu Ser Leu Gln Pro Leu Ser Pro Glu Lys Thr Arg Asn Pro Val Glu

            1380                1385                1390

Ser Ser Arg Pro Gly Val Ala Leu Ser Gln Asp Ser Glu Leu Ala Leu
        1395                1400                1405

Ser Leu Gln Gln Cys Glu Gln Leu Val Ala Glu Leu Gln Gly Asn Val
    1410                1415                1420

Arg Gln Ala Val Glu Leu Tyr Arg Ala Val Thr Ser Cys Lys Thr Pro
425 1430 1435 1440

Ser Ala Glu Gln Ser His Ile Thr Arg Leu Leu Arg Asp Thr Phe Ser
                1445                1450                1455

Pro Val Arg Gln Glu Leu Glu Val Leu Ala Gly Ala Val Leu Ser Ser
            1460                1465                1470

Pro Gly Gly Ser Pro Gly Ala Val Ala Ala Glu Gln Thr Gln Ala Leu
        1475                1480                1485

Leu Glu Gln Tyr Ser Glu Leu Leu Leu Arg Ala Val Glu Arg Arg Met
    1490                1495                1500

Glu Arg Arg Leu
505

<210> 15

<211> 244

<212> PRT

<213> Mouse

<400> 15

Ser Ser Lys Tyr Ser Asn Glu Ser Arg Ser Gln Ala Asp Ser Gly Phe
1               5                   10                  15

Leu Gly Leu Arg Pro Thr Ser Val Asp Pro Ala Leu Arg Arg Arg Arg
            20                  25                  30

Arg Gly Pro Arg Asn Lys Lys Arg Gly Trp Arg Arg Leu Ala Glu Glu
        35                  40                  45

Pro Leu Gly Leu Glu Val Asp Gln Phe Leu Glu Asp Val Arg Leu Gln
    50                  55                  60

Glu Arg Thr Thr Gly Gly Leu Leu Ala Glu Ala Pro Asn Glu Lys Leu
65                  70                  75                  80

Phe Phe Val Asp Thr Gly Phe Lys Arg Lys Glu Pro Arg Lys Lys Arg
                85                  90                  95

Thr Leu Val Gln Lys Lys Ser Gln Arg Leu Gln Lys Pro Leu Arg Val

            100                 105                 110

Asp Leu Ala Leu Glu Asn His Ser Lys Ile Pro Ala Pro Lys Asp Ile
        115                 120                 125

Leu Ala His Gln Val Pro Asn Ala Lys Lys Leu Arg Arg Lys Glu Glu
    130                 135                 140

Leu Trp Glu Lys Leu Ala Lys Gln Gly Glu Leu Pro Arg Asp Val Arg
145                 150                 155                 160

Lys Ala Gln Ala Arg Leu Leu Ser Pro Pro Thr Pro Lys Ala Lys Pro
                165                 170                 175

Gly Pro Gln Asp Ile Ile Glu Arg Pro Phe Tyr Asp Leu Trp Asn Pro
            180                 185                 190

Asp Asn Pro Leu Asp Thr Pro Leu Ile Gly Gln Asp Ala Phe Phe Leu
        195                 200                 205

Glu Gln Thr Lys Lys Lys Gly Val Arg Arg Pro Gln Arg Leu His Ile
    210                 215                 220

Lys Pro Ser Gln Val Pro Ala Val Glu Val Ile Pro Ala Gly Ala Ser
225                 230                 235                 240

Tyr Asn Pro Thr

<210> 16

<211> 484

<212> PRT

<213> Mouse

<400> 16
Met Ala Ala Gly Gly Asn Arg Asp Gly Glu Lys Arg Gly Ser Arg Ser
1               5                   10                  15

Gln Ala Asp Ser Gly Phe Leu Gly Leu Arg Pro Thr Ser Val Asp Pro
            20                  25                  30

Ala Leu Arg Arg Arg Arg Arg Gly Pro Arg Asn Lys Lys Arg Gly Trp
        35                  40                  45

Arg Arg Leu Ala Glu Glu Pro Leu Gly Leu Glu Val Asp Gln Phe Leu
    50                  55                  60

Glu Asp Val Arg Leu Gln Glu Arg Thr Thr Gly Gly Leu Leu Ala Glu
65                  70                  75                  80

Ala Pro Asn Glu Lys Leu Phe Phe Val Asp Thr Gly Phe Lys Arg Lys
                85                  90                  95

Glu Pro Arg Lys Lys Arg Thr Leu Val Gln Lys Lys Ser Gln Arg Leu
            100                 105                 110

Gln Lys Pro Leu Arg Val Asp Leu Ala Leu Glu Asn His Ser Lys Ile
        115                 120                 125

Pro Ala Pro Lys Asp Ile Leu Ala His Gln Val Pro Asn Ala Lys Lys

130                 135                 140

Leu Arg Arg Lys Glu Glu Leu Trp Glu Lys Leu Ala Lys Gln Gly Glu
145                 150                 155                 160

Leu Pro Arg Asp Val Arg Lys Ala Gln Ala Arg Leu Leu Ser Pro Pro
                165                 170                 175

Thr Pro Lys Ala Lys Pro Gly Pro Gln Asp Ile Ile Glu Arg Pro Phe
            180                 185                 190

Tyr Asp Leu Trp Asn Pro Asp Asn Pro Leu Asp Thr Pro Leu Ile Gly
        195                 200                 205

Gln Asp Ala Phe Phe Leu Glu Gln Thr Lys Lys Lys Gly Val Arg Arg
    210                 215                 220

Pro Gln Arg Leu His Ile Lys Pro Ser Gln Val Pro Ala Val Glu Val
225                 230                 235                 240

Ile Pro Ala Gly Ala Ser Tyr Asn Pro Thr Phe Glu Asp His Gln Ala
                245                 250                 255

Leu Leu Arg Glu Ala His Glu Val Glu Leu Gln Arg Glu Lys Glu Ala
            260                 265                 270

Glu Lys Leu Glu Arg Gln Leu Ala Leu Pro Thr Ser Glu Gln Ala Ala
        275                 280                 285

Thr Gln Glu Ser Val Phe Arg Glu Met Cys Glu Gly Leu Leu Glu Glu
    290                 295                 300

Ser Asp Gly Glu Asp Glu His Glu Ala Gly Arg Ala Gly Gln Pro Glu
305                 310                 315                 320

Ala Gly Asp Gly Thr Thr Glu Ile Ser Pro Thr Gly Ala Ala Gly Pro
                325                 330                 335

Glu Lys Arg Met Glu Lys Lys Thr Glu Gln Gln Arg Arg Arg Glu Lys

            340                 345                 350

Ala Ala Arg Lys Leu Arg Val Gln Gln Ala Ala Leu Arg Ala Ala Arg
        355                 360                 365

Leu Gln His Gln Glu Leu Phe Arg Leu Arg Gly Ile Lys Ala Gln Val
    370                 375                 380

Ala Arg Arg Leu Ala Glu Leu Ala Arg Arg Arg Glu Gln Arg Arg Ile
385                 390                 395                 400

Arg Arg Leu Ala Glu Ala Asp Lys Pro Arg Arg Leu Gly Arg Leu Lys
                405                 410                 415

Tyr Gln Ala Pro Asp Ile Asp Val Gln Leu Ser Ser Glu Leu Ser Gly
            420                 425                 430

Ser Leu Arg Thr Leu Lys Pro Glu Gly His Ile Leu Arg Asp Arg Phe
        435                 440                 445

Lys Ser Phe Gln Lys Arg Asn Met Ile Glu Pro Arg Glu Arg Ala Lys
    450                 455                 460

Phe Lys Arg Lys Tyr Lys Val Lys Leu Val Glu Lys Arg Ala Tyr Arg
465                 470                 475                 480

Glu Ile Gln Leu

<210> 17

<211> 27

<212> DNA

<213> Artificial Sequence

<220>

<223> Description of Artificial Sequence:Synthetic DNA

<400> 17
tagatatcgc cttggaacaa gagaaga
27

<210> 18
<211> 31
<212> DNA
<213> Artificial Sequence

<220>
<223> Description of Artificial Sequence:Synthetic DNA

<400> 18
atgaattctc agttgttctt tgtgacactg a 31

## Description

### TECHNICAL FIELD

The present invention relates to a novel polypeptide which binds to JNK3 which is an isozyme of c-Jun N-terminal kinase (JNK); a DNA encoding the polypeptide; a vector containing the DNA; a transformant transformed with the vector; and a method for producing the polypeptide.

Furthermore, the present invention relates to an antibody which recognizes the polypeptide; a microorganism, animal cell or animal which produces the antibody; a method of screening a compound having a JNK3 signal transduction inhibitory activity using the polypeptide, a part thereof or a microorganism, animal cell or the like expressing the polypeptide or a part thereof; and a method of screening a compound which regulates gene expression of the polypeptide using the cell.

### BACKGROUND ART

The cascade by mitogen-activated protein kinase (MAPK) which has an important role as an intracellular signal transduction molecule is an intracellular signal transduction pathway universally existing in eucaryotic organisms ranging from yeast to human.

In vertebral animals, three kinds of MAPK: extracellular signal-regulated kinase (ERK), p38 and JNK/SAPK (c-Jun amino-terminal kinase/stress-activated protein kinase), and a large number of their activating factors have been identified, and the presence of various functionally different MAPK pathways has been revealed.

It is considered that JNK, among MAPK, is activated by various extracellular stress factors, such as tumor necrosis factor-α (TNF-α), interleukin-1 (IL-1), epidermal growth factor (EGF), endotoxic lipopolysaccharide (LPS), heat shock, ultraviolet light (UV), X-ray and the like, and apoptosis (cell death) is induced by its activation (*Science*, *270*: 1326 (1995)).

That is, in cells exposed to stress, when the stress signal is transferred to small G-protein (such as Rho, Ras or the like), MEKK1 (MAPK kinase kinase 1) as a member of MAPK kinase kinase (MAPKKK) is activated, and the activated MEKK1 phosphorylates SEK1 (also called MKK4) as a member of MAPK kinase (MAPKK) to activate SEK1. The activated SEK1 phosphorylates JNK (MAPK), and the JNK is activated. It is believed that the activated JNK phosphorylates c-Jun which is one of the transcription factors in the cell nucleus, and the transcription activity of the phosphorylated c-Jun is accelerated, then apoptosis is induced in participation with AP-1, activating transcription factor 2 (ATF2) and the like. However, the process of this apoptosis in the nucleus is not well understood.

Apoptosis is induced when a neuronal cell, PC12, is cultured using a medium containing a nerve growth factor (NGF) as a proteinous nutrient factor of neuronal cells, and culturing is continued after removing NGF. It is known that increase in JNK activity is observed in apoptosis induction *(Science, 270*: 1326 (1995)).

It is known that, among three kinds of JNK (JNK1, JNK2, JNK3), JNK3 is expressed at particularly high level in the brain (*EMBO J*., *15*: 2760 (1996)).

Resistance to an attack caused by kainic acid (which is an excitatory amino acid receptor agonist) was confirmed in JNK3 knockout mice, and the apoptosis in the hippocampus CA3 region caused by kainic acid, which was found in wild type mice, was not found *(Nature, 389:* 865 (1997)). Based on this, it is considered that the neuronal protection function by the knockout of JNK3 molecule is caused by the disappearance of JNK3 pathway.

Neuronal cell death caused by apoptosis is reported in neurodegenerative diseases. That is, there are reports stating, for example, that neuronal cells undergoing apoptosis are frequently found in the hippocampus of Alzheimer's disease patients (*Experimental Neurology,* *133:* 225 (1995)), that fragmentation of DNA and apoptosis-specific changes in nuclei are observed in the brain of Alzheimer's disease patients (*Neuroreport*, *5*: 2529 (1994); *Neuroreport, 6*: 1053 (1995)), and that apoptosis of neurons in substania nigra is observed in Parkinson's disease (*J*. *Neurol*. *Sci*., *137:* 120 (1996)).

While JNK3 shows high expression in the brain, JNK1 and JNK2 are expressed in almost all tissues. The c-Jun protein, ATF2 and Elk-1 (one of the transcription factors which control expression of the gene) are considered to be the phosphorylation substrate of JNK1 and JNK2 (*Nature*, *389:* 865 (1997)).

Although JNK3 binds to the above phosphorylation substrates, since the binding affinity is weaker than that of JNK1 and JNK2 (*EMBO J*., *15:* 2760 (1996)), it is not clear whether they are true phosphorylation substrates of JNK3 in mammals.

Since the kinase activity of JNK3 can be measured using only the c-Jun protein as substrate and there are few reports in mammals about the binding protein which is considered to interact with JNK3, biochemical reactions using a protein which interacts with JNK3 and controls functions of JNK3 have been hardly studied.

As a polypeptide capable of binding to JNK3 other than the above phosphorylation substrates, JNK/SAPK-associated protein (JSAP1a) has recently been reported (*1997 Annual Meeting of The Molecular Biology Society of Japan* (December)).

As described below, it is assumed that JSAP1a functions as a scaffold protein of the JNK3 pathway. As the scaffold protein, JIP-1 (JNK interacting protein-1) which binds to JNK1 and JNK2 is known (*Science, 281*: 1671 (1998)). Variant JIP-1b having a sequence in which 47 amino acids are inserted after the 557th amino acid residue of JIP-1 is also known. The cDNA sequences encoding JIB-1 and JIP-1b have been registered in GenBank data base (accession numbers: AF003115 and AF054611, respectively).

Furthermore, it is known that STE5 protein functions as a scaffold protein in the signal transduction pathway of a mating pheromone of yeast *Saccharomyces cerevisiae*.

That is, STES protein is a scaffold protein which binds to all of the kinases STE11 (MAPKKK), STE7 (MAPKK) and FUS/KSS1 (MAPK) that exist in a series of MAP-kinase pathway in which the pheromone transfers a signal after binding to its receptor, thus having a function of transferring the signal efficiently *(Genes Dev*., *8*: 313 (1994); *Cell, 78:* 499 (1994); *Proc*. *Natl*. *Acad*. *Sci., U*.*S*.*A*., *91*: 7762 (1994)).

### DISCLOSURE OF THE INVENTION

An object of the present invention is to provide an agent for preventing or treating neurodegenerative diseases, such as Alzheimer's disease, Parkinson's disease, Huntington's disease, multiple sclerosis and the like, amyotrophic diseases such as amyotrophic lateral sclerosis and the like, ischemic diseases, brain damage due to stroke, schizophrenia, depression, epilepsy, or various immunological and inflammatory diseases, using a novel polypeptide which binds to JNK3 in the JNK3 cascade activated in response to a signal which induces stress and apoptosis, a DNA encoding the polypeptide, an antibody which recognizes the polypeptide and the like.

Activation of the JNK cascade is considered to be one of the mechanisms which cause apoptosis of cells. In addition, in neurodegenerative disease such as Alzheimer's disease or Parkinson's disease, apoptosis can be exemplified as the mechanism of cell death.

Thus, intensive studies were conducted based on a belief that an agent for treating these neurodegenerative diseases and others could be obtained if the JNK3 pathway which is especially highly expressed in the brain could be inhibited and blocked, and that an agent capable of selectively inhibiting and blocking the JNK3 pathway would provide a therapeutic agent having fewer side effects. Accordingly, the present invention has been accomplished.

Specifically, the present invention relates to the following inventions of (1) to (39).
(1) A polypeptide comprising an amino acid sequence selected from the amino acid sequences represented by any one of SEQ ID NOS:10 to 16.
(2) A polypeptide comprising an amino acid sequence in which at least one amino acid has been deleted, substituted or added in an amino acid sequence selected from the amino acid sequences represented by any one of SEQ ID NOS:14 to 16 and is capable of binding to JNK3.
   The number of amino acids which have been deleted, substituted or added is not particularly limited in the above; however, they are preferably from 1 to tens, particularly from 1 to several amino acids. Also, in order to bind the polypeptide comprising a deleted, substituted or added amino acid sequence to JNK3 in the same manner as the original polypeptide, it is preferred that it has a homology of at least 60% or more, generally 80% or more, particularly 95% or more, with the amino acid sequence of the original polypeptide. Hereinafter, the amino acid sequence in which at least one amino acid has been deleted, substituted or added means a polypeptide based on the definition described above.
   The deletion, substitution or addition of an amino acid can be carried out by the well-known site-specific mutagenesis technique, and specifically, it can be carried out according to the methods described in *Molecular Cloning, A Laboratory Manual*, 2nd ed., Cold Spring Harbor Laboratory Press (1989) (hereinafter referred to as "*Molecular Cloning,* 2nd ed."), *Current Protocols in Molecular Biology,* Supplement 1-38, John Wiley & Sons (1987-1997) (hereinafter referred to as *"Current Protocols in Molecular Biology"), Nucleic Acids Research*, *10*: 6487 (1982); *Proc*. *Natl*. *Acad. Sci*. *USA,* 79: 6409 (1982); *Gene*, *34*: 315 (1985); *Nucleic Acids Research, 13:* 4431 (1985); *Proc*. *Natl*. *Acad. Sci*. *USA*, 82: 488 (1985); *Proc. Natl*. *Acad. Sci*. *USA, 81:* 5662 (1984); *Science, 224:* 1431 (1984); PCT WO 85/00817 (1985); *Nature*, *316:* 601 (1985); and the like.
(3) A DNA which encodes the polypeptide of (1) or (2).
(4) A DNA comprising a nucleotide sequence selected from the nucleotide sequences represented by any one of SEQ ID NOS:2 to 8.
(5) A DNA which hybridizes with a DNA comprising the nucleotide sequence represented by any one of SEQ ID NOS:6 to 8 under stringent conditions, and encodes a polypeptide capable of binding to JNK3.
   The above "DNA which hybridizes with a DNA comprising the nucleotide sequence represented by any one of SEQ ID NOS:6 to 8 under stringent conditions, and encodes a polypeptide capable of binding to JNK3" means a DNA obtained by colony hybridization, plaque hybridization, Southern blot hybridization or the like using the DNA described in (3) or (4) as a probe. Examples include DNA which can be identified by carrying out hybridization at 65°C in the presence of 0.7-1.0 M NaCl using a filter on which a DNA prepared from colonies or plaques is immobilized, and then washing the filter with 0.1x to 2x SSC (saline-sodium citrate) solution (the composition of 1x SSC comprises 150 mM sodium chloride and 15 mM sodium citrate) at 65°C.
   The hybridization can be carried out according to methods described in experiment texts, such as *Molecular Cloning,* 2nd ed., *Current Protocols in Molecular Biology, DNA Cloning 1: Core Techniques, A Practical Approach,* Second Edition, Oxford University Press (1955) and the like.
   Specific examples of DNA which can be hybridized include a DNA having a homology of at least 80% or more, preferably 95% or more, with a nucleotide sequence represented by SEQ ID NOS:1 to 8 wherein the homology is calculated using BLAST (*J*. *Mol Biol*., *215:* 403 (1990), FAST (*Methods in Enzymology, 183:* 63-69) or the like.
(6) A recombinant DNA obtained by inserting the DNA of any one of (3) to (5) into a vector.
(7) The recombinant DNA according to (6), which is a recombinant DNA selected from plasmid pcDNA3-S-JSAP1b, plasmid pcDNA3-S-JSAP1c, plasmid pcDNA3-S-JSAP4, plasmid pGAD10-JSAP5, and plasmid pcDNA3-His-S-JSAP5.
(8) A transformant comprising the recombinant DNA of (6) or (7).
(9) The transformant according to (8), which is a transformant selected from a microorganism, an animal cell, a plant cell, and an insect cell.
(10) The transformant according to (9), which is a microorganism belonging to the genus *Escherichia*.
(11) The transformant according to (10), wherein the microorganism belonging to the genus *Escherichia* is a microorganism selected from *Escherichia coli* JSAP1b/pcDNA3 (FERM BP-6567), *Escherichia coli* JSAP1c/pcDNA3 (FERM BP-6568), *Escherichia coli* JSAP4/pcDNA3 (FERM BP-6569), *Escherichia coli* JSAP5/pGAD10 (FERM BP-6570), and *Escherichia coli* JSAP5/pcDNA3 (FERM BP-6928).
(12) A method for producing the polypeptide of (1) or (2), comprising culturing the transformant of any one of (8) to (11) in a medium to produce and accumulate the polypeptide of (1) or (2) in the culture, and recovering the polypeptide from the culture.
(13) An oligonucleotide which is selected from an oligonucleotide comprising a sequence identical to continuous 5 to 60 bases in a nucleotide sequence in any one of the DNA's of (3) to (5) and the DNA comprising the nucleotide sequence represented by SEQ ID NO:5, an oligonucleotide comprising a sequence complementary to the oligonucleotide, and an oligonucleotide analogue of these oligonucleotides.
(14) The oligonucleotide according to (13), wherein the oligonucleotide analogue is selected from oligonucleotide analogues in which: a phosphodiester bond is converted into a phosphorothioate bond, a phosphodiester bond is converted into an N3'-P5' phosphoamidate bond, a ribose-phosphodiester bond is converted into a peptide-nucleic acid bond, uracil is substituted with C-5 propynyluracil, uracil is substituted with C-5 thiazoleuracil, cytosine is substituted with C-5 propynylcytosine, cytosine is substituted with phenoxazine-modified cytosine, ribose is substituted with 2'-O-propylribose, and ribose is substituted with 2'-methoxyethoxyribose.
(15) A method for detecting mRNA encoding the polypeptide of (1) or (2), comprising using the oligonucleotide of (13) or (14).
(16) A method for inhibiting expression of the polypeptide of (1) or (2), comprising using the oligonucleotide of (13) or (14).
(17) An antibody which recognizes the polypeptide of (1) or (2).
(18) A method for immunologically detecting the polypeptide of (1) or (2), comprising using the antibody of (17).
(19) A method for immunohistologically staining of the polypeptide of (1) or (2), comprising using the antibody of (17).
(20) An immunohistologically staining agent, comprising the antibody of (17).
(21) A method of screening a compound having an inhibitory activity on binding of a polypeptide to JNK3, comprising bringing the polypeptide into contact with JNK3 and a test sample, said polypeptide comprising an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by any one of SEQ ID NOS:9 to 16 and being capable of binding to JNK3.
(22) A method of screening a compound having an inhibitory activity on phosphorylation of a polypeptide caused by activated JNK3, comprising bringing the polypeptide into contact with activated JNK3 and a test sample, said polypeptide comprising an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by any one of SEQ ID NOS:9 to 16 and being capable of binding to JNK3.
(23) A compound obtained by the method of (21) or (22) or a pharmacologically acceptable salt thereof.
   According to the description, the pharmacologically acceptable salt of a compound includes pharmacologically acceptable acid addition salts, metal salts, ammonium salts, organic amine addition salts, amino acid addition salts and the like.
(24) A method of screening a compound capable of changing expression of a gene encoding a polypeptide, comprising bringing a cell which expresses the polypeptide into contact with a test sample, said polypeptide comprising an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by SEQ ID NOS:9 to 16 and being capable of binding to JNK3.
(25) The method according to (24), wherein the expression of a gene is detected by the method of (15).
(26) The method according to (24), wherein the polypeptide is detected using the method of (18).
(27) A compound obtained by the method of any one of (24) to (26) or a pharmacologically acceptable salt thereof.
(28) An inhibitor of binding of a polypeptide and JNK3, wherein the polypeptide comprises an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by any one of SEQ ID NOS:9 to 16 and being capable of binding to JNK3.
(29) An inhibitor of phosphorylation of a polypeptide by activated JNK3 a polypeptide, wherein the polypeptide comprises an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by any one of SEQ ID NOS:9 to 16 and being capable of binding to JNK3.
(30) An agent for preventing neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the polypeptide of (1) or (2).
(31) An agent for treating neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the polypeptide of (1) or (2).
(32) An agent for preventing neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the oligonucleotide of (13) or (14).
(33) An agent for treating neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the oligonucleotide of (13) or (14).
(34) An agent for preventing neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the antibody of (17).
(35) An agent for treating neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the antibody of (17).
(36) A promoter DNA which controls transcription of a gene encoding the polypeptide of (1) or (2).
(37) A screening method of a compound capable of changing efficiency of transcription by the promoter DNA of (36), comprising bringing a test sample into contact with a transformant comprising a plasmid containing the promoter DNA and a reporter gene connected to the downstream of the promoter DNA; and measuring a translation product content of the reporter gene.
(38) The method according to (37), wherein the reporter gene is a gene selected from a chloramphenicol acetyltransferase gene, a β-galactosidase gene, a luciferase gene, and a green fluorescent protein gene.
(39) A compound obtained by the method of (37) or (38) or a pharmacologically acceptable salt thereof.

The present invention is explained in detail below.
<1> Acquisition of the DNA of the present invention and preparation of oligonucleotide
   (1) Production of cDNA library
      In order to produce a cDNA library, total RNA or mRNA is prepared from a suitable cell or tissue.
      As the method for preparing total RNA, a guanidine thiocyanate-cesium trifluoroacetate method *(Methods in Enzymology*, *154*: 3 (1987)), an acidic guanidine thiocyanate phenol chloroform (AGPC) method *(Analytical Biochemistry, 162*: 156 (1987); *Experimental Medicine*, *9*: 1937 (1991)) and the like can be used.
      As the method for preparing mRNA as poly(A)⁺ RNA from the total RNA, an oligo(dT)-immobilized cellulose column method (*Molecular Cloning,* 2nd ed.), an oligo-dT latex-aided method and the like can be used.
      Also, mRNA can be prepared directly from a tissue or cell by using a kit, such as Fast Track mRNA Isolation Kit (manufactured by Invitrogen), Quick Prep mRNA Purification Kit (manufactured by Pharmacia) or the like.
      Examples of suitable cell or tissue include brain cells or brain tissues of animals.
      Using the resulting total RNA or mRNA, a cDNA library is produced by a known method.
      Examples of the cDNA library production method include methods described *in Molecular Cloning,* 2nd ed., *Current Protocols in Molecular Biology, DNA Cloning 1:* Core *Techniques, A Practical Approach,* 2nd ed., Oxford University Press (1995) and the like, methods using a commercially available kit, such as Superscript Plasmid System for cDNA Synthesis and Plasmid Cloning (manufactured by Gibco BRL) and ZAP-cDNA Synthesis Kit (manufactured by Stratagene) and the like.
      Any of phage vectors, plasmid vectors and the like can be used as the cloning vector for producing cDNA library, so long as it can autonomously reproduce in *Escherichia coli* K12.
      Specific examples include ZAP Express (manufactured by Stratagene, *Strategies,* 5: 58 (1992)), pBluescript II SK(+) (*Nucleic Acids Research, 17*: 9494 (1989)), Lambda ZAP II (manufactured by Stratagene), λgt10 and λgt11 *(DNA Cloning, A Practical Approach, 1:* 49 (1985)), λTriplEx (manufactured by Clontech), λExCell (manufactured by Pharmacia), pT7T318U (manufactured by Pharmacia), pcD2 (*Mol*. *Cell. Biol*., 3: 280 (1983)), pUC18 (Gene, 33: 103 (1985)), pAMo *(J. Biol*. *Chem*., 268: 22782-22787 (1993)), alias pAMoPRC3Sc (Japanese Published Unexamined Patent Application No. 336963/93), pGAD10 (manufactured by Clontech) and the like.
      Any microorganism can be used as the host microorganism, so long as it belongs to *Escherichia coli.* Specific examples include *Escherichia coli* XL1-Blue MRF' (manufactured by Stratagene, *Strategies, 5*: 81 (1992)), *Escherichia coli* C600 *(Genetics, 39*: 440 (1954)), *Escherichia coli* Y1088 (*Science*, *222:* 778 (1983)), *Escherichia coli* Y1090 *(Science,* 222: 778 (1983)), *Escherichia coli* NM522 (*J*. *Mol. Bi*o*l*., 166: 1 (1983)), *Escherichia coli* K802 (*J*. *Mol. Biol*., *16:* 118 (1966)), *Escherichia coli* JM105 *(Gene, 38:* 275 (1985)), *Escherichia coil* SOLRTM Strain (manufactured by Stratagene), *Escherichia coli* LE392 *(Molecular Cloning,* 2nd ed.) and the like.
      In addition to the cDNA library produced by the above method, a commercially available cDNA library can also be used.
      Representative cDNA libraries include those derived from organs of human, cattle, mouse, rat, rabbit and the like which are available from Clontech, Life Tech Oriental, Health Science Research Resources Bank, Japan and the like.
   (2) Acquisition of the DNA of the present invention cDNA clone having the DNA of the present invention can be obtained from the cDNA library produced in the above (1) by the following two-hybrid system using yeast.
      A full length cDNA encoding JNK3, such as mouse JNK3 *(Nature Medicine*, *3*: 89 (1997)), is inserted into a cloning vector containing a sequence encoding the GAL4 DNA binding domain, such as the sequence of pAS2-1 (manufactured by Clontech), and then introduced into yeast CG-1945 (manufactured by Clontech).
      By the method of the above (1), a brain-derived cDNA is inserted into a cloning vector containing a sequence encoding the GAL4 transcription activation domain, such as the sequence of pGAD10 (manufactured by Clontech), to produce a cDNA library.
      The cDNA library is introduced into the above JNK3-containing CG-1945 to obtain a transformant.
      The CG-1945 is a reporter yeast strain having *HIS*3 and *1ac*Z genes which are under control of the GAL4 response sequence, and the *HIS*3 and *1ac*Z genes are expressed only when two proteins expressed from hybrid constructs of JNK3 and brain cDNA are bound. Thus, cDNA clone having the DNA of the present invention encoding a polypeptide capable of binding to JNK3 (JSAP: c-Jun N-terminal kinase/stress-activated protein kinase-associated protein) can be selected by selecting a strain from which histidine requirement has been removed (e.g., which grows on a histidine-free medium) and which has a β-galactosidase activity.
      The transformant from which histidine requirement has been removed and which has β-galactosidase activity can be obtained in one step, but the transformant of interest can also be obtained by first selecting a transformant from which histidine requirement has been removed or a transformant which has β-galactosidase activity (primary positive clone), and then selecting a transformant from which histidine requirement has been removed and which has β-galactosidase activity (secondary positive clone).
      The brain-derived cDNA fragment of interest is obtained by recovering the pGAD-derived plasmid by a known method from the resulting transformant of interest.
      Using the resulting cDNA fragment as the probe and labeling the probe, e.g., with an isotope or fluorescence, the full length cDNA of interest can be obtained by colony hybridization, plaque hybridization (*Molecular Cloning*, 2nd ed.) or the like.
      A nucleotide sequence of the DNA obtained by the above method can be determined by inserting the DNA fragment, directly or after digestion with a suitable restriction enzyme or the like, into a vector and analyzing it by a generally used nucleotide sequence analyzing method such as the dideoxy method of Sanger *et al*. (*Proc*. *Natl*. *Acad*. *Sci*. *USA*, *74*: 5463 (1997)) or using nucleotide sequence analyzing apparatus manufactured by Perkin Elmer (373A DNA sequencer), LI-COR, Pharmacia or the like.
      The DNA obtained by the above method includes DNA comprising the nucleotide sequence represented by any one of SEQ ID NOS:1 to 8.
      *Escherichia coli* JSAP1b/pcDNA3 comprising plasmid pcDNA3-S-JSAP1b containing DNA of the nucleotide sequence represented by SEQ ID NO:2, *Escherichia coli* JSAP1c/pcDNA3 comprising plasmid pcDNA3-S-JSAP1c containing DNA of the nucleotide sequence represented by SEQ ID NO:3, *Escherichia coli* JSAP4/pcDNA3 comprising plasmid pcDNA3-S-JSAP4 containing DNA of the nucleotide sequence represented by SEQ ID NO:6 and *Escherichia coli* JSAP5/pGAD10 comprising plasmid pGAD10-JSAP5 containing DNA of the nucleotide sequence represented by SEQ ID NO:7 have been deposited on November 6, 1998, and *Escherichia coli* JSAP5/pcDNA3 comprising plasmid pcDNA3-His-S-JSAP5 containing DNA of the nucleotide sequence represented by SEQ ID NO:8 on November 2, 1999, in National Institute of Bioscience and Human Technology, Agency of Industrial Science and Technology, Higashi 1-1-3, Tsukuba-shi, Ibaraki, Japan (postal code 305-8566), as FERM BP-6567, FERM BP-6568, FERM BP-6569, FERM BP-6570, and FERM BP-6928, respectively.
      Also, a DNA of interest derived from other tissue or other animal, such as human, can be obtained by selecting a DNA which hybridizes under stringent conditions with the DNA obtained by the above method.
      The DNA of interest can also be prepared by chemical synthesis using a DNA synthesizer based on the nucleotide sequence information obtained above. Examples of the DNA synthesizer include a DNA synthesizer manufactured by Shimadzu using the thiophosphite method, a DNA synthesizer model 392 manufactured by Perkin Elmer using the phosphoamidite method and the like.
      With regard to the novelty of the resulting nucleotide sequence, such can be confirmed by searching a nucleotide sequence data base, such as GenBank, EMBL, DDBJ or the like, using a homology searching program, such as BLAST or the like.
      With regard to the novel nucleotide sequences, known genes having the homology can be searched by first converting them into an amino acid sequence and then searching an amino acid sequence data base, such as GenPept, PIR, Swiss-Prot or the like, using a homology searching program, such as FASTA, FrameSearch or the like.
   (3) Preparation of the oligonucleotide of the present invention
      Oligonucleotides, such as antisense oligonucleotides, sense oligonucleotides and the like, having a partial sequence of the DNA of the present invention can be prepared by a known method or the above DNA synthesizer using the DNA of the present invention or a DNA fragment thereof obtained above.
      Examples of the oligonucleotide include DNA comprising a sequence which is the same as continuous 5 to 60 nucleotides in a nucleotide sequence of the above DNA, and DNA comprising a sequence complementary to the DNA. Specific examples include DNA comprising a continuos 5 to 60 nucleotides in a nucleotide sequence represented by SEQ ID NOS:1 to 8, and DNA having a sequence complementary to such DNA.
      When the oligonucleotide is used as the sense primer and antisense primer, the above oligonucleotide in which melting point (Tₘ) and the number of nucleotides are not significantly different from each other is preferred.
      Furthermore, analogues of the oligonucleotide can also be used as the oligonucleotide of the present invention.
      Examples of the oligonucleotide analogues include oligonucleotide analogues in which: a phosphodiester bond is converted to a phosphorothioate bond, a phosphodiester bond is converted to an N3'-P5' phosphoamidate bond, ribose and a phosphodiester bond is converted to a peptide nucleic acid bond, uracil is substituted with C-propynyluracil, uracil is substituted with C-5 thiazoluracil, cytosine is substituted with C-5 propynylcytosine, cytosine is substituted with phenoxazin-modified cytosine, ribose is substituted with 2'-O-propylribose, ribose is substituted with 2'-methoxyethoxyribose and the like (*Cell Engineering, 16*: 1463 (1997)).
<2> Preparation of the polypeptide of the present invention
   (1) Production of transformant
      Methods described in *Molecular Cloning,* 2nd ed., *Current Protocols in Molecular Biology* and the like can be used to express the DNA of the present invention encoding JSAP obtained by the method described in <1> above and produce the polypeptide of the present invention.
      Specifically, a recombinant vector to which the DNA of the present invention has been inserted into downstream of the promoter of a suitable expression vector is constructed, a transformant expressing the polypeptide of the present invention is obtained by introducing the vector into a host cell, and the transformant is cultured to produce the polypeptide of the present invention.
      Any of bacteria, yeast, animal cells, insect cells, plant cells and the like can be used as the host cell, so long as it can express the gene of interest.
      Examples of the expression vector include those which can autonomously replicate in the above host cell or which can be integrated into a chromosome and have a promoter at an operative position such that the DNA of the present invention can be transcribed.
      When prokaryote, such as a bacterium or the like, is used as the host cell, it is preferred that the expression vector of the polypeptide gene of the present invention can autonomously replicate in the prokaryote and is a recombinant vector constructed with a promoter, a ribosome binding sequence, the DNA of the present invention and a transcription termination sequence. A promoter-controlling gene can also be utilized.
      Examples of the expression vector include pBTrp2, pBTac1 and pBTac2 (all available from Boehringer Mannheim), pKK233-2 (manufactured by Pharmacia), pSE280 (manufactured by Invitrogen), pGEMEX-1 (manufactured by Promega), pQE-8 (manufactured by QIAGEN), pKYP10 (Japanese Published Unexamined Patent Application No. 110600/83), pKYP200 (*Agric*. *Biol*. *Chem*., *48*: 669 (1984)), pLSA1 (*Agric*. *Biol*. *Chem., 53:* 277 (1989)), pGEL1 (*Proc*. *Natl*. *Acad*. *Sci*. *USA*, *82:* 4306 (1985)), pBluescript II SK(-) (manufactured by Stratagene), pTrs32 (FERM BP-5408), pGHA2 (FERM B-400), pGKA2 (FERM BP-6798), pTerm2 (Japanese Published Unexamined Patent Application No. 22979/91, US 4686191, US 4939094, US 5160735), pGEX (manufactured by Pharmacia), pET (manufactured by Novagen), pSupex, pUB110, pTP5, pC194, pTrxFus (manufactured by Invitrogen), pMAL-c2 (manufactured by New England Biolabs) and the like.
      With regard to the promoter, any promoter can be used, so long as it can express in a host cell, such as *Escherichia coil, Bacillus subtilis* and the like. Examples include promoters derived from *Escherichia coli,* phage and the like, such as *trp* promoter (*Ptrp*), *lac* promoter (*Plac*), P_{L} promoter, P_{R} promoter, T7 promoter and the like; SPO1 promoter; SPO2 promoter; penP promoter; and the like. Also, artificially designed and modified promoters, such as a promoter in which two Ptrp are linked in series (Ptrpx2), tac promoter, lacT7 promoter, letI promoter or the like, can be used.
      As the ribosome binding sequence, it is preferred to use a plasmid in which the space between Shine-Dalgarno sequence and the initiation codon is adjusted to a suitable distance (for example, 6 to 18 nucleotides).
      The transcription termination sequence is not required for the expression of the DNA of the present invention. However, the transcription terminating sequence is preferably arranged at just downstream of the structural gene.
      Examples of the host cell include microorganisms belonging to the genus *Escherichia,* the genus *Serratia,* the genus *Bacillus*, the genus *Brevibacterium,* the genus *Corynebacterium,* the genus *Microbacterium*, the genus *Pseudomonas* and the like. Specific examples include *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coil* No.49, *Serratia ficaria, Serratia fonticola*, *Serratia liquefaciens,* Serratia *marcescens, Bacillus subtilis*, *Bacillus amyloliquefaciens*, *Brevibacterium ammoniagenes*, *Brevibacterium immariophilum* ATCC 14068, *Brevibacterium saccharolyticum* ATCC 14066, *Corynebacterium glutamicum* ATCC 13032, *Corynebacterium glutamicum* ATCC 14067, *Corynebacterium glutamicum* ATCC 13869, *Corynebacterium acetoacidophilum* ATCC 13870, *Microbacterium ammoniaphilum* ATCC 15354, *Pseudomonas* sp. D-0110 and the like. Any method can be used in the method for introducing the recombinant vector, so long as it is a method for introducing DNA into the above host cell. Examples include a method using a calcium ion (*Proc*. *Natl. Acad*. *Sci*. *USA, 69*: 2110 (1972)), a protoplast method (Japanese Published Unexamined Patent Application No. 248394/88), an electroporation method (*Gene*, *17*: 107 (1982); and *Molecular & General Genetics, 168*: 111 (1979)) and the like.
      When yeast is used as the host *cell,* examples of expression vector include YEp13 (ATCC 37115), YEp24 (ATCC 37051), YCp50 (ATCC 37419), pHS19, pHS15 and the like.
      Any promoter can be used so long as it can express in yeast. Examples include PHO5 promoter, PGK promoter, GAP promoter, ADH promoter, gal 1 promoter, gal 10 promoter, a heat shock polypeptide promoter, MFα1 promoter, CUP 1 promoter and the like.
      Examples of the host cell include yeast strains belonging to the genus *Saccharomyces,* the genus *Schizosaccharomyces,* the genus *Kluyveromyces,* the genus *Trichosporon,* the genus *Schwanniomyces* and the like. Specific examples include *Saccharomyces cerevisiae, Schizosaccharomyces pombe*, *Kluyveromyces lactis, Trichosporon pullulans*, *Schwanniomyces alluvius*, *Pichia pastoris* and the like.
      Any method can be used as the method for introducing the recombinant vector, so long as it is a method for introducing DNA into yeast. Examples include an electroporation method (*Methods in Enzymology*, *194*: 182 (1990)), a spheroplast method (*Proc*. *Natl*. *Acad*. *Sci*. *USA, 81:* 4889 (1978)), a lithium acetate method (*Journal of Bacteriology*, *153*: 163 (1983)) and the like.
      When an animal cell is used as the host, examples of expression vector include pcDNAI/Amp (manufactured by Invitrogen), pcDNAI and pcDM8 *(Nature, 329:* 840 (1987)), pAGE107 (Japanese Published Unexamined Patent Application No. 22979/91; *Cytotechnology,* 3: 133 (1990)), pREP4 (manufactured by Invitrogen), pAGE103 *(Journal of Biochemistry*, *101*: 1307 (1987)), pAMo, pAMoA, pAS3-3 (Japanese Published Unexamined Patent Application No. 227075/90) and the like.
      Any promoter can be used as the method for introducing the recombinant vector, so long as it can express in the animal cell. Examples include a promoter of IE (immediate early) gene of cytomegalovirus (CMV), an early promoter of SV40, a metallothionein promoter, a promoter of retrovirus, a heat shock promoter, SRα promoter and the like. Also, the enhancer of the IE gene of human CMV can be used together with the promoter.
      Examples of the animal cell include mouse myeloma cell, rat myeloma cell, mouse hybridoma cell, human Namalwa cell, human Namalwa KJM-1 cell, human fetal kidney cell, human leukemic cell, African grivet kidney cell, Chinese hamster CHO cell, HBT5637 (Japanese Published Unexamined Patent Application No. 299/88) and the like.
      Examples of the mouse myeloma cell include SP2/0, NS0 and the like. Examples of the rat myeloma cell include YB2/0 and the like. Examples of the human fetal kidney cell include BALL-1 and the like. Examples of African grivet kidney cell include COS-1, COS-7 and the like.
      Any method can be used as the method for introducing the recombinant vector into an animal cell, so long as it is a method for introducing DNA into an animal cell. Examples include an electroporation method (*Cytotechnology*, *3:* 133 (1990)), a calcium phosphate method (Japanese Published Unexamined Patent Application No. 227075/90), a lipofection method (*Proc*. *Natl*. *Acad*. *Sci*. *USA,* 84: 7413 (1987)), the method described in *Virology,* 52: 456 (1973), and the like.
      When an insect cell is used as the host, the polypeptide can be expressed by a known method described in, for example, *Bacurovirus Expression Vectors, A Laboratory Manual*, W.H. Freeman and Company, New York (1992), *Molecular Biology, A Laboratory Manual, Current Protocols in Molecular Biology*, *Bio/Technology*, *6*: 47 (1988) or the like.
      Specifically, a recombinant gene transfer vector and baculovirus are co-transfected into an insect cell to obtain a recombinant virus in an insect cell culture supernatant, and then the insect cell is infected with the resulting recombinant virus to express of the polypeptide.
      Examples of the gene transfer vector used in the method include pVL1392, pVL1393, pBlueBacIII (all manufactured by Invitrogen) and the like.
      Examples of the bacurovirus include *Autographa californica* nuclear polyhedrosis virus which infects insects of the family *Barathra* and the like.
      Examples of the insect cell include *Spodoptera frugiperda* ovary cell, *Trichoplusia ni* ovary cell, *Bombyx mori* ovary-derived culturing cell and the like.
      Examples of *Spodoptera frugiperda* ovary cell include Sf9 and Sf21 (*Bacurovirus Expression Vectors, A Laboratory Manual)* and the like. Examples of *Trichoplusia ni* ovary cell include High 5 and BTI-TN-5Bl-4 (manufactured by Invitrogen) and the like. Examples of the *Bombyx mori* ovary-derived culturing cell include *Bombyx mori* N4 and the like.
      The method for co-transfecting the above recombinant gene transfer vector and the above bacurovirus for the preparation of the recombinant virus include a calcium phosphate method (Japanese Published Unexamined Patent Application No. 227075/90), a lipofection method *(Proc. Natl. Acad*. *Sci*. *USA, 84*: 7413 (1987)) and the like.
      As the gene expression method, a secreted protein production, a fusion protein expression and the like can be effected according to the method described in *Molecular Cloning,* 2nd ed., in addition to direct expression.
      When the expression is carried out in yeast, an animal cell or an insect cell, a glycosylated polypeptide can be obtained.
      The resulting transformant is cultured in a medium, the polypeptide of the present invention is formed and accumulated in the medium, and the resulting polypeptide is recovered to produce the polypeptide of the present invention.
      Furthermore, a suitable expression vector for expressing the polypeptide of the present invention can be introduced into a cell obtained from the body of a patient, and then the cell is returned into the patient to express the polypeptide of the present invention in the patient's living body.
   (2) Culturing of the transformant
      Culturing the transformant of the present invention in a medium is carried out according to a known method used in culturing of the host.
      As a medium for culturing the transformant obtained by using, as the host, prokaryote (such as *Escherichia coli* or the like) or eukaryote (such as yeast or the like), the medium may be either a natural medium or a synthetic medium, so long as it contains a carbon source, a nitrogen source, an inorganic salt and the like which can be assimilated by the organism and the transformant can be cultured efficiently.
      Any source can be used as the carbon source, so long as it can be assimilated by the organism. Examples include carbohydrates, such as glucose, fructose, sucrose, molasses containing them, starch, starch hydrolysate and the like; organic acids, such as acetic acid, propionic acid and the like; alcohols, such as ethanol, propanol and the like; and so on.
      Examples of the nitrogen source include ammonia; ammonium salts of inorganic acids or organic acids, such as ammonium chloride, ammonium sulfate, ammonium acetate, ammonium phosphate and the like, other nitrogen-containing compounds, peptone, meat extract, yeast extract, corn steep liquor, casein hydrolysate, soybean meal and soybean meal hydrolysate, various fermented cells and hydrolysates thereof and the like.
      Examples of the inorganic salt include potassium dihydrogen phosphate, dipotassium hydrogen phosphate, magnesium phosphate, magnesium sulfate, sodium chloride, ferrous sulfate, manganese sulfate, copper sulfate, calcium carbonate and the like.
      Culturing is generally carried out under aerobic conditions by shaking culture, aeration stirring culture or the like. The culturing temperature is preferably from 15 to 40°C, and the culturing time is generally from 16 to 96 hours. The pH is maintained at 3.0 to 9.0 during the culturing. The pH is adjusted using inorganic or organic acid, an alkali solution, urea, calcium carbonate, ammonia or the like.
      Also, antibiotics, such as ampicillin, tetracycline and the like, can be added to the medium during the culturing, if desired.
      When a microorganism transformed with an expression vector using an inducible promoter as the promoter is cultured, an inducer can be added to the medium, if desired. For example, when a microorganism transformed with an expression vector using *lac* promoter is cultured, isopropyl-β-D-thiogalactopyranoside or the like can be added to the medium, and when a microorganism transformed with an expression vector using *trp* promoter is cultured, indoleacrylic acid or the like can be added to the medium.
      Examples of the medium for culturing a transformant obtained using an animal cell as the host include generally used RPMI 1640 medium *(The Journal of the American Medical Association, 199:* 519 (1967)), Eagle's MEM *(Science, 122:* 501 (1952)), DMEME (*Virology*, 8: 396 (1959)), and 199 Medium *(Proceeding of the Society for the Biological Medicine,* 73: 1 (1950)), media to which fetal calf serum or the like has been added to these media and the like.
      Culturing is generally carried out under conditions at pH of 6 to 8 and at 30 to 40°C for 1 to 7 days in the presence of 5% CO₂ or the like.
      Furthermore, if desired, antibiotics, such as kanamycin, penicillin, streptomycin and the like, can be added to the medium during the culturing.
      Examples of the medium for culturing a transformant obtained using an insect cell as the host include generally used TNM-FH medium (manufactured by Pharmingen), Sf-900 II SFM (manufactured by Life Technologies), ExCell 400 and ExCell 405 (both manufactured by JRH Biosciences), Grace's Insect Medium *(Nature, 195:* 788 (1962)) and the like.
      Culturing is generally carried out under conditions at pH of 6 to 7 and at 25 to 30°C for 1 to 5 days or the like.
      Furthermore, if desired, antibiotics, such as gentamicin and the like, can be added to the medium during the culturing.
   (3) Isolation and purification of the expressed polypeptide
      When the polypeptide of the present invention expressed by the above method is isolated and purified from the above culture medium of the transformant, usual methods for isolating and purifying enzymes can be used.
      For example, when the polypeptide of the present invention is expressed in a dissolved state inside the cells, the cells after completion of the culturing are recovered by centrifugation, suspended in an aqueous buffer and then disrupted using an ultrasonic oscillator, a French press, a Manton Gaulin homogenizer, a dynomill or the like to obtain a cell-free extract.
      A purified product can be obtained from a supernatant prepared by centrifuging the cell-free extract with a technique, such as solvent extraction; salting out with ammonium sulfate or the like; desalting; precipitation with an organic solvent or the like; anion exchange chromatography using a resin, such as diethylaminoethyl (DEAE)-Sepharose, DIAION HPA-75 (manufactured by Mitsubishi Chemical), *etc.;* cation exchange chromatography using a resin, such as S-Sepharose FF (manufactured by Pharmacia), *etc.;* hydrophobic chromatography using a resin, such as butyl-Sepharose, phenyl-Sepharose, *etc.;* gel filtration using a molecular sieve; affinity chromatography; chromatofocusing; electrophoresis, such as isoelectric focusing, *etc.;* or the like, alone or in combination.
      Also, when the polypeptide is expressed inside the cells in the form of an inclusion body, the cells are recovered and then disrupted in the same manner, the polypeptide is recovered from the precipitate fraction obtained by centrifuging the cells, and then the inclusion body of the polypeptide is solubilized with a protein modifier.
      The solubilized solution is diluted or dialyzed to a solution containing no protein denaturant or a solution containing such a low concentration of the polypeptide denaturant that the protein is not denaturated, the polypeptide having a normal stereostructure is folded, and then a purified product is obtained by an isolation and purification method similar to the above.
      When the polypeptide of the present invention or an analogue thereof, such as a glycosylated product or the like, is secreted extracellularly, the polypeptide of the present invention or the analogue thereof, such as the glycosylated product or the like, can be recovered from the culture supernatant.
      That is, the soluble fraction is obtained by treating the culture by a technique, such as centrifugation or the like, in the same manner as the above, and a purified product can be obtained from the soluble fraction by an isolation and purification method similar to the above.
      Furthermore, a fusion protein of the polypeptide of the present invention and other protein is produced, and it can be purified using affinity chromatography using a substance having affinity to the fusion protein. For example, a fusion protein of the present invention and protein A is produced according to the method of Lowe *et al*. (*Proc*. *Natl*. *Acad*. *Sci*. *USA, 86:* 8227 (1989); *Genes Develop*., *4*: 1288 (1990)), or the method described in Japanese Published Unexamined Patent Application No. 336963/93 or 823021/94, and it can be purified by affinity chromatography using immunoglubulin G.
      Moreover, the polypeptide of the present invention is produced as a fusion protein with Flag peptide, and the fusion protein can be purified by affinity chromatography using an anti-Flag antibody. Further purification can be carried out by affinity chromatography using the antibody against the polypeptide *per se.*
      Furthermore, the polypeptide of the present invention can be produced by a chemical synthesis method, such as Fmoc method (fluorenylmethyloxycarbonyl method), tBoc method (t-butyloxycarbonyl method) or the like.
      Also, the polypeptide can be chemically synthesized using a peptide synthesizer manufactured by Advanced ChemTech, Perkin-Elmer, Pharmacia, Protein Technology Instrument, Synthecell-Vega, Shimadzu Corporation or the like.
      The structure of the purified polypeptide of the present invention is determined by any method generally used in the field of protein chemistry, for example, the method described in *Protein Structure Analysis for Gene Cloning* (Hisashi Hirano, Tokyo Kagaku Dojin, 1993).
<3> Preparation of antibody which recognizes the polypeptide of the present invention
   (1) Preparation of polyclonal antibody
      A purified product of a full length or a partial fragment of the polypeptide obtained by the method described in the above <2> is used as an antigen, and administered to an animal to produce a polyclonal antibody.
      The peptide used as the antigen can be produced by a peptide synthesizer based on the amino acid sequence of the polypeptide of the present invention.
      A purified product of a full length or a partial fragment of the polypeptide obtained by phosphorylating the polypeptide obtained in the method described in the above <2> using activated JNK3 described below is used as an antigen, and administered to an animal to produce a polyclonal antibody which specifically recognizes the phosphorylated polypeptide of the present invention.
      Examples of the phosphorylated polypeptide of the present invention include the amino acid sequence represented by SEQ ID NO:9 in which at least one of 234th, 244th and 255th amino acids has been phosphorylated, the amino acid sequence represented by SEQ ID NO:10 in which at least one of 243rd, 253rd and 264th amino acids has been phosphorylated, the amino acid sequence represented by SEQ ID NO:11 in which at least one of 266th, 276th and 287th amino acids has been phosphorylated, and the amino acid sequence represented by SEQ ID NO:12 in which at least one of 265th, 275th and 286th amino acids has been phosphorylated.
      Examples of the animal to be administered include rabbits, goats, 3- to 20-week old rats, mice, hamsters and the like.
      The dose of the antigen is preferably 50 to 100 µg per animal.
      When the peptide is used, the peptide which covalently binds to a carrier protein, such as keyhole limpet haemocyanin, bovine thyroglobulin or the like, is preferably used as the antigen.
      The administration of the antigen is carried out 3 to 10 times at the intervals of 1 or 2 weeks after the first administration. Three to seven days after each administration, blood is collected from the venous plexus of the eyeground, and it is confirmed that the serum reacts with the antigen by the enzyme immunoassay *(Enzyme Immunoassay (ELISA),* Igaku Shoin (1988), *Antibodies - A Laboratory Manual*, Cold Spring Harbor Laboratory (1988)) or the like.
      A serum is obtained from a non-human mammal having a sufficient antibody titer against the antigen used for the immunization, and the serum is isolated and purified to obtain a polyclonal antibody.
      The methods for the isolation and purification include centrifugation, salting out by 40-50% ammonium sulfate, caprylic acid precipitation *(Antibodies, A Laboratory manual,* Cold Spring Harbor Laboratory (1988)), and chromatography using a DEAE-Sepharose column, an anion exchange column, a protein A or G-column, a gel filtration column and the like, which are used alone or in combination.
   (2) Preparation of monoclonal antibody
   (2-1) Preparation of antibody-producing cell
      Three to seven days after the antigen substance is finally administered to a rat having a serum showing a sufficient antibody titer, the rat spleen is excised.
      The spleen is cut to pieces in MEM (manufactured by Nissui Pharmaceutical), loosened using a pair of forceps, followed by centrifugation at 1,200 rpm for 5 minutes, and the resulting supernatant is discarded.
      The spleen cells in the precipitated fraction is treated with a Tris-ammonium chloride buffer (pH 7.65) for 1 to 2 minutes to eliminate erythrocytes and washed three times with MEM, and the resulting spleen cells are used as antibody-producing cells.
   (2-2) Preparation of myeloma cells
      An established cell line derived from a mouse or a rat is used as myeloma cells. Examples include 8-azaguanine-resistant mouse (BALB/c) myeloma cell lines, such as P3-X63Ag8-U1 (P3-U1) (*Curr*. *Topics in Microbiol. Immunol*., *81:* 1 (1978), *Eur*. *J*. *Immunol*., *6*: 511 (1976)), SP2/O-Ag14 (SP-2) *(Nature,* 276: 269 (1978)), P3-X63-Ag8653 (653) (*J*. *Immunol*., *123:* 1548 (1979)), P3-X63-Ag8 (X63) (*Nature*, *256*: 495 (1975)) and the like. These cell lines were subcultured in a 8-azaguanine medium (medium in which, to a medium obtained by adding glutamine (1.5 mM), 2-mercaptoethanol (5×10⁻⁵ M), gentamicin (10 µg/ml) and fetal calf serum (FCS) (manufactured by CSL, 10%) to RPMI-1640 medium (hereinafter referred to as the "normal medium"), 8-azaguanine (15 µg/ml) has been further added) and cultured in the normal medium 3 or 4 days before cell fusion, and 2×10⁷ or more of the cells are used for the fusion.
   (2-3) Production of hybridoma
      The antibody-producing cells obtained in (2-1) and the myeloma cells obtained in (2-2) are washed with MEM or PBS (disodium hydrogen phosphate: 1.83 g, sodium dihydrogen phosphate: 0.21 g, sodium chloride: 7.65 g, distilled water: 1 liter, pH: 7.2) and mixed to give a ratio of antibody-producing cells : myeloma cells = 5 to 10 : 1, followed by centrifugation at 1,200 rpm for 5 minutes, and the supernatant is discarded.
      The cells in the resulting precipitated fraction were thoroughly loosened, 0.2 to 1 ml of a mix solution of 2 g polyethylene glycol-1000 (PEG-1000), 2 ml MEM and 0.7 ml dimethylsulfoxide (DMSO) per 10⁸ antibody-producing cells is added to the cells under stirring at 37°C, and then 1 to 2 ml of MEM is further added thereto several times at 1 to 2 minute intervals.
      After the addition, MEM is added to prepare a total amount of 50 ml.
      The resulting prepared solution is centrifuged at 900 rpm for 5 minutes, and then the supernatant is discarded.
      The cells in the resulting precipitated fraction were gently loosened and then gently suspended in 100 ml of HAT medium (the normal medium to which hypoxanthine (10⁻⁴ M), thymidine (1.5×10⁻⁵ M) and aminopterin (4×10⁻⁷ M) have been added) by drawing up into and discharging from a measuring pipette.
      The suspension is poured into a 96 well culture plate at 100 µl/well and cultured at 37°C for 7 to 14 days in a 5% CO₂ incubator.
      After culturing, a part of the culture supernatant is recovered, and a hybridoma which specifically reacts with an antigen used for the immunization is selected to obtain the above antibody-producing cells according to the enzyme immunoassay described in *Antibodies, A Laboratory manual,* Cold Spring Harbor Laboratory Press, Chapter 14 (1998) or the like.
      A specific example of the enzyme immunoassay is described below.
      A full length or a partial fragment of the polypeptide of the present invention used as the antigen in the immunization is coated on a suitable plate, the hybridoma-culturing supernatant or a purified antibody described in (2-4) below is allowed to react therewith as the first antibody, an anti-rat immunoglobulin antibody labeled with biotin, an enzyme, a chemiluminescent substance, a radioactive compound or the like is allowed to react therewith as the second antibody, and a hybridoma which specifically reacts with the polypeptide of the present invention is selected as a hybridoma capable of producing the monoclonal antibody against the polypeptide of the present invention.
      Cloning is repeated using the hybridoma twice by limiting dilution analysis first in HT medium (a medium in which aminopterin has been removed from HAT medium) and then using normal medium, and a hybridoma which has a stable and sufficient antibody titer is selected as a hybridoma capable of producing the monoclonal antibody of the present invention.
   (2-4) Preparation of monoclonal antibody.
      The hybridoma cells capable of producing the polypeptide of the present invention obtained in (2-3) are injected intraperitoneally into a 8- to 10-week-old mouse or a nude mouse intraperitoneally treated with 0.5 ml of 2,6,10,14-tetramethylpentadecane (pristane), followed by 2 weeks of feeding at 5×10⁶ to 20x10⁶ cells/animal. The hybridoma causes ascites tumor in 10 to 21 days.
      The ascitic fluid is collected from the mouse or nude mouse, and centrifuged to remove solid contents.
      A monoclonal antibody can be purified and isolated from the resulting supernatant according to the method similar to that used in the polyclonal antibody.
      The subclass of the antibody can be determined using a mouse monoclonal antibody typing kit or a rat monoclonal antibody typing kit. The protein amount can be determined by the Lowry method or by calculation based on the absorbance at 280 nm.
<4> Screening method of useful medicament using novel polypeptide which can bind to JNK3
   (1) Preparation of fusion polypeptide between tag polypeptide and the polypeptide of the present invention used for screening
      1) Preparation of fusion polypeptide with thioredoxin·S-tag (hereinafter referred to as "Trx·S") peptide
         A vector capable of expressing a Trx·S-JSAP fusion polypeptide can be prepared by inserting a full length or a partial length DNA of cDNA encoding the polypeptide capable of binding to JNK3 (JSAP) obtained in <1> above into the downstream of the Trx·S sequence of an expression vector containing the Trx·S sequence, such as pET32 (manufactured by Novagen). In the same manner, a vector capable of expressing a Trx·S-ATF2-JSAP fusion polypeptide can be prepared by inserting the same into the downstream of a Trx·S-ATF2 sequence of an expression vector containing the Trx·S-ATF2 sequence, such as pET32a (manufactured by Novagen).
         The fusion polypeptide can be obtained using the resulting expression vector according to the method described in <2> above.
      2) Preparation of fusion polypeptide with S-tag peptide
         A vector capable of expressing an S-JSAP fusion polypeptide can be prepared by inserting a full length or a partial length DNA of cDNA encoding JSAP into the downstream of the S-tag sequence of an expression vector containing the S-tag sequence, such as an s-modified pcDNA3 in which the S-tag sequence has been inserted into pcDNA3 (manufactured by Invitrogen).
         The fusion polypeptide can be obtained using the resulting expression vector according to the method described in <2> above.
      3) Preparation of fusion polypeptide with GAL4AD peptide
         A vector capable of expressing a GAL4AD-JSAP fusion polypeptide can be prepared by inserting a full length or a partial length DNA of cDNA encoding JSAP into the downstream of a GAL4AD sequence of an expression vector containing the GAL4AD sequence, such as pGAD10 (manufactured by Clontech).
         The fusion polypeptide can be obtained using the resulting expression vector according to the method described in <2> above.
      4) Preparation of fusion polypeptide with Flag peptide
         A vector capable of expressing a Flag-JSAP fusion polypeptide can be prepared by inserting a full length or a partial length DNA of cDNA encoding JSAP into the downstream of a Flag sequence of an expression vector containing the Flag sequence, such as pFlag-CMV-2 (manufactured by Kodak) or Flag-modified pcDNA3 vector (manufactured by Invitrogen) in which a Flag tag sequence has been inserted into pcDNA3.
         The fusion polypeptide can be obtained using the resulting expression vector according to the method described in <2> above.
      5) Preparation of fusion polypeptide with glutathione S-transferase (hereinafter referred to as "GST")
         A vector capable of expressing a GST-JSAP fusion polypeptide can be prepared by inserting a full length or a partial length DNA of cDNA encoding JSAP into the downstream of a GST sequence of an expression vector containing the GST sequence, such as pGEX or pGEX-3X (both manufactured by Pharmacia).
         The fusion polypeptide can be obtained using the resulting expression vector according to the method described in <2> above. The fusion polypeptide can be purified using a Glutathione Sepharose 6B column (manufactured by Pharmacia).
      6) Preparation of fusion polypeptide with Myc tag peptide
         A vector capable of expressing a Myc-JSAP fusion polypeptide can be prepared by inserting a full length or a partial length DNA of cDNA encoding JSAP into the downstream of a Myc tag sequence of an expression vector containing the Myc tag sequence, such as a Myc-modified pcDNA3. Myc-modified pcDNA3 (manufactured by Invitrogen) is pcDNA3 modified by connecting a Myc tag code sequence to the upstream of the gene to express the protein to which the tag has been added.
         The fusion polypeptide can be obtained using the resulting expression vector according to the method described in <2> above.
      7) Preparation of fusion polypeptide with His-S-tag peptide

      A vector capable of expressing a His-S-JSAP fusion polypeptide can be prepared by inserting a full length or a partial length DNA of cDNA encoding JSAP into the downstream of a His-S-tag sequence of an expression vector containing the His-S-tag sequence, such as a His-S-modified pcDNA3. His-S-modified pcDNA3 (manufactured by Invitrogen) is pcDNA3 modified by connecting a His-S tag code sequence to the upstream of the gene to express the protein to which the tag has been added.
      The fusion polypeptide can be obtained using the resulting expression vector according to the method described in <2> above.
      According to the methods of the above 1) to 7), fusion polypeptides with the polypeptides related to the MAP kinase cascade can be prepared in the same manner.
   (2) Preparation of activated JNK3
      A vector is prepared by inserting a DNA encoding the full length or a partial length of Flag-JNK3 prepared according to the method of <4>(1) above into pFlag-CMV-2.
      Also, a DNA encoding a ΔMEKK1 (a peptide containing from the 1169th to 1488th amino acid residues of MEKK1 and constantly activated) is inserted into expression vector pEF-BOS.
      COS-7 cells are transfected with both of the resulting expression vectors using TransIT-LT1 (manufactured by Mirus), and the resulting COS-7 cells are cultured according to a known method to temporally express the Flag-JNK3 fusion polypeptide and ΔMEKK1.
      After culturing for 24 to 48 hours, the cells are dissolved in a buffer B (50 mM HEPES (pH 7.5), 150 mM NaCl, 1% NP-40, 10% glycerol, 2 mM MgCl₂, 1 mM EGTA, 20 mM β-glycerophosphate, 2 mM Na₃VO₄, 1 mM PMSF, 0.2 mM DTT), and the activated Flag-JNK3 is purified using an anti-Flag antibody column (manufactured by Kodak).
      The activated Flag-JNK3 can be stored in a buffer containing from 20 to 50% glycerin or a buffer containing no glycerin at -20°C to -80°C, and can be used by thawing prior to use.
   (3) Screening system using JNK3 binding activity as marker
      Since the JNK3 pathway can be inhibited by finding a compound which inhibits binding of JNK3 with each of JSAP obtained in <2> above, it is useful in preventing and treating a disease caused by the JNK3 pathway such as apoptosis or the like.
      The method of screening a compound having an activity of inhibiting each of JSAP with JNK3 is explained in detail below.
      1) Screening system 1 (method using cultured cells)
         Transfection of COS-7 cells with the S-JSAP expression vector obtained according to the method of <4>(1) above and the Flag-JNK3 expression vector is carried out using TransIT-LT1 (manufactured by Mirus), and the resulting COS-7 cells are cultured according to a known method in the presence or absence of a test compound to transiently express the S-JSAP and Flag-JNK3 fusion polypeptide.
         After culturing for 24 to 48 hours, the cultured cells are dissolved in the buffer B, S-protein agarose is added thereto, and then S-JSAP and polypeptide which binds to S-JSAP are precipitated and recovered.
         The recovered polypeptide fraction is separated by SDS-PAGE and transferred on membrane Immobilon-P (manufactured by Millipore).
         Western blotting is carried out using the membrane and anti-Flag M5 monoclonal antibody (manufactured by Kodak) as the probe, and the polypeptide which binds to the antibody (Flag-JNK3) is visualized using an ECL detection system (manufactured by Amersham) to determine the amount of JNK3 bound to JSAP.
         Accordingly, a compound which inhibits binding with JNK3 can be detected using as a control the case in which the test compound is not added.
         Examples of the test sample include synthetic compounds, natural proteins, artificially synthesized proteins, peptides, saccharides, lipids and modified products and derivatives thereof, as well as urine, body fluids, tissue extracts, cell culture supernatants and cell extracts of mammals (e.g., mouse, rat, guinea pig, hamster, swine, sheep, bovine, horse, dog, cat, monkey, human and the like), and also non-peptide compounds, fermentation products, extracts of plants and other organisms and the like. In the screening method described below, the test sample similar to these can be used.
      2) Screening system 2 (ELISA)
         The GST-JSAP fusion polypeptide prepared according to the method in <4>(1) above as such, or a JSAP polypeptide fragment obtained by digesting it with a protease factor Xa (manufactured by Sigma), is used in the binding reaction. Hereinafter, these polypeptides are called JSAP-related polypeptides.
         The polypeptides can be stored at -80°C to -20°C in a buffer containing from 20 to 50% of glycerin or a buffer containing no glycerin, which can be used by thawing prior to use.
         Each of the above JSAP-related polypeptides is added to a 96 well plate.
         The activated JNK3 prepared in <4>(2) is (or the activated JNK3 and a test compound are) added to a buffer such as a binding buffer (50 mM Tris-HCl (pH 7.5), 150 mM NaCl, 0.5% NP-40), followed by stirring.
         The thus stirred solution is added to the above plate.
         After the addition, the plate is incubated at 4°C for 1 to 2 hours.
         After incubation, the plate is washed three times with the same buffer, and the remaining activated JNK3 is determined by ELISA.
         For example, the amount of the remaining activated JNK3 can be determined by ELISA using Phosho-SAPK/JNK(Thr183/Tyr185) antibody (manufactured by New England Biolabs) capable of recognizing the activated JNK3 as the first antibody, and using an antibody capable of recognizing the above antibody as the second antibody.
         By the method, a compound which inhibits binding with JNK3 can be detected using as a control the case in which the test compound is not added.
   (4) Screening system using phosphorylation of JSAP1 by JNK3 as marker
      Because of the information regarding a) to c) obtained by Examples described below, establishment of a method for screening a compound having an inhibitory activity on phosphorylation of JSAP1 is important in detecting a compound useful in preventing and treating a disease caused by the JNK3 pathway.
      a) Inactivated JNK3 is bound to scaffold polypeptides JSAP1a, b, c and d which are selectively expressed in the brain.
      b) When the JNK3 pathway is activated (MAPKKK → MAPKK → JNK3) by various extracellular stresses, such as THF-α, IL-1, EGF, LPS and the like, JNK3 per se is activated (phosphorylated), and the activated JNK3 phosphorylates JSAP1a, b, c and d.
      c) After the phosphorylation of JSAP1, JNK3 is released from JSAP1 and transferred into the nucleus.

      It is believed that the JNK3 transferred into the nucleus activates various transcription factors and leads the cell into apoptosis and the like. Thus, a compound having an inhibitory activity on phosphorylation of JSAP1 by this JNK3 can inhibit the JNK3 pathway and therefore is useful in preventing and treating a disease caused by the JNK3 pathway, such as apoptosis or the like.
      A method for screening a compound having an inhibitory activity on phosphorylation of JSAP1 by JNK3 is described below in detail.
      1) Screening system (cell-free system)
      1)-1 Screening system 1 (method using radioisotope)
         A screening test solution is prepared by adding (i) GST-JSAP1 prepared according to the method described in <4>(1) or JSAP1 and (ii) the activated JNK3 prepared according to the method described in <4>(2) or activated JNK3 and a test compound to a buffer, such as a buffer A (20 mM HEPES, 10 mM MgCl₂, 5 mM β-mercaptoethanol, 0.1 mg/ml BSA (pH 7.4)) and mixing them. The screening test solution is added to a 96 well plate.
         After the addition, [γ-³²P]ATP or [γ-³³P]ATP is added to the plate. After the reaction for 5 to 30 minutes, 50 mM EDTA is added to stop the reaction.
         After termination of the reaction, the polypeptide in the reaction solution is trapped by vacuum filtration on the membrane of each well of a 96 well nitrocellulose membrane plate, a 96 well phosphocellulose membrane plate or a 96 well PVDF membrane plate, and the membrane is washed by aspiration with the same buffer.
         The membrane is put into a liquid scintillator MicroScint-20 (manufactured by Packard) and the ³²P or ³³P radioactivity on the membrane is counted using Top Count (manufactured by Packard).
         By the method, a compound having an inhibitory activity on phosphorylation of JSAP1 by JNK3 can be detected using as a control the count of a case in which the test compound is not added.
      1)-2 Screening system 2 (ELISA)
         An antibody (first antibody) which recognizes JSAP1 obtained according to the method described in <3> is coated on a 96 well plate.
         To a screening test solution prepared according to the method described in 1)-1, 50 µM of ATP is added.
         After the addition, the reaction is carried out at room temperature for 5 to 60 minutes, and the reaction is terminated with 0.25 N HCl.
         After the termination, the reaction solution is neutralized with a solution containing 0.25 N NaOH and 0.1 M Tris-HCl (pH 8.0).
         An aliquot of the neutralized solution is added to the above plate coated with the first antibody and allowed to stand.
         After the standing, the plate is washed with the same buffer, and then an antibody (second antibody) which recognizes the phosphorylated JSAP1 obtained according to the method described in <3> is added thereto.
         By determining the amount of the second antibody by ELISA in a known method, the amount of the phosphorylated JSAP1 is determined.
         By the method, a compound having an inhibitory activity on phosphorylation of JSAP1 by JNK3 can be detected using as a control the amount of phosphorylated JSAP1 in a case in which the test compound is not added.
         Although the compound which inhibits binding of the polypeptide of the present invention with JNK3 or the compound having the inhibitory activity on phosphorylation of JSAP1, obtained by the methods in the above 1)-1 and 1)-2, can be used directly as a therapeutic agent, generally, it is preferred to use the compound as a pharmaceutical preparation produced by a well-known method in the technical field of pharmaceutics by mixing it with at least one pharmaceutically acceptable carrier.
         As the administration method of the therapeutic agent, it is preferred to use the most effective method in carrying out the treatment, and methods by oral administration or by parenteral administration, such as buccal, airway, rectal, subcutaneous, intramuscular, intravenous or the like, can be used.
         Examples of dosage form of the therapeutic drug include ointments, sprays, capsules, tablets, granules, syrups, emulsions, suppositories injections, tapes and the like.
         Examples of preparations suitable for oral administration include emulsions, syrups, capsules, tablets, powders, granules and the like.
         Liquid preparations such as emulsions and syrups can be produced using, as additives, water; saccharides, such as sucrose, sorbitol, fructose, *etc.*; glycols, such as polyethylene glycol, propylene glycol, *etc.*; oils, such as sesame oil, olive oil, soybean oil, *etc.;* antiseptics, such as p-hydroxybenzoates, *etc*.; flavors, such as strawberry flavor, peppermint, *etc.;* and the like.
         Capsules, tablets, powders, granules and the like can be produced using, as additive, fillers, such as lactose, glucose, sucrose, mannitol, *etc.*; disintegrants, such as starch, sodium alginate, etc.; lubricants, such as magnesium stearate, talc, etc.; binders, such as polyvinyl alcohol, hydroxypropylcellulose, gelatin, *etc.;* surfactants, such as fatty acid ester, *etc*.; plasticizers, such as glycerin, etc.; and the like.
         Examples of the preparations suitable for parenteral administration include injections, suppositories, sprays and the like.
         The injections can be prepared using, for example, a carrier comprising a salt solution, a glucose solution, or a mixture of both or the like.
         The suppositories can be produced using, for example, a carrier, such as cacao butter, hydrogenated fat, carboxylic acid or the like.
         As the sprays, the phosphorylation inhibitor or binding inhibitor obtained in the foregoing can be used directly as sprays, but sprays prepared using a carrier or the like which does not stimulate buccal and airway mucous membranes of the recipient and can facilitate absorption of the compound by dispersing it as fine particles are preferred.
         Examples of the carrier include lactose, glycerol and the like.
         Depending on the properties of the agonist or antagonist obtained in the foregoing and of the carrier, it is possible to prepare pharmaceutical preparations, such as aerosols, dry powders and the like.
         The components exemplified as additive agents of oral preparations can also be added to these parenteral preparations.
         The dose or frequency of administration varies depending on the intended therapeutic effect, administration method, treating period, age, body weight and the like, but is generally from 10 µg/kg to 8 mg/kg per day and per adult.
<5> Screening and identification of compound which controls expression of the polypeptide of the present invention (hereinafter referred to as "expression-controlling compound")
   (1) Screening and identification of expression-controlling compound using the antibody of the present invention
      After allowing a cell expressing the polypeptide of the present invention to contact with a test sample, an expression-controlling compound existing in the cell or a cell culture supernatant can be screened and identified using the antibody of the present invention.
      As the cell, any one of cells, cell lines and tissues which express the polypeptide of the present invention can be used.
      Also, a cell, a cell line or tissue in which expression of the polypeptide was observed using the method for the immunological detection with the antibody described in <3> can be used.
      As a suitable cell line, a mouse-derived P19 cell (ATCC CRL-1825) which is differentiated to be a neuronal cell like with retinoic acid is exemplified.
      As the test sample, those which were exemplified in <4> above can be used.
      Cells expressing the polypeptide of the present invention are suspended in a medium in which the cells can grow, each test sample is added to the medium for contact with the cells, and then the amount of the polypeptide expressed in the cells is determined using the antibody of the present invention. As the determination method using the antibody, the immunocytochemistry described below is exemplified.
      Cultured adherent cells are washed with PBS, 3 ml of PBS containing 0.05% of trypsin and 0.02% of ethylenediaminetetraacetic acid (EDTA) was added thereto, and then, after removing an extra solution, incubated at 37°C for 5 minutes to peel off the cells from the flask.
      The suspension cells can be used as cultured cells, as they are.
      Cells used in the immunocytochemistry are suspended in a buffer for immunocytochemistry (PBS containing 1% BSA, 0.02% EDTA and 0.05% sodium azide) or the like and dispensed into a round bottom 96-well plate at 1×10⁵ to 20×10⁵ cells per well.
      The monoclonal antibody of the present invention is dispensed into the plate.
      As the monoclonal antibody, culture supernatant of the hybridoma obtained in <3>(2-3) capable of producing the monoclonal antibody of the present invention and the purified monoclonal antibody obtained in <3>(2-4) can be exemplified. Additionally, an antibody prepared by labeling the monoclonal antibody can be used.
      As the antibody prepared by labeling the monoclonal antibody, a biotin-labeled antibody can be exemplified.
      The biotin-labeled antibody can be prepared by a known method *(Enzyme Antibody Method,* published by Gakusai Kikaku (1985)).
      The above antibody is diluted to a concentration of 0.1 to 50 µg/ml using the buffer for immunocytochemistry or the buffer for immunocytochemistry containing 10% animal serum.
      The diluted antibody is dispensed to give an amount of 20 to 500 µl/well and incubated on ice for 30 minutes.
      When the unlabeled antibody is used, the cells are washed by adding the buffer for immunocytochemistry to the above plate, the buffer for immunocytochemistry containing about 0.1 to 50 µg/ml of an anti-mouse immunoglobulin antibody or anti-rat immunoglobulin antibody labeled with fluorescence dye, such as fluorescein isothiocyanate (FITC), phycoerythrin or the like, is dispensed at about 50 to 500 µl/well, and then the cells are incubated on ice in the dark for 30 minutes.
      When the monoclonal antibody labeled with biotin is used, streptoavidin labeled with a fluorescence dye, such as FITC, phycoerythrin or the like, is dispensed at about 50 to 500 µl/well into the above plate, and then incubated on ice in the dark for 30 minutes.
      In both cases, after incubation, the cells are thoroughly washed by adding the buffer for immunocytochemistry to the plate, and then analyzed by a fluorescence microscopy, a cell sorter or the like.
      The analyzed result obtained by carrying out the same procedure without adding test samples is compared with the analyzed result obtained by adding test samples, and a test sample capable of increasing or decreasing the amount of the polypeptide of the present invention is screened and thereby identified as an expression-controlling compound.
   (2) Screening and identification using transcription product determination system of the gene of the polypeptide of the present invention
      An expression-controlling compound can be screened and identified by allowing cells which express the polypeptide of the present invention or mRNA encoding the polypeptide to contact with each test sample, and then determining the amount of the mRNA.
      Cells which express the polypeptide of the present invention or mRNA encoding the polypeptide are suspended in a medium in which the cells can grow, each test sample is added to the medium for contact with the cells, and then the amount of the mRNA expressed by the cells is determined using a known Northern hybridization, RNA dot blot hybridization, RT-PCR or the like.
      As probes which can be used in the hybridization and the like and primers which can be used in the RT-PCR and the like, fragments of the gene which encode the polypeptide of the present invention can be exemplified.
      Specifically, an oligonucleotide having a sequence identical to continuous 5 to 60 bases in the nucleotide sequence represented by SEQ ID NOS:1 to 8 or an oligonucleotide having a sequence complementary to the oligonucleotide can be preferably used.
      The determination result obtained by carrying out the same procedure without adding test samples is compared with the determination result obtained by adding test samples, and a test sample capable of increasing or decreasing amount of mRNA encoding the polypeptide of the present invention is screened and thereby identified as an expression-controlling compound.
   (3) Screening and identification using reporter gene
      A cell transformed with a plasmid containing a DNA in which a reporter gene is connected to the downstream of a region which controls transcription of a gene which encodes the polypeptide of the present invention (hereinafter referred to as "transcription-controlling region") is allowed to contact with each test sample, and then an expression-controlling compound can be screened and identified by determining the amount of the expressed polypeptide encoded by the reporter gene.
      In general, the transcription-controlling region is contained in the 5' upstream of genes in most cases. The 5' upstream region of the gene encoding the polypeptide of the present invention can be prepared by, for example, using Genome Walker kits (manufactured by Clontech) or the like. Also, a fragment obtained by digesting the region into a suitable length using suitable restriction enzymes can be used as the transcription-controlling region.
      Any gene can be used as the reporter gene, so long as a translation product of the gene is stable inside the cells and the existing amount of the translation product can easily be determined. Examples include chloramphenicol acetyltransferase (CAT), β-galactosidase (β-gal), luciferase (luc), green fluorescent protein (GFP) and the like.
      As the host cell into which a reporter plasmid containing the transcription-controlling region is introduced, any cell can be used; however, a cell line in which expression of the polypeptide of the present invention or mRNA encoding the polypeptide is confirmed as described in <5>(1) is preferably used.
      As the test samples, those described in <4> above can be used.
      The host cell is transformed by a known method using a plasmid prepared by connecting the reporter gene to the downstream of the transcription-controlling region by a known method.
      Alternatively, a cell line in which a part of the gene encoding the polypeptide of the present invention is substituted with a reporter gene can be obtained by preparing a gene targeting vector in which a positive selection marker (G418 resistance gene or the like) and a negative selection marker (thymidine kinase gene of herpes simplex virus, diphtheria toxin A fragment gene or the like) are connected (*Nature*, *336:* 348 (1988); *Analytical Biochemistry, 214:* 77 (1993); *Gene Targeting, The Practical Approach Series,* IRL Press (1993)).
      The transformant cells are suspended, for example, in a medium in which the cells can grow, each test sample is added to the medium for contact with the cells, and then the amount of the polypeptide encoded by the reporter gene expressed by the cells is detected and determined using a method suitable for the polypeptide.
      Examples of the detection and determination method include the method described in, for example, *Molecular Cloning,* 2nd ed., Chapter 16, p. 60 in the case of CAT; the method described in, for example, *Molecular Cloning,* 2nd ed., Chapter 16, p. 66 in the case of β-gal; the method described in, for example, *Experimental Medicine, Supplement*, *Biomanual Series 4*, *Gene Introduction and Expression Analysis Method, 81* (1994) in the case of luc; the method described in, for example, *Proc. Natl*. *Acad*. *Sci*. *USA*, *94:* 4653 (1997) in the case of GFP; and the like.
      The determination result obtained by carrying out the same procedure without adding test samples is compared with the determination result obtained by adding test samples, and a test sample capable of increasing or decreasing amount of the polypeptide encoded by the reporter gene is searched and thereby identified as an expression-controlling compound.
<6> Use of the DNA, polypeptide, antibody, binding inhibitor, phosphorylation inhibitor and expression-controlling compound of the present invention
   (1) The DNA of the present invention can be used for detecting or determining mRNA of the gene of the polypeptide of the present invention in human tissues and human-derived cells by carrying out Northern hybridization of RNA extracted from the tissues and cells in the same manner as described in <1>(1) using the DNA as the probe. Expression distribution of the polypeptide of the present invention in tissues can be known by comparing the expression amounts of mRNA in various tissues.
   (2) The oligonucleotide of the present invention can be used for detecting and determining mRNA encoding the polypeptide of the present invention by carrying out RT-PCR (reverse transcription PCR; *PCR Protocols* (1990)) of RNA extracted from human tissues and human-derived cells in the same manner as described in <1>(1) using the oligonucleotide of the present invention as a specific primer of the DNA of the present invention.
      The method for determining mRNA can be used in the diagnosis of disease states related to the gene.
      Importance of the gene product in disease states can be revealed by determining the mRNA in animal models of various disease states. Also, medicaments can be evaluated by comparing expression amounts of the mRNA in the presence or absence of each medicament.
   (3) More definite expression distribution, such as specification of the expressing cells of the polypeptide of the present invention in tissues, can be known by carrying out in situ hybridization *(Methods in Enzymology, 254:* 419 (1995)) of tissue sections of human using the oligonucleotide of the present invention as the probe.
      Information concerning which tissues and cells express the polypeptide of the present invention and information concerning what kind of stimulation changes the expression level in cells are useful in analyzing participation of the polypeptide of the present invention in physiological functions and disease states.
   (4) Mutation of the gene encoding the polypeptide of the present invention can be detected by carrying out Southern hybridization (*Molecular Cloning,* 2nd ed.) on genomic DNA using the DNA of the present invention as the probe.
      Neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, which have a possibility that mutation of the gene is a cause thereof, can be diagnosed by detecting the mutation.
   (5) Neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, in which the gene encoding the polypeptide of the present invention is involved in the onset of diseases, can be prevented or treated by inhibiting transcription of the gene encoding the polypeptide of the present invention or translation of the mRNA (*Chemistry*, *46: 681* (1991); *Bio/Technology*, *9:* 358 (1992)) using the antisense oligonucleotide (RNA/DNA) of the present invention.
      The above antisense oligonucleotide is administered to the living body by designing and preparing it based on an oligonucleotide having a sequence complementary to continuous 5 to 60 bases in the nucleotide sequence of the DNA of SEQ ID NOS:1 to 8 encoding the polypeptide of the present invention, preferably a nucleotide sequence complementary to 5 to 60 bases existing in the translation initiation region of the DNA encoding the polypeptide of the present invention.
      The medicament containing the DNA of the present invention can be prepared using a method similar to the preparation method of pharmaceutical preparations of the phosphorylation inhibitor or binding inhibitor of the polypeptide of the present invention described in <4> above, and the thus prepared pharmaceutical preparations can be administered in the same manner as in <4> above.
   (6) The polypeptide of the present invention can be obtained by the method described in <2> using the DNA of the present invention.
      An agent for treating or preventing neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases is considered as the use of the polypeptide of the present invention.
      The medicament containing the polypeptide of the present invention can be prepared using a method similar to the preparation method of pharmaceutical preparations of the phosphorylation inhibitor or binding inhibitor of the polypeptide of the present invention described in <4> above, and the thus prepared pharmaceutical preparations can be administered in the same manner as in <4> above.
   (7) The oligonucleotide of the present invention can be used in the gene therapy as a vector for gene therapy by inserting it, as a single-stranded or double-stranded chain, into viral vector, such as retrovirus, adenovirus, adeno-associated virus or the like, or other vector.
   (8) An antibody for the polypeptide of the present invention can be produced by the method described in <3> using the polypeptide of the present invention as the antigen.
      The polypeptide of the present invention can be detected or determined immunologically using the antibody for the polypeptide of the present invention.
      Examples include detection methods, such as ELISA using a microtiter plate, immunocytochemistry by enzyme-labeled antibody method or fluorescent antibody method, Western blotting method and the like.
      Examples include sandwich ELISA using two monoclonal antibodies having different epitopes, among antibodies which react with the polypeptide of the present invention in solution, and a radioimmunoassay using an antibody capable of recognizing the polypeptide of the present invention labeled with a radioisotope, such as ¹²⁵I or the like, and the polypeptide of the present invention.
      The antibody of the present invention can also be applied to immunohistochemistry using pathologic tissue sections.
      The polypeptide of the present invention existing in cells or tissues of a normal person and a patient is immunologically detected or determined using the antibody of the present invention, the amounts between the normal person and patient are compared, and then a difference in expression level is determined to diagnose neurodegenerative diseases (Alzheimer's disease, Parkinson's disease and the like), ischemic diseases, brain damage due to stroke, epilepsy, or various immunological and inflammatory diseases of patients.
   (9) Neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases can be prevented or treated by administering an antibody which inhibits a function of the polypeptide of the present invention (JNK3 scaffold polypeptide or substrate of phosphorylation by JNK3, or binding with JNK3).
      The medicament containing the antibody of the present invention can be prepared using a method similar to the preparation method of pharmaceutical preparations of the phosphorylation inhibitor or binding inhibitor of the polypeptide of the present invention described in <4> above, and the thus prepared pharmaceutical preparations can be administered in the same manner as in <4> above.
   (10) The phosphorylation inhibitor and binding inhibitor of the present invention and the compound which controls expression of the gene of the polypeptide of the present invention can be used for preventing or treating neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases.

### BRIEF DESCRIPTION OF THE DRAWINGS

Fig. 1 is a restriction enzyme map of pcDNA3-S-JSAP1a-Δ1.
Fig. 2 is a restriction enzyme map of pcDNA3-S-JSAP1a-Δ2.
Fig. 3 is a restriction enzyme map of pcDNA3-S-JSAP1a-Δ3.
Fig. 4 is a restriction enzyme map of pcDNA3-S-JSAP1a-Δ4.
Fig. 5 is a restriction enzyme map of pcDNA3-S-JSAP1a-Δ5.
Fig. 6 is a restriction enzyme maps of pcDNA3-S-JSAP1a to pcDNA3-S-JSAP1d.
Fig. 7 is a restriction enzyme map of pcDNA3-S-JSAP3.
Fig. 8 is a restriction enzyme map of pcDNA3-S-JSAP4.
Fig. 9 is a restriction enzyme map of pGAD10-JSAP5.
Fig. 10 is a restriction enzyme map of pcDNA3-His-S-JSAP5.
Fig. 11 is an electrophoresis pattern showing a result of Northern hybridization for mRNA of the mouse liver, spleen, kidney, brain, heart, lung and testis using a partial sequence of each cDNA of mouse JNK3 and mouse JSAP1a as the probe. A result on β-actin is also shown as an expression control.
Fig. 12 is an electrophoresis pattern showing a result of test on the binding ability of JSAP1a to JNK1, JNK2, JNK3, ERK2 and p38 analyzed by Western blotting. Lower column shows a result of Western blotting carried out using a cell extract in order to confirm the expression amount of each of Flag-JNK1, 2, 3, ERK2 and p38 in COS-7 cells.
Fig. 13 is an electrophoresis pattern showing a result of the analysis of JNK3 binding region in JSAP1a.
Fig. 14 is an electrophoresis pattern showing a result of the phosphorylation of JSAP1a by JNK3.
Fig. 15 is an electrophoresis pattern showing a result of the analysis of the phosphorylation of JSAP1a and intracellular localization of JNK3. JNK3 was detected by antibody staining. The cell nucleus was detected by Hoechst staining.
Fig. 16 is an electrophoresis pattern showing a result of Western blotting analysis on the binding ability of JSAP1a to SEK1. Lower two columns show a result of Western blotting carried out using a cell extract in order to confirm the expression level of each Flag-SEK1 and activated SEK1 in COS-7 cells. FL means a full length polypeptide.
Fig. 17 is an electrophoresis pattern showing a result of Western blotting analysis on the binding ability of JSAP1a to MKK7. Lower column shows a result of Western blotting carried out using a cell extract in order to confirm the expression level of each Flag-MKK7 in COS-7 cells. FL means a full length polypeptide.
Fig. 18 is an electrophoresis pattern showing a result of Western blotting analysis on the binding ability of JSAP1a to MEK1, MKK6 and MKK7. Lower column shows a result of Western blotting carried out using a cell extract in order to confirm the expression amount of each of Flag-MEK1, MKK6 and MKK7 in COS-7 cells. FL means a full length polypeptide.
Fig. 19 is an electrophoresis pattern showing a result of Western blotting analysis on the binding ability of JSAP1a to an N-terminal moiety (1st to 640th amino acid residues) of MEKK1. Lower column shows a result of Western blotting carried out using a cell extract in order to confirm the expression amount of the N-terminal moiety of each Flag-MEKK1 in COS-7 cells. FL means a full length polypeptide.
Fig. 20 is an electrophoresis pattern showing a result of Western blotting analysis on the binding ability of JSAP1a to an N-terminal moiety (1st to 327th amino acid residues) or C-terminal moiety (316th to 648th amino acid residues) of Flag-c-Raf1. Lower column shows a result of Western blotting carried out using a cell extract in order to confirm the expression level of each of Flag-Raf-N and Raf-C in COS-7 cells. FL means a full length polypeptide.
Fig. 21 shows a result of examination on the effects of Cdc42 and JSAP1a on the JNK3 activity, carried out using a LUC reporter system in which the JNK3 activity in P19 cells is measured as the GAL4-c-Jun transcription activity. The longitudinal axis shows a relative luciferase activity corresponding to the JNK3 activity.
Fig. 22 shows a result of examination on the effects of ΔRaf1 and JSAP1a on the ERK activity, carried out using a LUC reporter system in which the ERK activity in P19 cells is measured as the GAL4-Elk1 transcription activity. The longitudinal axis shows a relative luciferase activity corresponding to the ERK activity.

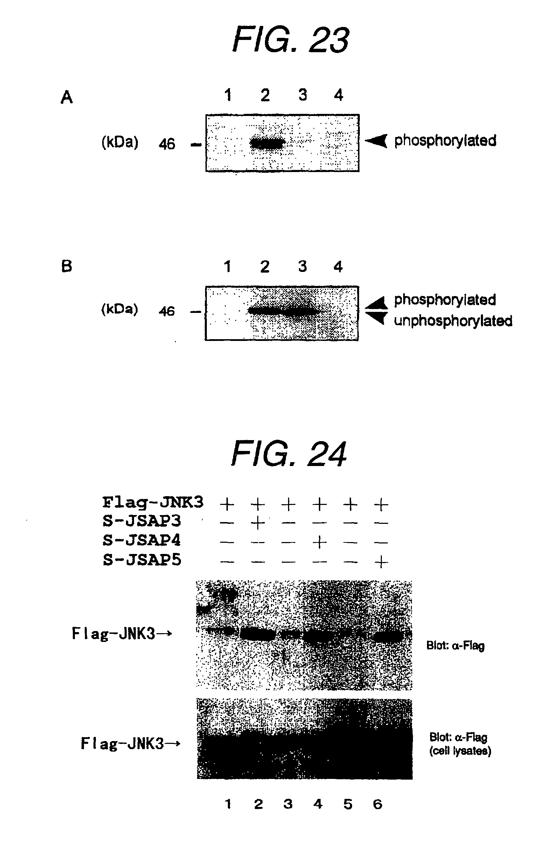

Fig. 23 is an electrophoresis pattern showing a result of Western blotting analysis, carried out using a ΔMEKK1 enzyme solution, a JNK3 enzyme solution and an activated JNK3 enzyme solution obtained by introducing ΔMEKK1 and JNK3 genes in COS-7 cells singly or simultaneously. "A" shows a result of staining of activated JNK3 with an antibody which recognizes phosphorylated JNK3, and "B" shows a result pf staining of JNK3 with anti-Flag antibody. The ΔMEKK1 enzyme solution was applied to lane 1 of the electrophoresis, and the activated JNK3 enzyme solution to lane 2, the JNK3 enzyme solution to lane 3 and a disrupted solution of COS-7 cells without gene introduction to lane 4.
Fig. 24 is an electrophoresis pattern showing a result of Western blotting analysis on the binding ability of JSAP3, 4 and 5 to JNK3. Lower column shows a result of Western blotting carried out using a cell extract in order to confirm the expression amount of each of Flag-JNK3 in COS-7 cells.
Fig. 25 is an electrophoresis pattern showing a result of autoradiography on the binding ability of JSAP3 to ATF2.
Fig. 26 is an electrophoresis pattern showing a result of autoradiography on the expression of ³⁵S-labeled JSAP4 molecules having various length.
Fig. 27 is an electrophoresis pattern showing a result of autoradiography of the JNK3 binding region of JSAP4. This is a result of the analysis of the binding of ³⁵S-labeled JSAP4 molecules having various length with GST or GST-JNK3, and it is observed as a band.
Fig. 28 is an electrophoresis pattern showing a result of Western blotting analysis on the binding ability of JSAP4 to JNK1, JNK2 and ERK2. Among the three columns of blotting, the second and third columns show results of Western blotting carried out using cell extracts in order to confirm the expression amounts of JSAP4 and each MAPK (JNK1, JNK2, JNK3 and ERK2), respectively, in COS-7 cells.
Fig. 29 shows a result of Northern hybridization carried out on mRNA of the mouse testis, large intestine, heart, lung, kidney, brain, spleen and liver using a partial sequence of cDNA of JSAP4 as the probe. A result on β-actin is also shown as an expression control.
Fig. 30 shows a result of examination on the effects of MEKK1, TAK1 and JSAP4 on the JNK activity, carried out using a LUC reporter system in which the JNK activity in COS-7 cells is measured as the GAL4-c-Jun transcription activity. The longitudinal axis shows a relative luciferase activity corresponding to the JNK activity.
Fig. 31 shows a result of examination on the effects of ΔRaf1 and JSAP4 on the ERK activity, carried out using a LUC reporter system in which the ERK activity in COS-7 cells is measured as the GAL4-Elk1 transcription activity. The longitudinal axis shows a relative luciferase activity corresponding to the ERK activity.

### Description of the Symbols:

kb: kilobase pairs
Ap: ampicillin-resistant gene
knt: kilonucleotides

### BEST MODE FOR CARRYING OUT THE INVENTION

The present invention is explained based on the examples, but the scope of the present invention is not limited thereto.

Unless otherwise noted, the known methods described in *Molecular Cloning,* 2nd ed. were used as the gene manipulation techniques in the following examples.

### Example 1 Cloning of cDNA encoding a polypeptide having the activity to bind to JNK3

(1) Cloning from mouse brain-derived cDNA library
   A full length cDNA encoding the mouse JNK3 (*Nature Medicine, 3*: 89 (1997)) was inserted into the *Nco*I-*Bam*HI site of cloning vector pAS2-1 (manufactured by Clontech) containing a sequence encoding GAL4 DNA binding domain (pAS2-1-JNK3), and then introduced into yeast CG-1945 (manufactured by Clontech).
   A mouse brain cDNA library in cloning vector pGAD10 (manufactured by Clontech) containing a sequence encoding the GAL4 transcription activation domain was introduced into the above yeast which had been introduced with pAS2-1-JNK3 to obtain yeast transformants.
   Strains from which the histidine requirement was removed (capable of growing on a histidine-free medium) or strains having β-galactosidase activity were selected from the yeast transformants (primary positive clones).
   Clones having no histidine requirement and having β-galactosidase activity were selected from the resulting primary positive clones (secondary positive clones).
   Plasmid derived from pGAD10 was recovered from the resulting clones.
   As a result, four kinds of partial length cDNA fragments having different sequences were obtained from the mouse brain cDNA library by a two-hybrid system using the above yeast.
   Using these obtained fragments as probes, a λZAPII mouse brain cDNA library (manufactured by Clontech) was screened in a known method, and seven kinds of cDNA clones encoding JSAP1a, JSAP1b, JSAP1c, JSAP1d, JSAP3, JSAP4 and JSAP5 as polypeptides capable of binding to JNK3 (JSAP) were obtained.
   The cDNA encoding JSAP1a, JSAP1b, JSAP1c, JSAP1d, JSAP3 or JSAP4 was obtained as a full length cDNA, but the cDNA encoding JSAP5 was obtained as a partial length CDNA.
   It was found that the cDNA encoding JSAP1a, JSAP1b, JSAP1c, JSAP1d, JSAP3 or JSAP4 contained an open reading frame (hereinafter referred to as "ORF") of 3,918 bp, 3,945 bp, 4,014 bp, 4,011 bp, 1,293 bp or 4,527 bp, respectively, which encoded amino acid residues of 1305 residues, 1314 residues, 1337 residues, 1336 residues or 1508 residues, respectively (SEQ ID NOS:1 to 6).
   Also, JSAP1a, JSAP1b, JSAP1c and JSAP1d were splice variants derived from the same gene. That is, a variant in which a nucleotide sequence of 27 bp had been inserted into JSAP1a was JSAP1b, a variant in which nucleotide sequences of 3 bp and 93 bp were inserted was JSAP1c, and a variant in which a nucleotide sequence of 93 bp was inserted was JSAP1d.
   Specifically, the insertion site of JSAP1b is a 27 bp DNA sequence moiety encoding 9 amino acid residues from the 201st serine residue to the 209th serine residue of SEQ ID NO:10; the insertion sites of JSAP1c are a 3 bp DNA sequence moiety encoding the 201st serine residue and a 93 bp DNA sequence moiety encoding 31 amino acid residues from the 219th valine residue to the 249th glutamine residue of SEQ ID NO:11; and the insertion site of JSAP1d is a 93 bp DNA sequence moiety encoding 31 amino acid residues from the 218th valine residue to the 248th glutamine residue of SEQ ID NO:12.
   JSAP1a coincided with a known sequence (SEQ ID NO:9, 1997 The Molecular Biology Society of Japan (December)).
   It was estimated that JSAP3 is a mouse homologue of human C-terminal-binding protein 1 (CtBP) (*EMBO J*., *17:* 5129 (1998)) (SEQ ID NO:13).
   In JSAP4 having the amino acid sequence represented by SEQ ID NO:14, a sequence called WD40-repeat was found in the 87th to 121st positions, the 341st to 373rd positions, the 658th to 690th positions and the 700th to 732nd positions of the amino acid sequence.
   It is expected that the sequence can be concerned in an interaction (binding) with other protein, and it can be found in a molecule which takes part in the intracellular signal transduction, such as G-protein (*FEBS Lett., 307:* 131 (1994)). Based on Examples described below, it was revealed that the sequence has an important function on signal transduction in the JNK3 pathway.
   The cDNA encoding JSAP5 was a DNA constituted by 734 bp shown in SEQ ID NO:7. It was found that this cDNA does not contain initiation and termination codons and therefore is a partial length. The cDNA encoded 244 amino acid residues (SEQ ID NO:15).
   Full length cDNA of JSAP5 (SEQ ID NO:8) was obtained from the λZAPII mouse brain cDNA library (manufactured by Clontech) by a known method using a probe prepared based on the information on the cDNA nucleotide sequence of JSAP5. It was found that the cDNA contains an ORF of 1,455 bp and encodes 484 amino acid residues (SEQ ID NO:16, hereinafter referred to as "JSAP5F"). In the following experiment, cDNA of JSAP5 as a partial length cDNA of JSAP5F was used.
(2) Preparation of various polypeptides and vectors expressing the polypeptides for analyzing properties of JSAP

In order to analyze functions of the resulting polypeptide JSAP capable of binding to JNK3, the following polypeptides and vectors which express the polypeptides were prepared.
1) Preparation of vectors which express fusion polypeptides with thioredoxin·S-tag (hereinafter referred to as "Trx·S") peptide
   By a known method, cDNA samples of JNK1, JNK2, JNK3 and SEK1 were obtained from a λZAPII mouse brain cDNA library (manufactured by Clontech), and the MEKK1 cDNA from a λZAPII mouse spleen cDNA library (manufactured by Clontech).
   The c-Raf1 cDNA was obtained from a cDNA library provided by Health Science Research Resources Bank, Japan.
   Samples of cDNA of human lymphocyte-derived MKK6, p38, ERK2, c-Jun(1-79) and ATF2 and mouse thymus-derived MEK1, MKK7 and Cdc42 were obtained by using a PCR method.
   Each of the resulting cDNA was inserted into the downstream of the Trx·S sequence of pET32a (manufactured by Novagen) to prepare a respective vector which expresses Trx·S-JNE1, Trx·S-JNK2, Trx·S-JNK3, Trx·S-SEK1, Trx·S-MEKK1, Trx·S-c-Raf1, Trx·S-MKK6, Trx·S-p38, Trx·S-ERK2, Trx·S-c-Jun, Trx·S-ATF2, Trx·S-MEK1, Trx·S-MKK7 or Trx·S-Cdc42.
   A DNA encoding the JSAP obtained in the above step (1) or a fragment of the DNA was inserted into each of the restriction enzyme sites described blow existing in the downstream of the Trx·S sequence of expression vector pET32a (manufactured by Novagen) to prepare a respective vector which expresses Trx·S-JSAP1a, Trx·S-JSAP1b, Trx·S-JSAP1c, Trx·S-JSAP1d, Trx·S-JSAP3, Trx·S-JSAP4 or Trx·S-JSAP5.
   Polypeptide encoded by each DNA inserted and each restriction enzyme site for insertion are shown below.
   JSAP1a (115th to 274th amino acid residues):
      *Nco*I-*Bam*HI site
   JSAP1a (115th to 504th amino acid residues):
      *Eco*RI site
   JSAP1a (268th to 486th amino acid residues):
      *Nco*I site
   JSAP1a (486th to 744th amino acid residues):
      *Nco*I-*Bam*HI site
   JSAP1a (744th to 1194th amino acid residues):
      *BamHI* site
   JSAP1b (115th to 283rd amino acid residues):
      *Nco*I-*Bam*HI site
   JSAP1c (115th to 306th amino acid residues):
      *Nco*I-*Bam*HI site
   JSAP1d (115th to 305th amino acid residues):
      *Nco*I-*Bam*HI site
   JSAP3 (full length):
      *Eco*RI-*Sal*I site
   JSAP4 (1042nd to 1331st amino acid residues):
      *Eco*RI-*Hin*dIII site
   JSAP5 (partial length):
      *Eco*RI site

   Also, Trx·S-ATF2 (1st to 107th amino acid residues) and Trx·S-ATF2 (1st to 116th amino acid residues) were prepared by inserting a DNA encoding 1st to 107th amino acid residues of ATF2 and a DNA encoding 1st to 116th amino acid residues of ATF2 into *Bam*HI-*Xho*I and *Bam*HI-*Xho*I sites, respectively, existing in the downstream of the Trx·S sequence of the expression vector pET32a (manufactured by Novagen).
2) Preparation of vectors which express fusion polypeptides with Flag peptide
   Each full length cDNA encoding JNK1, JNK2, JNK3, ERK2 or p38 was inserted into the *Not*I-*Bam*HI site existing in the downstream of the Flag sequence of mammal expression vector pFlag-CMV-2 (manufactured by Kodak) to prepare a corresponding vector which expresses Flag-JNK1, Flag-JNK2,
   Flag-JNK3, Flag-ERK2 or Flag-p38, respectively.
   Also, a DNA encoding each of the following polypeptides was inserted into each of the following restriction enzyme sites existing in the downstream of the Flag sequence of Flag-modified pcDNA3 vector to prepare a respective vector which expresses Flag-SEK1, Flag-MKK6, Flag-MKK7, Flag-MEK1, Flag-MEKK1, Flag-c-Raf1, Flag-Raf-C, Flag-MEKK-N or Flag-TAK1.
   Polypeptide encoded by each DNA inserted and each restriction enzyme site for insertion are shown below.
   SEK1 (full length):
      *Hin*dIII-*Xba*I site
   MKK6 (full length):
      *Hin*dIII-*Xba*I site
   MKK7 (full length):
      *Hin*dIII-*Xba*I site
   MEK1 (full length):
      *Hin*dIII-*Xba*I site
   MEKK1 (full length):
      *Bam*HI-*Eco*RV site
   c-Raf1 (full length):
      *Eco*RI-*Xho*I site
   Raf-N (1st to 327th amino acid residues):
      *Eco*RI-*Eco*RV site
   Raf-C (316th to 648th amino acid residues):
      *Eco*RV-*Xho*I site
   MEKK-N (1st to 640th amino acid residues):
      *Bam*HI-*Eco*RI site
   TAK1 (full length):
      *Eco*RI-*Xho*I site

   The DNA encoding TAK1 (TGF-β-activated kinase 1) (*Science*, *270*: 2008 (1995)) was obtained by a known method from a mouse cell strain BAF-B03 cDNA library.
3) Preparation of vectors which express fusion polypeptides with GST
   DNA encoding 1st to 79th amino acid residues of c-Jun into the *Bam*HI-*Eco*RI site of GST fusion protein expression vector pGEX-3X (manufactured by Pharmacia) to prepare GST-c-Jun(1-79) expression vector.
   *E*. *coli* was transformed using the expression vector by a known method to express GST-c-Jun(1-79).
   The thus expressed GST-c-Jun(1-79) was purified using Glutathione Sepharose 4B (manufactured by Pharmacia).
   *Nco*I (blunt ended)-*Bam*HI (blunt ended) DNA fragment encoding JNK3 (full length) was inserted into the *Bam*HI (blunt ended) site of GST fusion protein expression vector pGEX-2T (manufactured by Pharmacia) to prepare GST-JNK3 expression vector.
   *E. coli* was transformed using the expression vector by a known method to prepare GST-JNK3. The thus expressed GST-JNK3 was purified using Glutathione Sepharose 4B (manufactured by Pharmacia).
4) Preparation of vectors which express fusion polypeptides with S-tag peptide
   DNA encoding each of the polypeptides described below or a fragment of the DNA was inserted into each of the restriction enzyme sites described blow existing in the downstream of the S-tag sequence of expression vector S-modified pcDNA3 to prepare a respective vector which expresses S-JSAP1a, S-JSAP1aΔ1, S-JSAP1aΔ2, S-JSAP1aΔ3, S-JSAP1aΔ4, S-JSAP1aΔ5, S-JSAP1b, S-JSAP1c, S-JSAP1d, S-JSAP3, S-JSAP4, S-JSAP4 (1-754), S-JSAP4 (755-1508), S-JSAP4 (755-1062), S-JSAP4 (1063-1331) or S-JSAP4(1332-1508).
   Polypeptide encoded by each DNA inserted and each restriction enzyme site for insertion are shown below.
   JSAP1a (full length):
      *Nco*I site
   JSAP1aΔ1 (1st to 1053rd amino acid residues):
      *Not*I-*Xho*I site
   JSAP1aΔ2 (744th to 1305th amino acid residues):
      *Not*I site
   JSAP1aΔ3 (1054th to 1305th amino acid residues):
      *Bam*HI site
   JSAP1aΔ4 (343rd to 1053rd amino acid residues):
      *Hin*dIII-*Xho*I site
   JSAP1aΔ5 (1st to 343rd amino acid residues):
      *Hin*dIII site
   JSAP1b (full length):
      *Not*I site
   JSAP1c (full length):
      *Not*I site
   JSAP1d (full length):
      *Not*I site
   JSAP3 (full length):
      *Eco*RI-*Xho*I/*Sal*I
   JSAP4 (full length):
      *Eco*RI-*Hin*dIII
   JSAP4 (1st to 754th amino acid residues):
      *Eco*RI-*Hin*dIII
   JSAP4 (755th to 1508th amino acid residues):
      *Eco*RI-*Hin*dIII
   JSAP4 (755th to 1062nd amino acid residues):
      *Eco*RI-*Hin*dIII
   JSAP4 (1063rd to 1331st amino acid residues):
      *Eco*RI-*Hin*dIII
   JSAP4 (1332nd to 1508th amino acid residues):
      *Eco*RI-*Hin*dIII

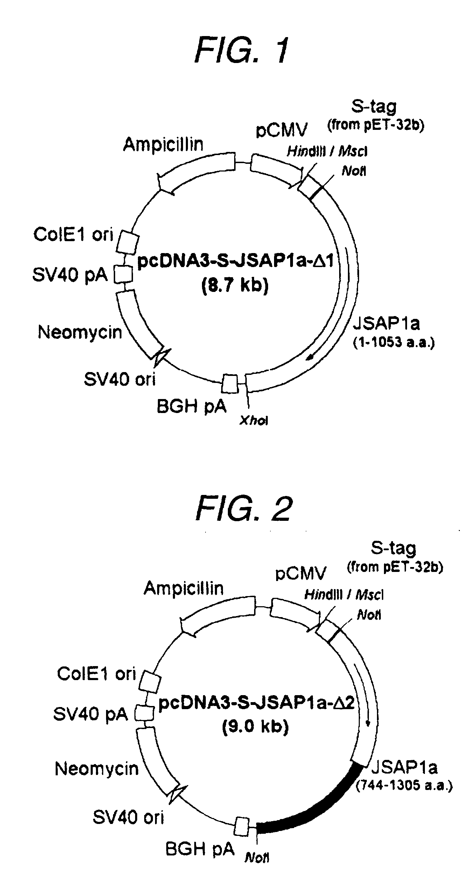

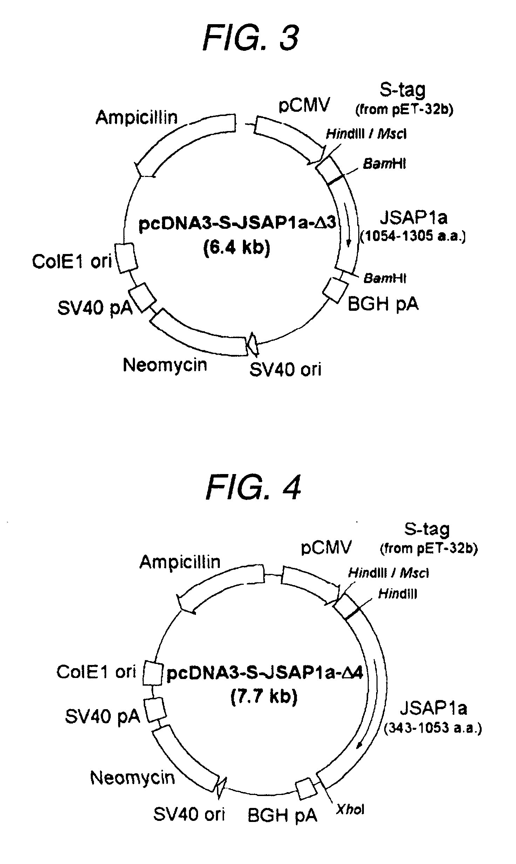

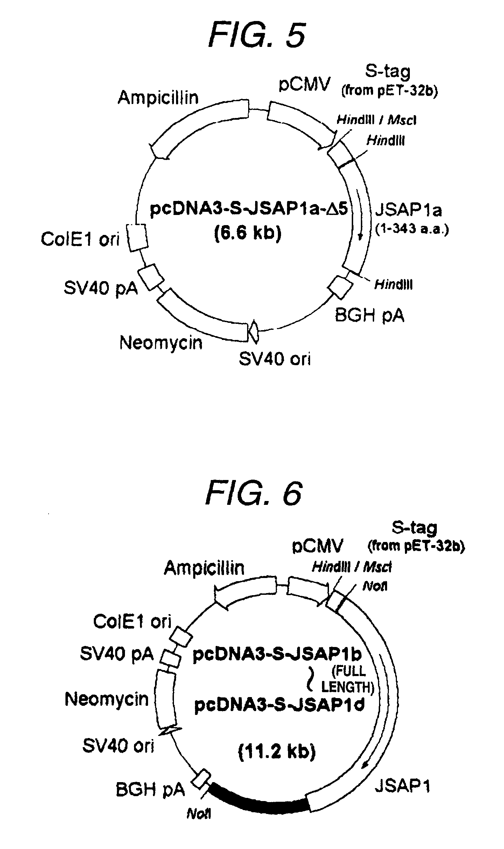

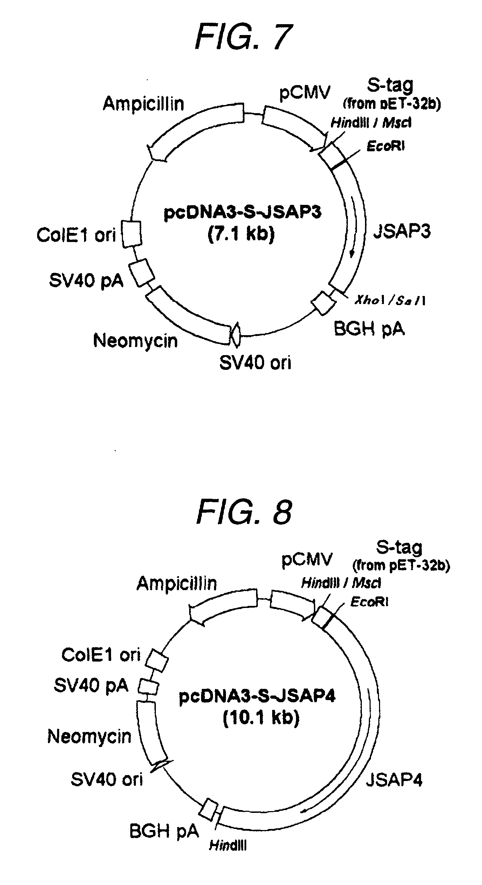

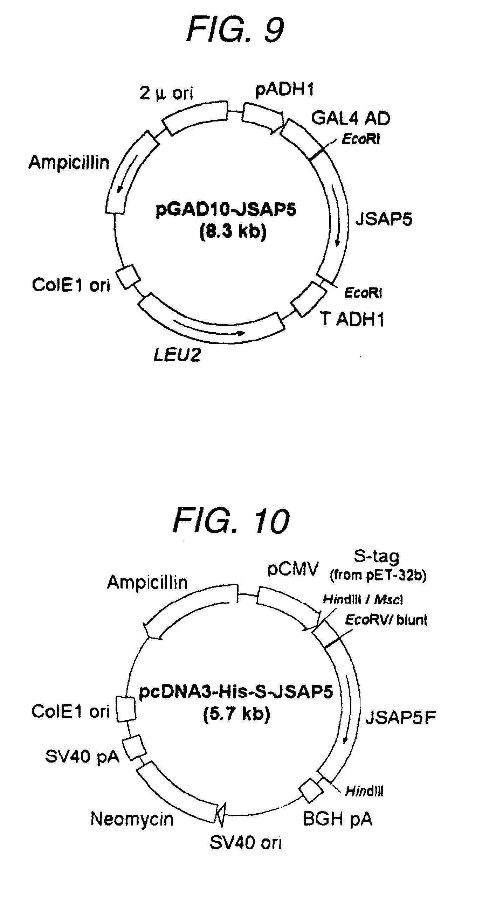

   Also, cDNA (partial length) of JSAP5 was inserted into the *EcoRI* site existing in the downstream of the GAL4AD sequence of expression vector pGAD10 (manufactured by Clontech), and cDNA (full length) of JSAP5F into the *Eco*RV (blunt ended)-*Hin*dIII site existing in downstream of the His-S-tag sequence of expression vector His-S-modified pcDNA3 (Figs. 1 to 10).
5) Preparation of vector which expresses JNK3
   Plasmid pGEM-NCO in which the *Nco*I site had been added to pGEM-3Zf(+) (manufactured by Promega) was prepared by digesting pGEM-3Zf(+) with *Eco*RI and then carrying out self-ligation by adding an oligonucleotide linker having a nucleotide sequence of GCCATGC.
   JNK3 (full length) was inserted into the *Nco*I-*Bam*HI site of the pGEM-NCO to prepare expression vector (pGEM-JNK3).
6) Preparation of vector which expresses constitutively activated Cdc42
   Constitutively activated Cdc42 was prepared by converting the 12th glycine of Cdc42 into valine by point mutation (Cdc42(G12V)). A DNA fragment (*Bam*HI-blunt end) encoding the Cdc42(G12V) (full length) was inserted into the *Bam*HI-*Eco*RV site existing in the downstream of S-tag sequence of the expression vector s-modified pcDNA3.
7) Preparation of vector which expresses constitutively activated MEKK1
   cDNA encoding ΔMEKK1 (1169th to 1488th amino acid residues; constitutively activated as truncated form of MEKK1) was inserted into the *Xba*I site of pEF-BOS vector (*Nucleic Acids Res*., *18*: 5322 (1990)).
8) 5XGAL4-LUC reporter, GAL4-c-Jun and GAL4-E1k1 expression vectors
   All of the 5XGAL4-LUC reporter, GAL4-c-Jun and GAL4-E1k1 expression vectors were purchased from Stratagene and used.
9) RL (*Renilla* luciferase) control vector
   The RL control vector was purchased from Promega and used.
10) Preparation of vectors which express fusion polypeptides with Myc-tag peptide
   cDNA encoding JSAP4 (full length) or JSAP4 (1063rd to 1331st amino acid residues) was inserted into the *EcoRI-Not*I site existing in the downstream of Myc-tag sequence of the expression vector Myc-modified pcDNA3 to prepare a respective vector which expresses Myc-JSAP4 (full length) or Myc-JSAP4 (1063rd to 1331st).
11) Preparation of vectors which express fusion polypeptides with His-S-tag peptide
   DNA encoding each of the polypeptides described below was inserted into each of the restriction enzyme sites described blow existing in the downstream of the His-S-tag sequence of expression vector His-S-modified pcDNA3 which encodes the His-S-tag to prepare a respective vector which expresses MAPK-His-S, MAPKKK-His-S, JNK1-His-S, JNK2-His-S, JNK3-His-S, ERK2-His-S or MEKK1-His-S.
   The insertion restriction enzyme site of each DNA encoding the full length polypeptide is shown below.
   JNK1 *(Not*I (blunt end)-*Bam*HI DNA fragment):
      *Eco*RV-*Bam*HI
   JNK2 (*Not*I (blunt end)-*Bam*HI DNA fragment):
      *Eco*RV-*Bam*HI
   JNK3 (*Not*I (blunt end)-*Bam*HI (blunt end) DNA fragment):
      *Eco*RV
   ERK2 (BamHI DNA fragment):
      *Bam*HI
   MEKK1 (*Hin*dIII DNA fragment):
      *Hin*dIII

### Example 2 JSAP1a, JSAP1b, JSAP1c and JSAP1d

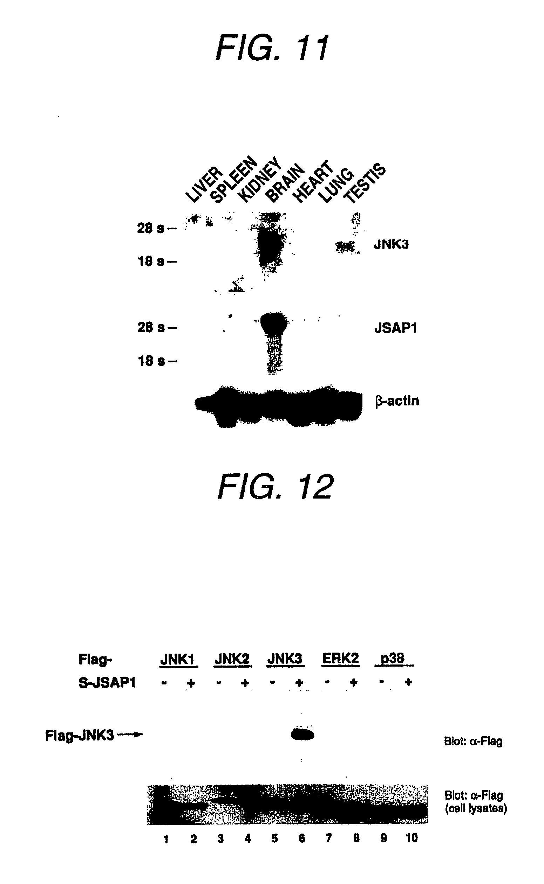

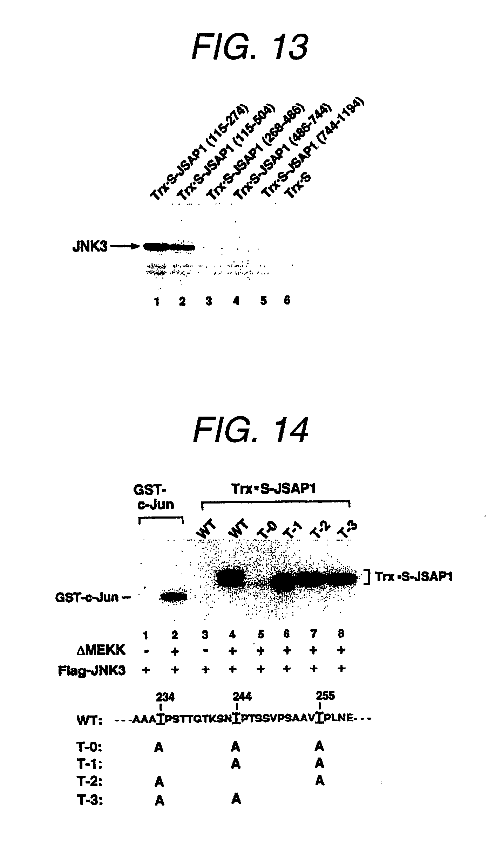

Since the results of analysis of JSAP1a, JSAP1b, JSAP1c and JSAP1d described below were identical to one another, the results of JSAP1a are shown in the drawings as the representative example. Hereinafter, JSAP1a, JSAP1b, JSAP1c and JSAP1d are collectively to as "JSAP1".
1) Analysis of the expression of JNK3 and JSAP1 mRNA by Northern hybridization
   Northern hybridization was carried out according to the method described in *Proc. Natl*. *Acad*. *Sci*. *USA*, *92*: 4972 (1995).
   Specifically, each of the liver, spleen, kidney, brain, heart, lung and testis of mouse was analyzed using ³²P-labeled JNK3, JSAP1 and β-actin as the probe.
   The results are shown in Fig. 11.
   Expression of JNK3 was found specifically in brain. With regard to JSAP1a, JSAP1a mRNA having a size of about 6 kb was found in specifically brain.
2) Analysis of binding specificity and binding region of JSAP1 for various MAPK's
   Each of the S-JSAP1a, S-JSAP1b, S-JSAP1c or S-JSAP1d (full length) expression vector or the Flag-JNK1, Flag-JNK2, Flag-JNK3, Flag-ERK2 or Flag-p38 expression vector, prepared in Example 1(2), was transfected to COS-7 cells using TransIT-LT1 (manufactured by Mirus), and then the COS-7 cells were cultured to transiently express the polypeptide derived from the respective introduced vectors.
   After culturing for 34 hours, the cells were dissolved in buffer B, S Protein Agarose(manufactured by Novagen) was added thereto, and then S-JSAP1 and a polypeptide capable of binding to S-JSAP1 were precipitated and recovered.
   The resulting recovered fraction was separated by SDS-PAGE and transferred on membrane Immobilon-P (manufactured by Millipore).
   Western blotting was carried out using the membrane and anti-Flag M5 monoclonal antibody (manufactured by Kodak) as the probe, and the polypeptide capable of binding to the antibody (Flag-JNK3) was visualized using an ECL detection system (manufactured by Amersham) to examine MAPK capable of binding to JSAP1.
   The results are shown in Fig. 12. It was found that JSAP1 binds only to JNK3 and does not bind to other MAPK.
   The binding region of JSAP1 to JNK3 was analyzed by the following method.
   A fusion polypeptide of Trx·S with a partial fragment of JSAP1, each Trx·S-JSAP1 (fragment) was expressed in *E. coli* using the expression vector prepared in Example 1(2) by a known method and then recovered by allowing it to bind to S Protein Agarose.
   ³⁵S-labeled form of full length JNK3 was prepared by *in vitro* translation using the expression vector pGEM-JNK3 prepared in Example 1(2) by TNT T7 Quick Coupled Transcription/Translation System (manufactured by Promega).
   The resulting ³⁵S-labeled JNK3 and Trx·S-JAP1 (fragment) were mixed in buffer A (50 mM Tris-HCl (pH 7.5), 150 mM NaCl, 0.5% NP-40), and the reaction was carried out at 4°C for 2 hours while stirring the reaction solution by rotating its container tube.
   After the reaction, the reactant was washed three times with buffer A, and the resulting precipitate was separated by SDS-PAGE and analyzed by autoradiography.
   As a result, it was found that the binding region of JSAP1 to JNK3 is present in a region of the 115th to 274th (JSAP1a), 115th to 283rd (JSAP1b), 115th to 306th (JSAF1c) or 115th to 305th (JSAP1d) amino acid residues.
   The results in the case of JSAP1a are shown in Fig. 13. In the case of JSAP1a, the binding region was the 115th to 274th amino acid residues.
3) Phosphorylation of JSAP1 by JNK3 and loss of binding ability of JNK3 by phosphorylation
   In the binding region of JSAP1 with JNK3, threonine residues having a possibility of being phosphorylated by proline-directed serine/threonine kinase are present as shown below.
   JSAP1a: 234th, 244th and 255th amino acid residues
   JSAP1b: 243rd, 253rd and 264th amino acid residues
   JSAP1c: 266th, 276th and 287th amino acid residues
   JSAP1d: 265th, 275th and 286th amino acid residues

   Fusion polypeptides of each JSAP fragment containing the above positions expected to be phosphorylated with Trx·S, Trx·S-JSAP1a (115th to 274th amino acid residues), Trx·S-JSAP1b (115th to 283rd amino acid residues), Trx·S-JSAP1c (115th to 306th amino acid residues) and Trx·S-JSAP1d (115th to 305th amino acid residues) were prepared according to Example 1(2).
   COS-7 cells were transfected using TransIT-LT1 (manufactured by Mirus) with the Flag-JNK3 expression vector and ΔMEKK1 expression vector, or the Flag-JNK3 expression vector alone, prepared in Example 1(2), and then the COS-7 cells were cultured to transiently express the polypeptide derived from each of the introduced vectors.
   After culturing for 34 hours, the cells were dissolved in buffer B and then Flag-JNK3 was immunoprecipitated using anti-Flag M5 monoclonal antibody (manufactured by Kodak) immobilized on protein-G-agarose.
   Using the resulting JNK3 or activated JNK3 and the Trx·S-JSAP1a (115th to 274th amino acid residues), Trx·S-JSAP1b (115th to 283rd amino acid residues), Trx·S-JSAP1c (115th to 306th amino acid residues) or Trx·S-JSAP1d (115th to 305th amino acid residues) prepared above and bound to S protein agarose, phosphorylation was carried out according to the method described in *Cell*, *76*: 1025 (1994) by adding ³²P-labeled ATP ([γ-³²P]ATP). As a positive control of phosphorylation, GST-c-Jun (1st to 79th amino acid residues) was used as the substrate (*EMBO J., 15:* 2760 (1996)).
   The reactant was separated by SDS-PAGE and then analyzed by autoradiography.
   The results are shown in Fig. 14. The JSAP1 was efficiently phosphorylated (lane 4 in Fig. 14). Phosphorylation of c-Jun as the positive control was also confirmed (lane 2 of Fig. 14).
   Each of the above threonine residues having a possibility of being phosphorylated in JSAP1 was converted into an alanine residue by site-specific mutagenesis using overlapping PCR *(Current Protocols in Molecular Biology* (John Wiley & Sons Inc., New York, 1989)), and the converted polypeptide was expressed and recovered according to the method of Example 1(2).
   The resulting polypeptides WT to T-3 are shown below.
   (i) Trx·S-JSAP1a (115th to 274th amino acid residue region)
      - WT:: No substitution by an alanine residue
      - T-0:: Substitution with an alanine residue at the 234th, 244th and 255th positions
      - T-1:: Substitution with an alanine residue at the 244th and 255th positions
      - T-2:: Substitution with an alanine residue at the 234th and 255th positions
      - T-3:: Substitution with an alanine residue at the 234 and 244th positions
   (ii) Trx·S-JSAP1b (115th to 283rd amino acid residue region)
      - WT:: No substitution by an alanine residue
      - T-0:: Substitution with an alanine residue at the 243rd, 253rd and 264th positions
      - T-1:: Substitution with an alanine residue at the 253rd and 264th positions
      - T-2:: Substitution with an alanine residue at the 243rd and 264th positions
      - T-3:: Substitution with an alanine residue at the 243rd and 253rd positions
   (iii) Trx·S-JSAP1c (115th to 306th amino acid residue region)
      - WT:: No substitution by an alanine residue
      - T-0:: Substitution with an alanine residue at the 266th, 276th and 287th positions
      - T-1:: Substitution with an alanine residue at the 276th and 287th positions
      - T-2:: Substitution with an alanine residue at the 266th and 287th positions
      - T-3:: Substitution with an alanine residue at the 266th and 276th positions
   (iv) Trx·S-JSAP1d (115th to 305th amino acid residue region)
      - WT:: No substitution by alanine residue
      - T-0:: Substitution with an alanine residue at the 265th, 275th and 286th positions
      - T-1:: Substitution with an alanine residue at the 275 and 286th positions
      - T-2:: Substitution with an alanine residue at the 265 and 286th positions
      - T-3:: Substitution with an alanine residue at the 265 and 275th positions

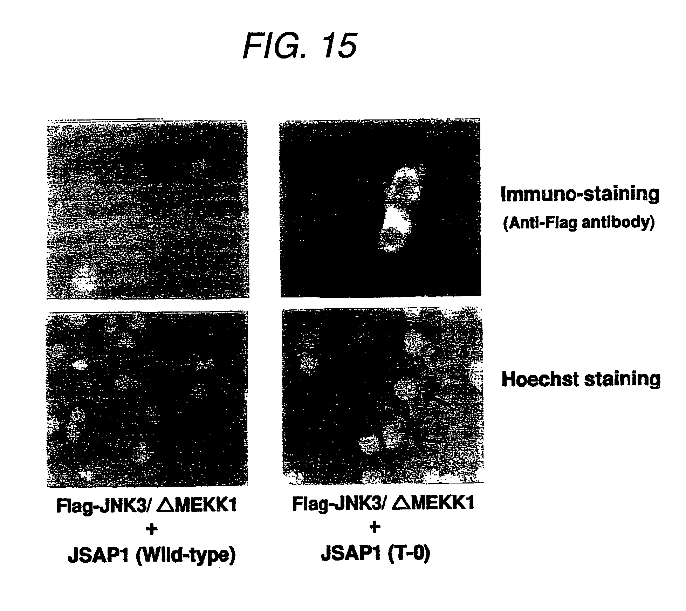

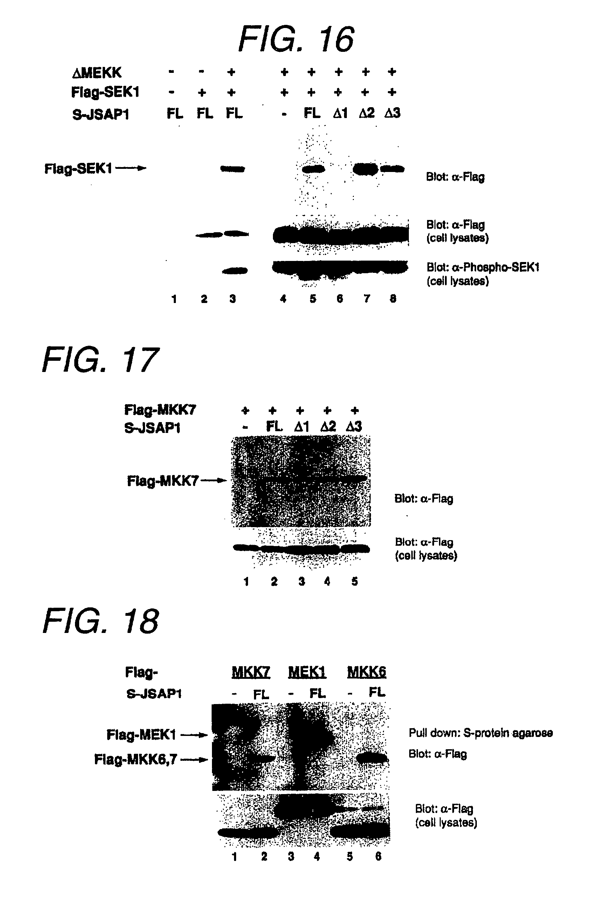

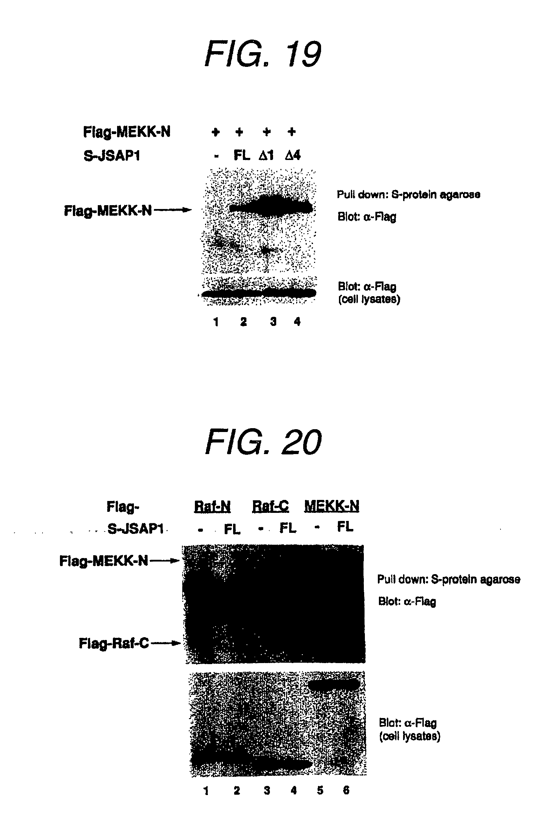

   Phosphorylation was carried out using the resulting polypeptides.
   The results in the case of Trx·S-JSAP1a are shown in Fig. 14.
   In all of Trx·S-JSAP1a to d, phosphorylation was not observed only in T-0 in which all of the threonine residues expected to undergo phosphorylation were substituted with alanine (lane 5 in Fig. 14), and WT and all of other alanine substitution products (T-1 to 3) were phosphorylated (lanes 6 to 8 in Fig. 14).
   According to the method of Example 2(1), the T-0 which were not phosphorylated in the above, Flag-JNK3 and ΔMEKK1 were expressed in P19 cells which were simultaneously differentiated by retinoic acid, and the cells were stained with anti-Flag M5 monoclonal antibody (manufactured by Kodak).
   The same test was carried out using WT which was phosphorylated in the above, Flag-JNK3 and ΔMEKK1, and the cells were stained.
   The results in the case of JSAP1a are shown in Fig. 15.
   In each case of JSAP1, JNK3 was present only in the cytoplasm and unable to transfer into the nucleus when T-0 was used, but JNK3 was transferred into the nucleus when WT was used.
   The above results show that, when JSAP1 is phosphorylated by JNK3, the JNK3 bound to JSAP1 is dissociated from JSAP1 and transferred into the nucleus.
4) Binding of various MAPKK and MAPKKK with JSAP1
   COS-7 cells were transfected using TransIT-LT1 (manufactured by Mirus) with three kinds of vectors, the S-JSAP1 (full length) expression vector, the expression vector of Flag-SEK1 (Flag-fusion polypeptide of SEK1 (MKK4) as MAPKK), and the ΔMEKK1 expression vector, or two kinds of vectors, the S-JSAP1 (full length) expression vector and the Flag-SEK1 expression vector, prepared in Example 1(2), and then the COS-7 cells were cultured to transiently express the polypeptide derived from each of the introduced vectors.
   After culturing for 34 hours, the cells were dissolved in buffer B and then S-JSAP1 and a polypeptide which binds to S-JSAP1 was immunoprecipitated using S-protein agarose (manufactured by Novagen).
   The resulting fraction was separated by SDS-PAGE and transferred on membrane Immobilon-P (manufactured by Millipore), and then Flag-SEK1 was visualized by an ECL detection system (manufactured by Amersham) using anti-Flag M5 monoclonal antibody (manufactured by Kodak) as the probe.
   The results are shown in Fig. 16. The binding with JSAP1 was observed when SEB1 was activated by ΔMEKK1 (lanes 2 and 3 in Fig. 16). The activation of SEK1 by ΔMEKK1 was confirmed by Western blotting using a monoclonal antibody which recognizes SEK1 activated by phosphorylation (manufactured by NEB).
   Based on the results obtained in the above, the SEK1 binding region in JSAP1 was analyzed in the same manner as the above. That is, various deletion mutants of S-JSAP1 were produced, and binding of each of them to Flag-SEK1 was examined.
   The results obtained using deletion mutants derived from JSAP1a are shown in lanes 4 to 8 of Fig. 16. SEK1 was able to bind to the full length FL (1st to 1305th residues), a deletion mutant Δ2 (having 744th to 1305th residues) and a deletion mutant Δ3 (having 1054th to 1305th residues) of JSAP1a (lanes 5, 7 and 8 in Fig. 16), but unable to bind to a deletion mutant Δ1 (having 1st to 1053rd residues) (lane 6 in Fig. 16).
   Based on the above, it was found that SEK1 binds to the 1054th to 1305th amino acid residues C-terminal portion of JSAP1a.
   Binding of other members of MAPKK to JSAP1a was also analyzed in the same manner as the above.
   The results are shown in Figs. 17 and 18.
   Similar to the case of SEK1, MKK7 (other MAPKK of the JNK pathway) bound to the 1054th to 1305th residues at the C-terminal portion of JSAP1a (Fig. 17). MEK1 and MKK6 as MAPKK of the ERK or p38 pathway, respectively, also bound to JSAP1a (Fig. 18).
   COS-7 cells were transfected using TransIT-LT1 (manufactured by Mirus) with the expression vector of S-JSAP1a (full length) FL, a deletion mutant Δ1 (having 1st to 1053rd amino acid residues) or a deletion mutant Δ4 (having 343rd to 1053rd amino acid residues), and the expression vector of Flag-MEKK-N as a fusion polypeptide of an N-terminal sequence polypeptide of an MEKK1, which is MAPKKK (MEKK1-N: 1st to 640th amino acid residues) with Flag-tag, obtained in Example 1(2), and then the COS-7 cells were cultured for transient expression of the polypeptide derived from each of the introduced vectors.
   Since the expression of full length MEKK1 in COS-7 cells was considerably low, MEKK1-N as a partial sequence of MEKK1 was used in this experiment.
   The results are shown in Fig. 19. The MEKK1-N bound to each of FL, Δ1 and Δ4 of JSAP1a. Since it bound to Δ4, it was considered that MEKK1-N binds to the region of 343rd to 1053rd amino acid residues.
   The similar experiment was carried out using a polypeptide having a region other than the N-terminal moiety of MEKK1, and it was confirmed that the polypeptide does not bind to JSAP1a at a region other than the above N-terminal moiety.
   Since MEKK1 binds to JSAP1a Δ1 (1st to 1053rd amino acid residues) having higher affinity than that of the full length JSAP1a (lane 3 in Fig. 19), there is a possibility that the region of 1054th to 1305th residues at the C-terminal of JSAP1a considered in the above to be the binding region of SEK1 has a function of inhibiting the binding of MEKK1.
   In addition, among the MAPK pathway, c-Raf1 as a member of MAPKKK involved in the ERK pathway was examined for its binding to JSAP1.
   COS-7 cells were transfected with vector expressing Flag-Raf-N or Flag-Raf-C obtained in Example 1(2), wherein an N-terminal portion region (1st to 327th amino acid residue) or a C-terminal portion region (316th to 648th amino acid residue) of the c-Raf1 is fused with the Flag peptide, and the expression vector of each S-JSAP1 obtained in Example 1(2) using TransIT-LT1 (manufactured by Mirus), and then the COS-7 cells were cultured to transiently express the polypeptide derived from each of the introduced vectors.
   In this case, since the expression of full length c-Raf1 in COS-7 cells was considerably low, Raf-N and Raf-C as partial sequences of c-Raf1 were used in this experiment.
   The results are shown in Fig. 20. The Raf-C bound to JSAP1 (lane 4 in Fig. 20), but Raf-N did not bind (lane 2 in Fig. 20). Also, the binding affinity of Raf-C was lower than the affinity of MEKK1 (lanes 4 and 6 in Fig. 20).
   It was found from the above that JSAP1-binding regions of MAPKKK (MEKK1), MAPK (SEK1 and MKK7) and MAPK (JNK3) related to the JNK3 pathway are different from each other.
   In the case of JSAP1a, the binding region for MEKK1 was the 343rd to 1053rd amino acid residue region, the binding region for SEK1 and MKK7 was the 1054th to 1305th residue region, and the binding region for JNK3 was a 115th to 274th residue region.
   It was considered that JSAP1 has a leucine-zipper structure. Amino acid sequence numbers of leucine residues constituting the leucine-zipper structure in each JSAP1 are shown below.
   JSAP1a: 392, 399, 406, 413, 420, 427
   JSAP1b: 401, 408, 415, 422, 429, 436
   JSAP1c: 424, 431, 438, 445, 452, 459
   JSAP1d: 423, 430, 437, 444, 451, 458

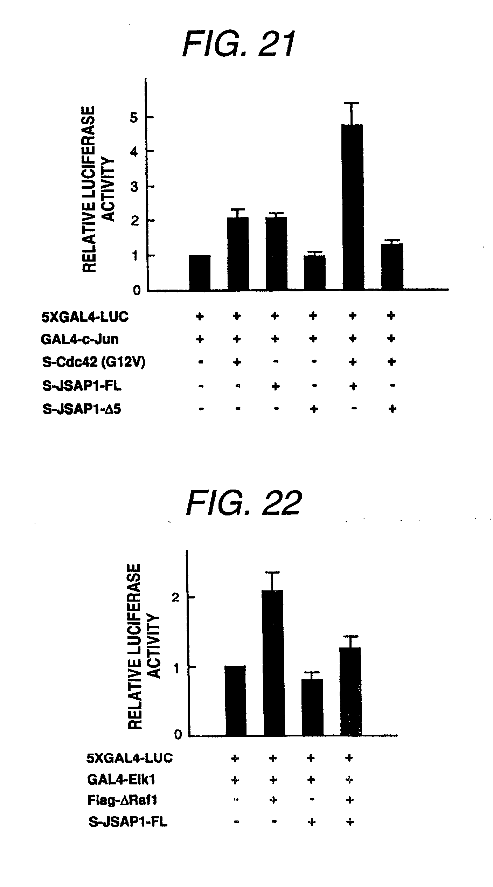

   Based on the above, it was considered that each
   JSAP1 is functioning by existing as a homo or hetero dimer.
5) Analysis of function of JSAP1 as scaffold protein in JNK3 pathway using reporter system
   The activation of the JNK3 pathway by over-expression of full length JSAP1 was analyzed.
   5XGAL4-LUC reporter expression vector (manufactured by Stratagene), GAL4-c-Jun expression vector (1st to 223rd residues containing a c-Jun activation domain, manufactured by Stratagene) and RL control vector (manufactured by Promega) were introduced into P19 cells differentiated by retinoic acid, originally expressing JSAP1a and JNK3.
   Into the P19 cells, full length S-JSAP1a-FL expression vector or S-JSAP1a-Δ5 (1st to 343rd residues) expression vector and/or constitutively activated S-Cdc42 (G12V) expression vector were introduced.
   The P19 cells were cultured to transiently express the polypeptide derived from each of the introduced vectors.
   After culturing for 24 hours, the luciferase activity was measured, and the GAL4-c-Jun transcription activity, namely JNK3 activity, was calculated. The relative value of the luciferase activity was obtained by correcting it with the activity value of RL luciferase.
   The results are shown in Fig. 21.
   Cdc42 (G12V) increased the JNK3 activity, and over-expression of JSAP1a increased the JNK3 activity to a similar level of that of Cdc42 (G12V).
   When cells in which Cdc42 (G12V) and JSAP1a were simultaneously expressed, the JNK activity was increased additively to the case of single use.
   On the other hand, the JNK3 activity was inhibited when JSAP1a-Δ5 (1st to 343rd amino acid residues) and Cdc42 (G12V) were simultaneously expressed.
   The influence of over-expression of full length JSAP1 upon the ERK pathway was examined in the same manner as the above.
   5XGAL4-LUC reporter expression vector, GAL4-Elk1 (307th to 427th residues containing an Elk1 activation domain) expression vector and RL control vector were introduced into P19 cells differentiated by retinoic acid.
   Into the P19 cells were introduced S-JSAP1a-FL (full length JSAP1a) expression vector and/or the expression vector of constitutively activated ΔRaf1 *(Mol. Cell*. *Biol*., *9*: 639 (1989)) polypeptide with Flag (Flag-△Raf1).
   The P19 cells were cultured for transient expression of the polypeptide derived from each of the introduced vectors.
   The luciferase activity was measured after culturing for 24 hours, and the GAL4-Elk1 transcription activity, namely the ERK activity, was calculated. The relative value of the luciferase activity was obtained by correcting it with the activity value of RL luciferase.
   The results are shown in Fig. 22.
   It was revealed that over-expression of JSAP1a inhibits the ERK activity.
   The JSAP1a bound to all of the MAKPKKK, MAPKK and JNK3 existing in the JNK3 pathway, and it was concluded from the above results that it functions as an important scaffold polypeptide which effectively and specifically activate the JNK3 pathway.
6) Construction of system for screening inhibitor which inhibits phosphorylation of JSAP1 by JNK3
   A fusion protein of a partial protein corresponding to the 114th to 274th positions of the amino acid sequence of JSAP1a (containing the JNK3 binding region and three Thr residues to be phosphorylated) with GST was prepared as shown below, to be used as the phosphorylation substrate of JNK3.

A forward primer having the nucleotide sequence represented by SEQ ID NO:17 and a reverse primer having the nucleotide sequence represented by SEQ ID NO:18 were synthesized as the JSAP1a-specific PCR primers.

The forward primer has a nucleotide sequence in which an *Eco*RV recognition sequence is introduced into just before a nucleotide sequence corresponding to a region of the 446th to 464th positions in the nucleotide sequence of cDNA encoding the JSAP1a having the nucleotide sequence represented by SEQ ID NO:1.

The reverse primer has a nucleotide sequence in which a stop codon is introduced into just after a nucleotide sequence corresponding to a complementary sequence of a region of the 909th to 928th positions in the nucleotide sequence of cDNA encoding the JSAP1a having the nucleotide sequence represented by SEQ ID NO:1, and also an *EcoRI* recognition sequence is introduced further just thereafter.

PCR by KOD DNA polymerase (manufactured by Toyobo) was carried out using these two kinds of primers and, as the template, the expression plasmid pET32a containing full length JSAP1a prepared in Example 1(2) by Thermal cycler 450 (manufactured by Takara).

The thus amplified JSAP1a cDNA fragment was separated by agarose gel electrophoresis, and the fragment was extracted using Qiaex II DNA Extraction Kit (manufactured by Quiagen).

In the same manner, a DNA fragment of pBluescript II KS⁻ digested with restriction enzymes *Eco*RI and *Eco*RV was extracted.

The resulting linear chain DNA fragment of pBluescript II KS⁻ and JSAP1a DNA fragment were connected by carrying out the ligation reaction at 16°C using DNA Ligation Kit (manufactured by Takara) to obtain plasmid pRH1001 in which the JSAP1a DNA fragment is inserted into pBluescript II KS⁻.

Competent cells of *E. coli* were transformed with the pRH1001, and then the *E*. *coli* was cultured overnight on LB plate medium (1% tryptone, 0.5% yeast extract, 1% NaCl) to which ampicillin had been added.

*E*. *coli* obtained from the thus formed colony was cultured in 2 ml of TB medium (a medium prepared by adding 900 ml of water to 12 g of tryptone, 24 g of yeast extract and 4 ml of glycerol, sterilizing the mixture using an autoclave, cooling it to 60°C and then adding thereto 100 ml of a sterilized potassium phosphate solution (0.17 M KH₂PO₄ and 0.72 M K₂HPO₄)) to which ampicillin had been added, and pRH1001 was extracted from the E. *coli*.

By digesting the pRH1001 with *Eco*RI and *Eco*RV, and determining nucleotide sequence of the resulting DNA fragment, it was confirmed that it coincides with the nucleotide sequence encoding JSAP1a.

A DNA fragment of pRH1001 digested with *Eco*RI and *Eco*RV was separated by an agarose gel electrophoresis, and the fragment was extracted using Qiaex II DNA Extraction Kit (manufactured by Quiagen).

In the same manner, a DNA fragment of pGEX-3X (manufactured by Pharmacia) digested with restriction enzymes *Eco*RI and *Sma*I was extracted.

The resulting linear chain DNA fragment of pGEX-3X and pRH1001 DNA fragment were connected by carrying out the ligation reaction at 16°C using DNA Ligation Kit (manufactured by Takara) to obtain plasmid pRH1003 in which the JSAP1a DNA fragment is inserted into pBluescript II KS⁻.

Competent cells of *E. coli* were transformed with the pRH1003, and then the *E. coli* was cultured overnight on LB plate medium to which ampicillin had been added.

*E*. *coli* obtained from the thus formed colony was cultured in 2 ml of TB medium to which ampicillin had been added, and pRH1003 was extracted from the *E*. *coli.*

The pRH1003 is *E. coli* expression vector having a DNA fragment encoding a GST-JSAP1 fusion protein.

The above *E. coil* having pRH1003 was pre-cultured at 37°C overnight in 20 ml of TB medium to which ampicillin had been added.

The resulting culture was added to 440 ml of TB medium to which ampicillin had been added, and cultured at 25°C for 5 hours, and then isopropyl-β-D-thiogalactopyranoside (IPTG) was added to the culture, followed by culturing at 25°C for 15 hours.

Bacterial cells were recovered from the resulting culture by centrifugation. To the cells, 20 ml of PBS containing 0.1 mg/ml of lysozyme was added and incubated at 4°C for 1 hour.

After incubation, 200 µl of 10% N-lauroylsarcosine was added thereto and the bacterial cells were disrupted using a sonicator.

The thus disrupted bacterial cells were centrifuged at 100,000 × g and 4°C for 1 hour to obtain a cytoplasm fraction.

The GST-JSAP1 fusion protein was purified from the cytoplasm fraction by the following method using Glutathione Sepharose 4B (manufactured by Pharmacia)

That is, 5 ml of gel of the Glutathione Sepharose 4B was packed in a column and equilibrated with PBS containing 0.1% N-lauroylsarcosine. The equilibrated Glutathione Sepharose 4B was allowed to adsorb GST-JSAP1 fusion protein and washed with 30 ml of PBS containing N-lauroylsarcosine, and then the GST-JSAP1 fusion protein was eluted with 20 ml of an elution buffer (PBS containing 5 mM reduced form glutathione and 0.1% N-lauroylsarcosine). The eluate was dialyzed against PBS to obtain a purified GST-JSAP1 fusion protein solution.

Since the purified GST-JSAP1 fusion protein solution can be preserved at -80°C, it was used by thawing it prior to use.

ΔMEKK1, JNK3 and activated JNK3 were prepared and obtained as shown below.

COS-7 cells were inoculated into a 60 mm plate at a 2×10⁵ to 5×10⁵ cells/ml DMEM, followed by culturing overnight at 37°C.

To 300 µl of OPTI-MEM (Gibco BRL, manufactured by Life Technologies), 9 µl of FuGene 6 transfection reagent (manufactured by Hoffmann-La Roche) was added, and to the resulting solution, 0.1 to 0.5 µg of the expression vector inserted with cDNA encoding ΔMEKK1 and/or 4.5 to 4.9 µg of the expression vector inserted with cDNA encoding JNK3, prepared in Example 1(2), were added and incubated at room temperature for 15 minutes.

The resulting mixture solution was added slowly by three drop portions to the above COS-7 cells and mixed, and then the cells were cultured at 37°C for 30 to 40 hours.

The cells were recovered using a scraper and washed with 10 ml of PBS.

To the resulting COS-7 cells, 1 ml of a cell lysis buffer (50 mM HEPES/NaOH (pH 7.6), 150 mM NaCl, 0.3% (V/V) Nonidet P-40, 20 mM MgCl₂, 1 mM ethyleneglycol bis(β-aminoethyl)-N,N,N',N'-tetraacetic acid (EGTA), 20 mM β-glycerophosphate, 10 mM Na₃VO₄, 10 mM NaF, 40 µg/ml phenylmethylsulfonyl fluoride (PMSF), 1 µg/ml pepstatin A, 1 µg/ml leupeptin, 1 µg/ml chymostatin, 2 mM dithiothreitol (DTT)) was added to disrupt the cells.

The resulting cell lysate was centrifuged at 20,000 × g and 4°C to obtain a supernatant. The supernatant of cell lysate derived from cells introduced with an expression vector into which cDNA encoding ΔMEKK1 had been inserted was used as a ΔMEKK1 enzyme solution, the supernatant of cell lysate derived from an expression vector into which cDNA encoding JNK3 had been introduced was used as a JNK3 enzyme solution and the supernatant of cell lysate derived from cells introduced with two kinds of expression vectors into which cDNA encoding ΔMEKK1 and JNK3 had been inserted was used as an activated JNK3 enzyme solution.

A system for screening an inhibitor which inhibits phosphorylation of JSAP1 by activated JNK3 is described below.

After the reaction in homogenous solution using activated JNK3, [γ-³³P]-ATP (74 TBq/mmol, manufactured by New England Nuclear) and GST-JSAP1, the phosphorylation of JSAP1 by activated JNK3 was measured using, as an index, the radioactivity of ³³P incorporated into GST-JSAP1.

A reaction solution for screening (3 µM [γ-³³P]-ATP (28 kBq/ml), 2.3 µM GST-JSAP1, 20 mM MgCl₂, 20 mM β-glycerophosphate, 20 mM p-nitrophenyl phosphate, 1 mM Na₃VO₄, 1 mM NaF, 2 mM DTT, 1% DMSO or a test compound containing 1% DMSO, 2% (V/V) of any one of the three kinds of enzyme solutions prepared in the above (the activated JNK3 enzyme solution is used in the actual screening), 50 mM HEPES/NaOH (pH 7.6)) was dispensed in 100 µl portions into each well of a 96 well plate (manufactured by Corning) incubated at 30°C for 10 to 30 minutes.

After incubation, 100 µl of ethanol was added to the reaction solution to terminate the reaction and to precipitate GST-JSAP1.

The precipitate was filtered and adsorbed on UniFilter Plate GF/B (manufactured by Packard) using FilterMate Harvester (manufactured by Packard).

The plate was washed at least three times with PBS and dried, and then 40 µl of a scintillator Microscint-20 (manufactured by Packard) was added thereto to measure the radioactivity using a detector TopCount-HTS (manufactured by Packard).

The results are shown in Table 1.

**Table 1**

| Phosphorylation of GST-JSAP1 by COS-7 lysate | |
|---|---|
| Enzyme solution *¹ | Phosphorylation activity (pmol/mg/min) |
| - | 23 |
| ΔMEKK1 | 26 |
| JNK3 | 37 |
| Activated JNK3 | 978 |

| | |
|---|---|
| *¹: Enzyme solution used at the time of preparation of the reaction solution for screening | |

In the above system, GST-JSAP1 was specifically phosphorylated only when the activated JNK3 enzyme solution was used in the reaction solution for screening.

Accordingly, the above system in which the activated JNK3 enzyme solution was used in the reaction solution for screening has a possibility of being used as a system which can efficiently screen an inhibitor that inhibits phosphorylation of JSAP1 by JNK3. The true phosphorylation activity can be calculated by subtracting the phosphorylation activity in a system which does not use the activated JNK3 enzyme solution.

The fact that the above activated JNK3 enzyme solution is a solution containing the activated JNK3 was confirmed by the following experiment.

Using 10 µg (as the amount of enzyme) of each of the ΔMEKK1 enzyme solution, JNK3 enzyme solution and activated JNK3 enzyme solution prepared above, SDS-PAGE was carried out and each of the electrophoresed proteins was transferred on membrane Hybond-ECL (manufactured by Amersham-Pharmacia Biotech).

Western blotting was carried out using the membrane, and anti-active JNK polyclonal antibody (manufactured by Promega) and anti-Flag M2 monoclonal antibody (manufactured by Sigma) as probes. Development of color was carried out using a detection buffer (100 mM Tris/HCl (pH 9.5), 100 mM NaCl, 5 mM MgCl₂, 0.33 mg/ml nitro blue tetrazolium (NBT), 0.17 mg/ml 5-bromo-4-chloro-3-indolyl phosphate (BCIP), p-toluidine salt). The results are shown in Fig. 23.

The band of phosphorylated activated JNK3 was observed only in the activated JNK3 enzyme solution (lane 2 in Fig. 23A). The band of JNK3 protein was observed only in the JNK3 enzyme solution (lane 3 in Fig. 23B), and band shift of JNK3 by phosphorylation was observed (lanes 2 and 3 in Fig. 23B).

Km values of ATP and GST-JSAP1 as the substrates for phosphorylation reaction in the screening system constructed above were calculated according to the method described in *Introduction to Enzyme Kinetics, Biochemical Experimentation, 21,* edited by Masatake Ohnishi, Japan Scientific Societies Press (1987).

Both of ATP and GST-JSAP1 showed Michaelis-Menten substrate-reaction initial rate curve, and it was found by Lineweaver-Burk plot that Km values of ATP and GST-JSAP1 were 6.3 µM and 0.48 µM, respectively.

### Example 3 Binding of JSAP3 to JNK3

COS-7 cells were transfected with the S-JSAP3 (full length) expression vector and the Flag-JNK3 expression vector, prepared in Example 1(2), using TransIT-LT1 (manufactured by Mirus), and then the COS-7 cells were cultured to transiently express the polypeptide derived from each of the introduced vectors.

After culturing for 34 hours, the cells were dissolved in buffer B, S Protein Agarose was added thereto, and then S-JSAP3 and polypeptide capable of binding to S-JSAP3 were precipitated.

The resulting fraction was separated by SDS-PAGE and transferred on membrane Immobilon-P (manufactured by Millipore).

Western blotting was carried out using the membrane and anti-Flag M5 monoclonal antibody (manufactured by Kodak) as the probe, and the polypeptide capable of binding to the antibody (Flag-JNK3) was visualized using ECL detection system (manufactured by Amersham).

The results are shown in Fig. 24.

It was confirmed that JSAP3 binds to JNK3 (lane 2 in Fig. 24).

Binding of JSAP3 to a transcription factor ATF2 and the ATF2 binding region were analyzed by the following method.

According to the method described in Example 1(2), JSAP3 (full length polypeptide), Trx·S, Trx·S-ATF2 (1st to 107th amino acid residues) and Trx·S-ATF2 (1st to 116th amino acid residues) fusion polypeptide were expressed in *E*. *coli* and then recovered by allowing them to bind to S Protein Agarose.

³⁵S-labeled form of S-JSAP3 was prepared using the S-JSAP3 (full length) expression vector obtained in Example 1(2) by *in vitro* translation with TNT T7 Quick Coupled Transcription/Translation System (manufactured by Promega).

The resulting ³⁵S-labeled form of S-JSAP3 and each of Trx·S, Trx·S-ATF2 (1st to 107th amino acid residues) and Trx·S-ATF2 (1st to 116th amino acid residues) were mixed in buffer A, and the reaction was carried out at 4°C for 2 hours while stirring the reaction solution by rotating its container tube.

After the reaction, the reactant was washed three times with buffer A, and the resulting precipitate was separated by SDS-PAGE and analyzed by autoradiography.

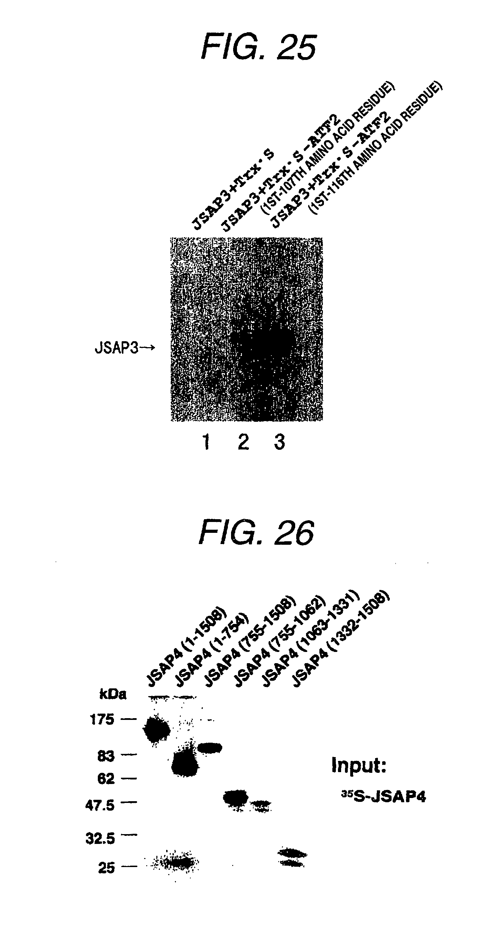

The results are shown in Fig. 25.

It was found that JSAP3 binds to a region of 108th to 116th amino acid residues of ATF2.

Among the JSAP3-binding ATF2 region of 108th to 116th residues, a sequence of the 108th to 112th residues completely coincided with an already reported CtBP binding sequence motif PLDLS (*J*. *Biol*. *Chem*., *273:* 8549 (1998)).

### Example 4 JSAP4

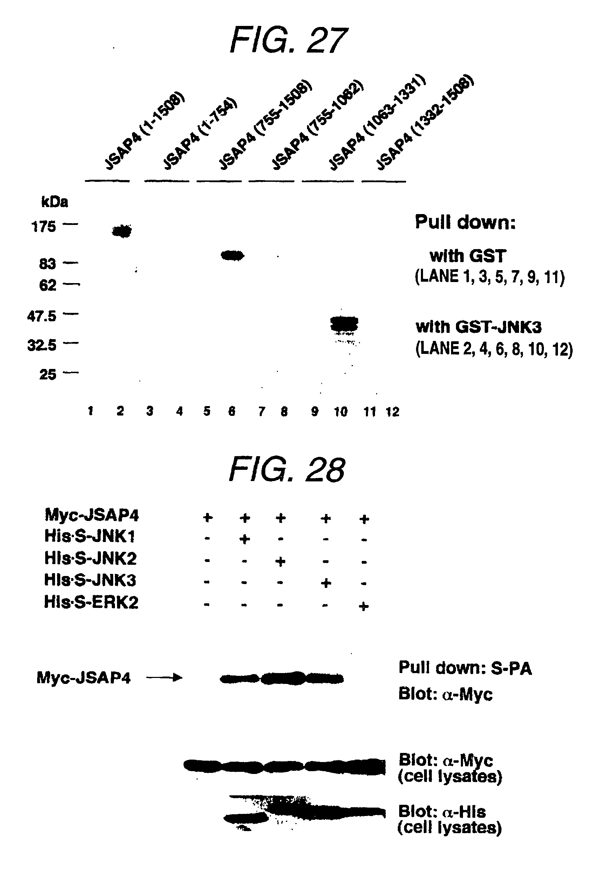

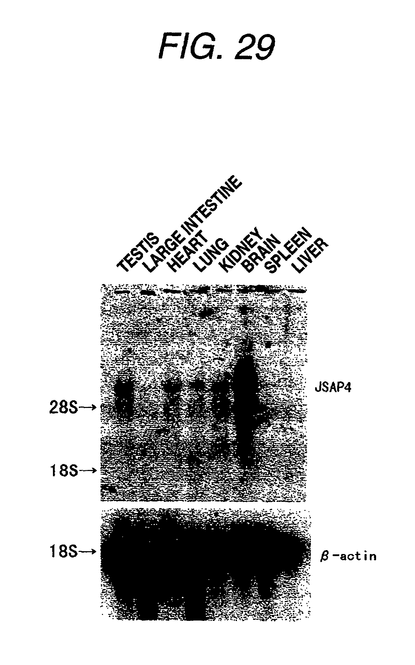

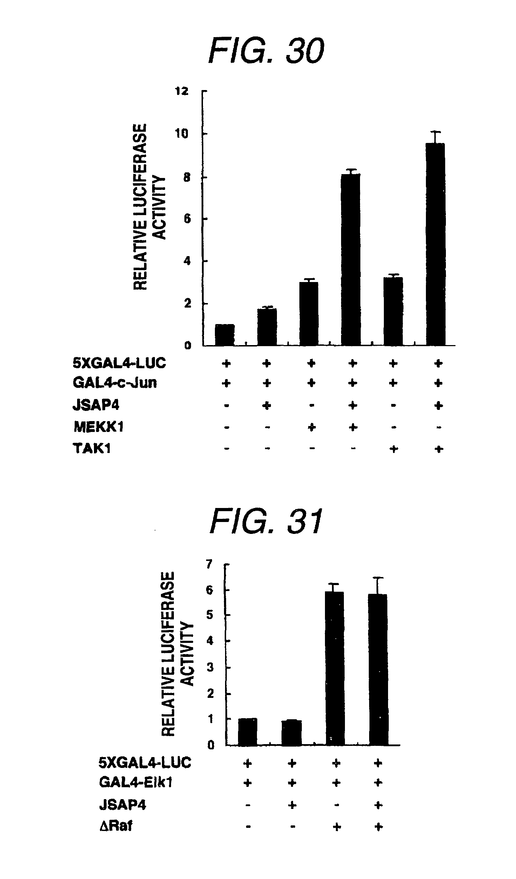

1) Binding of JSAP4 to JNK3
   COS-7 cells were transfected with the S-JSAP4 (full length) expression vector and the Flag-JNK3 expression vector, prepared in Example 1(2), using TransIT-LT1 (manufactured by Mirus), and then the COS-7 cells were cultured to transiently express the polypeptide derived from each of the introduced vectors.
   After culturing for 34 hours, the cells were dissolved in buffer B, S Protein Agarose was added thereto, and then S-JSAP4 and polypeptide capable of binding to S-JSAP4 were precipitated.
   The resulting fraction was separated by SDS-PAGE and transferred on membrane Immobilon-P (manufactured by Millipore).
   Western blotting was carried out using the membrane and anti-Flag M5 monoclonal antibody (manufactured by Kodak) as the probe, and the polypeptide capable of binding to the antibody (Flag-JNK3) was visualized using ECL detection system (manufactured by Amersham).
   The results are shown in Fig. 24.
   It was confirmed that JSAP4 binds to JNK3 (lane 4 in Fig. 24).
   The JNK3 binding region of JSAP4 was analyzed by the following method.
   The GST or GST-JNK3 fusion protein expression vector obtained in Example 1(2) was expressed in *E*. *coli*, and the protein was adsorbed to Glutathione-Agarose (manufactured by Sigma).
   Using the S-JSAP4 expression vector of full length or partial length JSAP4 obtained in Example 1(2), ³⁵S-labeled form of various S-JSAP4 preparations were prepared by *in vitro* translation using TNT T7 Quick Coupled Transcription/Translation System (manufactured by Promega), separated by SDS-PAGE and then analyzed by autoradiography.
   The results are shown in Fig. 26.
   The resulting ³⁵S-labeled form of each of various S-JSAP4 preparations and the GST or GST-JNK3 fusion protein adsorbed on Glutathione-Agarose were put into a tube containing buffer A and mixed while rotating the tube, and then the mixture was incubated at 4°C for 2 hours.
   After incubation, the reactant was washed three times with buffer A, and the protein adsorbed to Glutathione-Agarose was separated by SDS-PAGE and analyzed by autoradiography.
   The results are shown in Fig. 27.
   It was found that JNK3 binds to a region of the 1063rd to 1331st amino acid residues of JSAP4.
2) Binding of JSAP4 to JNK1 and JNK2
   COS-7 cells were co-transfected with the Myc-tag-added Myc-JSAP4 (1063rd to 1331st amino acid residue region; a region which binds to JNK3) expression vector and the His-S-tag-added His-S-JNK1, His-S-JNK2, His-S-JNK3 or His-S-ERK2 expression vector, prepared in Example 1(2), was carried out using FuGene 6 transfection reagent (manufactured by F. Hoffmann-La Roche).
   The COS-7 cells were cultured to transiently express the polypeptide derived from each of the introduced vectors.
   After culturing for 34 hours, the cells were dissolved in buffer B and immunoprecipitated using S-protein agarose (manufactured by Novagen).
   The resulting precipitation fraction was separated by SDS-PAGE and transferred on membrane Immobilon-P (manufactured by Millipore).
   Western blotting was carried out using the membrane and anti-Myc monoclonal antibody 9E10 (manufactured by Boehringer Mannheim) as the probe, and the Myc-JSAP4 capable of binding to the antibody was visualized using ECL detection system (manufactured by Amersham). Also, the expression of Myc-JSAP4 in respective co-transfected cells was confirmed by Western blotting using anti-Myc monoclonal antibody 9E10 (manufactured by Boehringer Mannheim), and the expression of His-S-JNK1, His-S-JNK2, His-S-JNK3 and His-S-ERK2 was confirmed by Western blotting using anti-His polyclonal antibody (manufactured by Santa Cruz) after the cells were lysed.
   The results are shown in Fig. 28.
   It was revealed that JSAP4 binds to JNK1, JNK2 and
   JNK3, whereas it does not bind to ERK2.
3) Expression analysis of JSAP4 mRNA by Northern hybridization
   Northern hybridization was carried out according to the method described in *Proc*. *Natl*. *Acad*. *Sci*. *USA, 92*: 4972 (1995).
   Specifically, each of mouse testis, large intestine, heart, lung, kidney, brain, spleen and liver was analyzed using ³²P-labeled JSAP4 and β-actin cDNA probes.
   The results are shown in Fig. 29.
   Expression of JSAP4 was found slightly in the testis, heart and kidney, and particularly, its most abundant expression was found in the brain.
   Since a sequence called WD40-repeat was found in JSAP4, it was expected that the sequence could be concerned in an interaction (binding) with other protein, and it was expected that it would take part in the JNK3 pathway on its signal transduction (*FEBS Lett., 307*: 131 (1994)).
4) Function analysis of JSAP4 in JNK3 pathway using reporter system
   Full length JSAP4 was over-expressed, and influence thereof on the JNK3 pathway was analyzed by the following method.
   5XGAL4-LUC reporter expression vector (manufactured by Stratagene), GAL4-c-Jun expression vector (1st to 223rd residues containing a c-Jun activation domain, manufactured by Stratagene) and RL control vector (manufactured by Promega) were introduced into COS-7 cells using FuGENE6 transfection reagent (manufactured by F. Hoffmann-La Roche).
   At least one of the Myc-JSAP4-FL expression vector, His-S-MEKK1 expression vector and Flag-TAK1 expression vector prepared in Example 1(2) was introduced into the COS-7 cells using the FuGENE6 transfection reagent.
   The COS-7 cells were cultured to transiently express the-polypeptide derived from each of the introduced vectors.
   The luciferase activity was measured after culturing for 34 hours, and the GAL4-c-Jun transcription activity, namely JNK3 activity, was calculated. The relative value of the luciferase activity was obtained by correcting it with the activity value of RL luciferase.
   The results are shown in Fig. 30.
   Increase in the JNK activity was slight by the JSAP4 *per se,* whereas the MEKK1 and TAK1 as members of MAPKKK increased the JNK activity 3 times and 3.2 times, respectively. However, the activation of JNK by MEKK1 and TAK1 was further 2.7 times and 3 times increased, respectively, by the over-expression of JSAP4.
   Influence of the over-expression of full length JSAP4 on the ERK pathway was examined in the same manner as the above.
   5XGAL4-LUC reporter expression vector, GAL4-Elk1 (307th to 427th residues containing an Elkl activation domain) expression vector and RL control vector were introduced into COS-7 cells using the FuGENE6 transfection reagent.
   The Myc-JSAP4-FL expression vector and/or the expression vector of continuously activated ΔRaf1 *(Mol. Cell. Biol*., *9*: 639 (1989)) polypeptide with Flag (Flag-ΔRaf1) prepared in Example 1(2) were introduced into the COS-7 cells using the FuGENE6 transfection reagent.
   The COS-7 cells were cultured to transiently express the polypeptide derived from each of the introduced vectors.
   The luciferase activity was measured after culturing for 34 hours, and the GAL4-Elk1 transcription activity, namely ERK activity, was calculated. The relative value of the luciferase activity was obtained by correcting it with the activity value of RL luciferase.
   The results are shown in Fig. 31.
   The over-expression of JSAP4 had no influence on the activity of the ERK pathway. Also, it had no influence on the activation of ERK by the activated ΔRaf1.
   Based on the results of 1) and 2) of Example 4, it was concluded that JSAP4 binds to JNK1, JNK2 and JNK3 and has a function of specifically increasing efficiency of the activation of the JNK pathway.

### Example 5 Binding of JSAP5 to JNK3

Transfection of COS-7 cells with the S-JSAP5 (partial length) expression vector and Flag-JNK3 expression vector prepared in Example 1(2) was carried out using TransIT-LT1 (manufactured by Mirus), and then the COS-7 cells were cultured for transient expression of the polypeptide derived from each of the introduced vectors.

After 34 hours of the culturing, the cells were dissolved in buffer B, S Protein Agarose was added thereto, and then S-JSAP5 and polypeptide capable of binding to S-JSAP5 were precipitated.

The resulting fraction was separated by SDS-PAGE and transferred on membrane Immobilon-P (manufactured by Millipore).

Western blotting was carried out using the membrane and anti-Flag M5 monoclonal antibody (manufactured by Kodak) as the probe, and the polypeptide capable of binding to the antibody (Flag-JNK3) was visualized using ECL detection system (manufactured by Amersham).

The results are shown in Fig. 24.

It was confirmed that JSAP5 binds to JNK3 (lane 6 of Fig. 24).

### INDUSTRIAL APPLICABILITY

Neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, and various immunological and inflammatory diseases can be prevented and treated using the DNA of the novel polypeptide having a JNK3 binding activity obtained in the present invention.

### FREE TEXT OF SEQUENCE LISTINGS:

SEQ ID NO:17-Explanation of artificial sequence: Synthetic DNA
SEQ ID NO:18-Explanation of artificial sequence: Synthetic DNA

## Claims

1. A polypeptide comprising an amino acid sequence selected from the amino acid sequences represented by any one of SEQ ID NOS:10 to 16.

2. A polypeptide comprising an amino acid sequence in which at least one amino acid has been deleted, substituted or added in an amino acid sequence selected from the amino acid sequences represented by any one of SEQ ID NOS:14 to 16 and is capable of binding to JNK3.

3. A DNA which encodes the polypeptide of claim 1 or 2.

4. A DNA comprising a nucleotide sequence selected from the nucleotide sequences represented by any one of SEQ ID NOS:2 to 8.

5. A DNA which hybridizes with a DNA comprising the nucleotide sequence represented by any one of SEQ ID NOS:6 to 8 under stringent conditions, and encodes a polypeptide capable of binding to JNK3.

6. A recombinant DNA obtained by inserting the DNA of any one of claims 3 to 5 into a vector.

7. The recombinant DNA according to claim 6, which is a recombinant DNA selected from plasmid pcDNA3-S-JSAP1b, plasmid pcDNA3-S-JSAPlc, plasmid pcDNA3-S-JSAP4, plasmid pGAD10-JSAP5, and plasmid pcDNA3-His-S-JSAP5.

8. A transformant comprising the recombinant DNA of claim 6 or 7.

9. The transformant according to claim 8, which is a transformant selected from a microorganism, an animal cell, a plant cell, and an insect cell.

10. The transformant according to claim 9, which is a microorganism belonging to the genus *Escherichia*.

11. The transformant according to claim 10, wherein the microorganism belonging to the genus *Escherichia* is a microorganism selected from *Escherichia coli* JSAP1b/pcDNA3 (FERM BP-6567), *Escherichia coli* JSAP1c/pcDNA3 (FERM BP-6568), *Escherichia coli* JSAP4/pcDNA3 (FERM BP-6569), *Escherichia coli* JSAP5/pGAD10 (FERM BP-6570), and *Escherichia coli* JSAP5/pcDNA3 (FERM BP-6928).

12. A method for producing the polypeptide of claim 1 or 2, comprising culturing the transformant of any one of claims 8 to 11 in a medium to produce and accumulate the polypeptide of claim 1 or 2 in the culture, and recovering the polypeptide from the culture.

13. An oligonucleotide which is selected from an oligonucleotide comprising a sequence identical to continuous 5 to 60 bases in a nucleotide sequence in any one of the DNA's of claims 3 to 5 and the DNA comprising the nucleotide sequence represented by SEQ ID NO:5, an oligonucleotide comprising a sequence complementary to the oligonucleotide, and an oligonucleotide analogue of these oligonucleotides.

14. The oligonucleotide according to claim 13, wherein the oligonucleotide analogue is selected from oligonucleotide analogues in which: a phosphodiester bond is converted into a phosphorothioate bond, a phosphodiester bond is converted into an N3'-P5' phosphoamidate bond, a ribose-phosphodiester bond is converted into a peptide-nucleic acid bond, uracil is substituted with C-5 propynyluracil, uracil is substituted with C-5 thiazoleuracil, cytosine is substituted with C-5 propynylcytosine, cytosine is substituted with phenoxazine-modified cytosine, ribose is substituted with 2'-O-propylribose, and ribose is substituted with 2'-methoxyethoxyribose.

15. A method for detecting mRNA encoding the polypeptide of claim 1 or 2, comprising using the oligonucleotide of claim 13 or 14.

16. A method for inhibiting expression of the polypeptide of claim 1 or 2, comprising using the oligonucleotide of claim 13 or 14.

17. An antibody which recognizes the polypeptide of claim 1 or 2.

18. A method for immunologically detecting the polypeptide of claim 1 or 2, comprising using the antibody of claim 17.

19. A method for immunohistologically staining of the polypeptide of claim 1 or 2, comprising using the antibody of claim 17.

20. An immunohistologically staining agent, comprising the antibody of claim 17.

21. A method of screening a compound having an inhibitory activity on binding of a polypeptide to JNK3, comprising bringing the polypeptide into contact with JNK3 and a test sample, said polypeptide comprising an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by any one of SEQ ID NOS:9 to 16 and being capable of binding to JNK3.

22. A method of screening a compound having an inhibitory activity on phosphorylation of a polypeptide caused by activated JNK3, comprising bringing the polypeptide into contact with activated JNK3 and a test sample, said polypeptide comprising an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by any one of SEQ ID NOS:9 to 16 and being capable of binding to JNK3.

23. A compound obtained by the method of claim 21 or 22 or a pharmacologically acceptable salt thereof.

24. A method of screening a compound capable of changing expression of a gene encoding a polypeptide, comprising bringing a cell which expresses the polypeptide into contact with a test sample, said polypeptide comprising an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by SEQ ID NOS:9 to 16 and being capable of binding to JNK3.

25. The method according to claim 24, wherein the expression of a gene is detected by the method of claim 15.

26. The method according to claim 24, wherein the polypeptide is detected using the method of claim 18.

27. A compound obtained by the method of any one of claims 24 to 26 or a pharmacologically acceptable salt thereof.

28. An inhibitor of binding of a polypeptide and JNK3, wherein the polypeptide comprises an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by any one of SEQ ID NOS:9 to 16 and being capable of binding to JNK3.

29. An inhibitor of phosphorylation of a polypeptide by activated JNK3, wherein the polypeptide comprises an amino acid sequence selected from the amino acid sequences represented by any one of SEQ ID NOS:9 to 16 or a polypeptide comprising an amino acid sequence in which at least one amino acid has been deleted, substituted or added in an amino acid sequence selected from the amino acid sequences represented by any one of SEQ ID NOS:9 to 16 and being capable of binding to JNK3.

30. An agent for preventing neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the polypeptide of claim 1 or 2.

31. An agent for treating neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the polypeptide of claim 1 or 2.

32. An agent for preventing neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the oligonucleotide of claim 13 or 14.

33. An agent for treating neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the oligonucleotide of claim 13 or 14.

34. An agent for preventing neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the antibody of claim 17.

35. An agent for treating neurodegenerative diseases, such as Alzheimer's disease, Parkinson's disease, Huntington's disease, multiple sclerosis and the like, amyotrophic diseases, such as amyotrophic lateral sclerosis and the like, ischemic diseases, brain damage due to stroke, schizophrenia, depression, epilepsy, or various immunological and inflammatory diseases, comprising the antibody of claim 17.

36. A promoter DNA which controls transcription of a gene encoding the polypeptide of claim 1 or 2.

37. A method of screening a compound capable of changing efficiency of transcription by the promoter DNA of 36, comprising bringing a test sample into contact with a transformant comprising a plasmid containing the promoter DNA and a reporter gene connected to the downstream of the promoter DNA; and measuring a translation product content of the reporter gene.

38. The method according to claim 37, wherein the reporter gene is a gene selected from a chloramphenicol acetyltransferase gene, a β-galactosidase gene, a luciferase gene, and a green fluorescent protein gene.

39. A compound obtained by the method of claim 37 or 38 or a pharmacologically acceptable salt thereof.
